# EUROPEAN PATENT SPECIFICATION

(11) **EP 0 418 175 B1**
(45) Date of publication and mention of the grant of the patent: **17.07.1996**
(21) Application number: 90420399.9
(22) Date of filing: 10.09.1990
(51) Int. Cl.: C07D 261/08, A01N 43/80, C07C 49/84, C07C 225/16, C07C 255/32, C07D 261/14

(54) **Isoxazoles herbicides**
Isoxazolderivate als Herbizide
Isoxazoles herbicides

(30) Priority: 11.09.1989 GB 8920519
(43) Date of publication of application: 20.03.1991
(73) Proprietor: RHONE POULENC AGRICULTURE LTD., Ongar, Essex CM5 OHW (GB)
(72) Inventor: Roberts, David Alan, Research Station of, Ongar, Essex CM5 0HW (GB); Cramp, Susan Mary, Research Station of, Ongar, Essex CM5 0HW (GB); Wallis, Derek Ian, Research Station of, Ongar, Essex CM5 0HW (GB); Bulot, Jean Paul, F-69570 Dardilly (FR)
(74) Representative: Brachotte, Charles

(56) References cited:
- EP-A- 0 129 846
- EP-A- 0 168 737
- EP-A- 0 176 846
- EP-A- 0 220 523
- EP-A- 0 229 635
- DE-A- 2 330 913
- FR-A- 2 023 474
- FR-A- 2 453 168
- US-A- 4 173 650
- JOURNAL OF THE AMERICAN CHEMICAL SOCIETY, vol. 58, no. 8, August 1936, Washington, DC (US); C.E. GROTHAUS et al., pp. 1334-1336
- CHEMICAL ABSTRACTS, vol. 111, no. 11, 10 September 1989, Columbus, OH (US); p. 730, AN 97105j
- ANGEWANDTE CHEMIE, INTERNATIONAL EDITION, vol. 21, no. 7, July 1982, Weinheim (DE); K.-D. KAMPE, pp. 540-541
- JOURNAL OF HETEROCYCLIC CHEMISTRY, vol. 20, no. 3, May-June 1983, Provo, UT (US); G. MENOZZI et al., pp. 645-648

## Description

This invention relates to 4-benzoyl isoxazole derivatives, compositions containing them and their use as herbicides.

In US patent No 4,173,650 (American Cyanamid Co) is described a 4-benzoyl-isoxazole of formula I below which is used in mixture as an intermediate to compounds having anti-inflammatory activity. 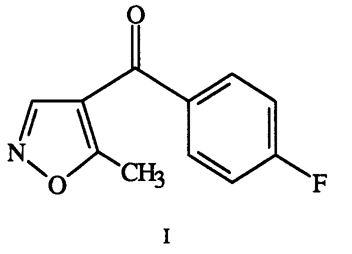
This reference discloses 4-benzoylisoxazoles of formula Ia: 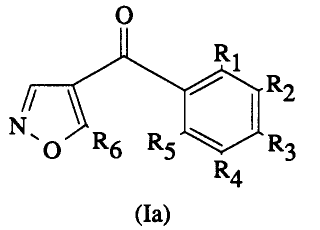
in which R₁, R₂, R₃, R₄ and R₅ are each individually selected from the group consisting of hydrogen, halogen, C₁₋₄ alkyl, C₁₋₄ alkoxy, trifluoromethyl and trichloromethyl with the proviso that at least two of R₁ to R₅ must be hydrogen but not all of R₁ to R₅ are hydrogen; and R₆ is C₁₋₄ alkyl; wherein the 4-benzoylisoxazole of formula Ia is in a mixture with a 5-phenylisoxazole of formula Ib: 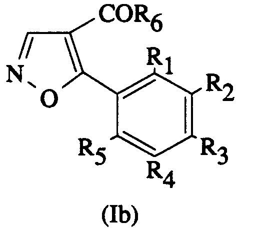
wherein R₁, R₂, R₃, R₄, R₅ and R₆ are as defined above.

D Grothaus (J. Amer. Chem. Soc., 1936 58 1334) describes the preparation of 5-amino-4-benzoylisoxazole (II) 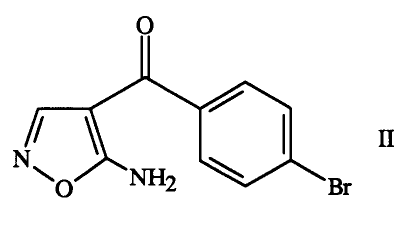
Neither of the above publications claim any use of the compounds as herbicides.

The present invention relates to 4-benzoyl isoxazoles derivatives of general formula III 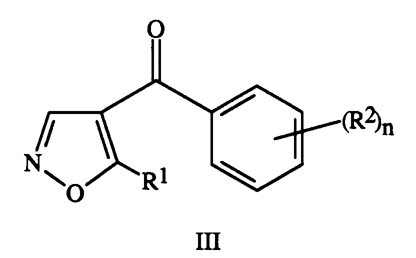
wherein R¹ represents:
a straight- or branched- chain alkyl, alkenyl, or alkynyl group containing up to 6 carbon atoms which is optionally substituted by one or more halogen atoms ; or
a cycloalkyl group containing from 3 to 6 carbon atoms optionally substituted by one or more R³ groups or one or more halogen atoms or an ester group, CO₂R³¹ ; or
a cycloalkenyl group containing 5 or 6 carbon atoms optionally substituted by one or more R³ groups or one or more halogen atoms or an ester group, CO₂R³¹;
or an aryl or aralkyl (e.g. benzyl) group, preferably of the formula (R²)_{q}-phenyl-[-CR³R⁴-]ₚ- (aryl contains 6 to 10 carbon atoms; aralkyl generally contains 7 to 11 carbon atoms); or
a group, CO₂R³ ; or
an aldehyde or acyl group, COR³ ; or
a cyano group ; or
a nitro group ; or
an amino group, NR³R⁴ ; or
a halogen atom (F,Cl,Br,I) ; and
R² represents:
a nitro group ; or
a cyano group ; or
a halogen atom (F,Cl,Br,I) ; or
a group R⁵ ; or
a sulphenyl, sulphinyl or sulphonyl group S(O)ₘR⁵ ; or
a sulphamoyl group SO₂NR³R⁴ ; or
a group CO₂R³ ; or
an aldehyde or acyl group COR³ ; or
a carbamoyl or thiocarbamoyl group CONR³R⁴ or CSNR³R⁴ ; or
an alkoxy group OR⁵ ; or
an alkyl group (with 1 to 3 carbon atoms) substituted by OR⁵, and
R³ and R⁴, which may be the same or different, each represents :
a hydrogen atom or a straight -or branched- chain alkyl group containing up to 6 carbon atoms which is optionally substituted by one or more halogen atoms ;
R³¹ is as defined for R³ but does not represent hydrogen;
R⁵ represents :
a straight -or branched- chain alkyl group containing up to 6 carbon atoms which is optionally substituted by one or more halogen atoms ;
m represents zero, 1 or 2;
n represents an integer from 1 to 5;
p represents zero or 1;
q represents zero or an integer from 1 to 5;
provided that
(a) when R¹ represents C₁₋₄ alkyl, n is an integer from one to three and R² is selected from the group consisting of halogen, C₁₋₄ alkyl, C₁₋₄ alkoxy, trifluoromethyl and trichloromethyl, the compound is not present in a mixture with a compound of formula:- 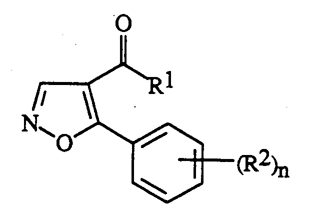 wherein R¹ is C₁₋₄ alkyl, n is an integer from one to three and R² is selected from the group consisting of halogen, C₁₋₄ alkyl, C₁₋₄ alkoxy, trifluoromethyl and trichloromethyl; and
(b) when R¹ is -NH₂, (R²)ₙ is not 4-bromo; (c) when R¹ is methyl, (R²)ₙ is not 4-fluoro; or an agriculturally acceptable salt thereof.
and agriculturally acceptable salts thereof, which possess valuable herbicidal properties.

It is to be understood that when n or q is an integer from 2 to 5, the substituents R² may be the same or different, and also the substituents R², R³ and R⁴ may be the same or different.

Furthermore, in certain cases the substituents R¹, R², R³, R⁴, R⁵ contribute to optical and/or stereosomerism. All such forms are embraced by the present invention.

The compound wherein R¹ is methyl and (R²)ₙ is 4-fluoro is not considered per se as part of the invention, nor is the compound wherein R¹ is NH₂ and (R²)ₙ is 4-bromo, nor the compounds of formula (Ia) above when present in mixture with the compounds of formula (Ib) above; but compositions containing them and their uses as herbicides are considered as a part of the invention.

By the term "agriculturally acceptable salts" is meant salts the cations or anions of which are known and accepted in the art for the formation of salts for agricultural or horticultural use. Preferably the salts are water-soluble. Suitable salts with bases include alkali metal (eg. sodium and potassium), alkaline earth metal (eg. calcium and magnesium), ammonium and amine (eg. diethanolamine, triethanolamine, octylamine, morpholine and dioctylmethylamine) salts. Suitable acid addition salts of the compounds of general formula III, which may be formed when the compounds incorporate an amino radical, include salts with inorganic acids, for example hydrochlorides, sulphates, phosphates and nitrates and salts with organic acids, for example acetic acid. It is to be understood that where reference is made in the present specification to the compounds of general formula III, such reference is intended to include also the salts with agriculturally acceptable acids or bases of compounds of general formula III where appropriate.

Among the compounds and/or the compositions and/or the method of use of the invention, it is preferred to use the compounds of general formula III wherein R¹ is alkyl or cycloalkyl and n is 2 or 3. Preferably one of the groups represented by (R²)ₙ is in the ortho position on the phenyl ring.

Further preferred compounds are also compounds of formula III wherein:
R¹ is alkyl optionally substituted by halogen ; or cycloalkyl optionally substituted by alkyl or halogen, and/or,
R² is halogen ; nitro ; R⁵ ; S(O)ₘR⁵ ; OR⁵ ; alkyl substituted by OR⁵ ; or COOR³¹ ; and/or
R⁵ is alkyl (with 1 to 3 carbon atoms) optionally substituted by fluorine or chlorine
Compounds of formula III which are of interest as herbicides include the following:
1. 5-Methyl-4-(2-nitro-4-trifluoromethylbenzoyl)-isoxazole
2. 5-Methyl-4-(2-nitrobenzoyl)-isoxazole
3. 4-(2-Nitro-4-trifluoromethylbenzoyl)-5-phenylisoxazole
4. 4-(2,4-Dinitrobenzoyl)-5-methylisoxazole
5. 5-(4-Chlorophenyl)-4-(2-nitro-4-trifluoromethylbenzoyl)-isoxazole
6. 4-(2-Chlorobenzoyl)-5-methylisoxazole
7. 5-Methyl-4-(2-nitro-4-methylsulphonylbenzoyl)-isoxazole
8. 5-(1-Methylethyl)-4-(2-nitro-4-trifluoromethylbenzoyl)-isoxazole
9. 4-(4-Chlorobenzoyl)-5-methylisoxazole
10. 4-(4-Methylbenzoyl)-5-methylisoxazole
11. 5-(4-fluorophenyl)-4-(2-nitro-4-trifluoromethylbenzoyl)-isoxazole
12. 5-Ethyl-4-(2-nitro-4-trifluoromethylbenzoyl)-isoxazole
13. 4-(4-Chloro-2-nitrobenzoyl)-5-methylisoxazole
14. 4-(2-Nitro-4-trifluoromethylbenzoyl)-5-propylisoxazole
15. 5-Cyclopropyl-4-(2-nitro-4-trifluoromethylbenzoyl)-isoxazole
16. 4-(2,3-Dichloro-4-methylsulphonylbenzoyl)-5-methylisoxazole
17. 5-(1,1-Dimethylethyl)-4-(2-nitro-4-trifluoromethylbenzoyl)-isoxazole
18. 4-(4-Methoxybenzoyl)-5-methylisoxazole
19. 5-Methyl-4-(4-methyl-2-nitrobenzoyl)-isoxazole
20. 4-(2,3-Dichloro-4-methylsulphonylbenzoyl)-5-(1-methylethyl)-isoxazole
21. 5-Cyclopropyl-4-(2,3-dichloro-4-methylsulphonylbenzoyl)-isoxazole
22. 4-(2-Nitro-4-trifluoromethylbenzoyl)-5-phenylmethylisoxazole
23. 4-(2-Chloro-4-trifluoromethylbenzoyl)-5-cyclopropylisoxazole
24. 5-Methyl-4-(2-nitro-4-pentafluoroethylbenzoyl)-isoxazole
25. 5-Cyclopropyl-4-[4-(1,1-dimethylethyl)-2-nitrobenzoyl]-isoxazole
26. 4-[4-(1,1-Dimethylethyl)-2-nitrobenzoyl]-5-methylisoxazole
27. 5-Cyclopentyl-4-(2-nitro-4-trifluoromethylbenzoyl)-isoxazole
28. 4-(2,4-Dichlorobenzoyl)-5-methylisoxazole
29. 4-(2-chloro-4-methylsulphonylbenzoyl)-5-methylisoxazole
30. 4-(2-Chloro-4-trifluoromethylbenzoyl)-5-methylisoxazole
31. 5-Methyl-4-(2-trifluoromethylbenzoyl)-isoxazole
32. 5-Methyl-4-(2,4-bis-trifluoromethylbenzoyl)-isoxazole
33. 4-(2-Chloro-4-methylsulphonylbenzoyl)-5-cyclopropylisoxazole
34. 5-Cyclopropyl-4-(2-trifluoromethylbenzoyl)-isoxazole
35. 5-Cyclopropyl-4-(2,4-dichlorobenzoyl)-isoxazole
36. 4-[2,3-Dichloro-4-(methylthio)benzoyl]-5-methylisoxazole
37. 5-Cyclopropyl-4(2,4-bis-trifluoromethylbenzoyl)-isoxazole
38. 4-(4-Chloro-2-trifluoromethylbenzoyl)-5-methylisoxazole
39. 4-(4-Cyano-2-nitrobenzoyl)-5-methylisoxazole
40. 5-Amino-4-(2-nitro-4-trifluoromethylbenzoyl)-isoxazole
41. 4-(4-Chloro-2-trifluoromethylbenzoyl)-5-cyclopropylisoxazole
42. 5-(1-Methylethyl)-4-(2-nitro-4-pentafluoroethylbenzoyl)-isoxazole
43. 4-(2-Chloro-4-methylsulphonylbenzoyl)-5-(1-methylethyl)-isoxazole
44. 5-Cyclopropyl-4-(4-fluoro-2-nitrobenzoyl)-isoxazole
45. 5-Cyclopropyl-4-(2-nitro-4-pentafluoroethylbenzoyl)-isoxazole
46. 4-(2,3-Dichloro-4-methylsulphinylbenzoyl)-5-methylisoxazole
47. 5-Cyclobutyl-4-(2-nitro-4-trifluoromethylbenzoyl)-isoxazole
48. 4-(4-Fluoro-2-nitrobenzoyl)-5-methylisoxazole
49. 5-(1-methylcyclopropyl)-4-(2-nitro-4-trifluoromethylbenzoyl)-isoxazole
50. 5-(4-Nitrophenyl)-4-(2-nitro-4-trifluoromethylbenzoyl)-isoxazole
51. 5-(4-Methoxyphenyl)-4-(2-nitro-4-trifluoromethylbenzoyl)-isoxazole
52. 4-(2-chloro-3-ethoxy-4-methylsulphonylbenzoyl)-5-methylisoxazole
53. 4-(3-Cyanobenzoyl)-5-methylisoxazole
54. 5-Cyclopropyl-4-(4-methylsulphonyl-2-trifluoromethylbenzoyl)-isoxazole
55. 5-Cyclopropyl-4-(2-nitro-4-methylsulphonylbenzoyl)-isoxazole
56. 4-(2-Chloro-3-ethoxy-4-methylsulphonylbenzoyl)-5-cyclopropylisoxazole
57. 4-(2-Chloro-3-ethoxy-4-ethylsulphonylbenzoyl)-5-methylisoxazole
58. 4-(2-Chloro-3-ethoxy-4-methylsulphonylbenzoyl)-5-(1-methylethyl)-isoxazole
59. 4-(2-Chloro-3-ethoxy-4-ethylsulphonylbenzoyl)-5-cyclopropylisoxazole
60. 5-(1-Ethoxycarbonylcyclopropyl)-4-(2-nitro-4-trifluoromethylbenzoyl)-isoxazole
61. 4-(3-Methoxycarbonyl-2-methyl-4-methylsulphonylbenzoyl)-5-methylisoxazole
62. 5-(2-Methylcyclopropyl)-4-(2-nitro-4-trifluoromethylbenzoyl)-isoxazole
63. 4-[2-Chloro-3-(1-methylethoxy)-4-methylsulphonylbenzoyl]-5-methylisoxazole
64. 5-Methyl-4-[2-methyl-3-(1-methylethoxycarbonyl)-4-methylsulphonylbenzoyl]-isoxazole
65. 5-Cyclopropyl-4-[2-methyl-3-(1-methylethoxycarbonyl)-4-methylsulphonyl-benzoyl]-isoxazole
66. 5-Cyclopropyl-4-(2,3,4-trichlorobenzoyl)-isoxazole
The numbers 1 to 66 are assigned to the above compounds for identification and reference hereinafter.

The compounds of general formula III can be prepared by the application or adaptation of known methods (ie. methods heretofore used or described in the chemical literature), for example as hereinafter described.

In the following description where symbols appearing in formulae are not specifically defined, it is to be understood that they are "as hereinbefore defined" in accordance with the first definition of each symbol in this specification.

It is to be understood that, in the descriptions of the following processes, the sequences may be performed in different orders, and that suitable protecting groups may be required to achieve the compounds sought.

According to a feature of the present invention the compounds of general formula III (with the exception of compounds in which R¹ is a cyano group, a nitro group, an amino group or a halogen atom) may be prepared by the reaction of a compound of general formula IV with a salt of hydroxylamine such as hydroxylamine hydrochloride in a solvent such as ethanol or acetonitrile, optionally in the presence of a base such as triethylamine at a temperature between room temperature and the reflux temperature of the solvent 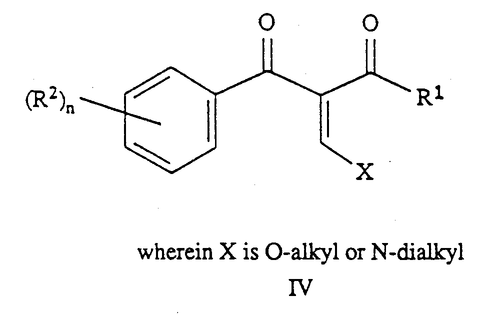
According to a further feature of the present invention the compounds of general formula III, in which R² is a halogen atom, an alkyl group, an alkylthio group or an alkoxy group, may be prepared by the reaction of a compound of general formula V with an excess of the appropriately substituted benzene in the presence of a Lewis acid catalyst such as aluminium chloride at a temperature between room temperature and 100°C. 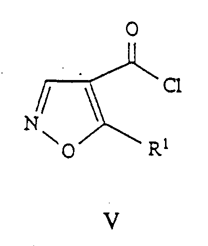
According to a further feature of the present invention the compounds of general formula III, in which R¹ is an unsubstituted amino group, may be prepared by the reaction of a compound of general formula VI with a salt of hydroxylamine such as hydroxylamine hydrochloride in a solvent such as ethanol or acetonitrile optionally in the presence of a base such as triethylamine at a temperature between room temperature and the reflux temperature of the solvent. 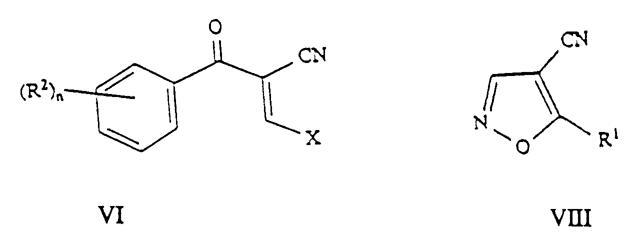
According to a further feature of the present invention, compounds of general formula III may be prepared by the reaction of compounds of general formula VIII with an organometallic reagent such as a Grignard reagent in an inert solvent such as ether or tetrahydrofuran at temperatures between room temperature and the reflux temperature of the solvent.

Intermediates in the preparation of compounds of general formula III are prepared by the application or adaptation of known methods. For instance compounds of general formula IV or VI are prepared by the reaction of a diketone or ketonitrile of general formula VII with an ortho ester such as triethyl orthoformate in the presence of an acid catalyst such as acetic anhydride at the reflux temperature of the mixture (as described by Schwan et al ; J. Heterocycl. Chem. 1976 13 973) or by the reaction of the diketone or ketonitrile of general formula VII with an amide acetal such as dimethyl formamide dimethyl acetal in an inert solvent such as toluene at a temperature between room temperature and the reflux temperature of the solvent (as described by W D Jones et al J. Heterocycl. Chem. 1987 24 1221) 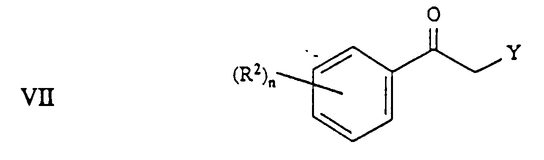
wherein Y is C0R¹ or C≡N
Intermediates of general formula V may be prepared from the appropriate carboxylic acids by the reaction with for example thionyl chloride at the reflux temperature of the mixture (as described by Doleschall and Seres, J. Chem. Soc. Perkin Trans. I 1988, 1973).

Intermediates of general formula VIII may be prepared from intermediates of general formula V by reaction with aqueous ammonia at a temperature between 0°C and room temperature, followed by dehydration using for example phosphorus oxychloride at a temperature between room temperature and 100°C.

Preparation of the diketone intermediates VII is described extensively in the literature for example Treibs and Hintermeier (Chem. Ber 1954 87 1163) describe the preparation of diketones by decarboxylation of t-butyl diketoesters with 4-toluene sulphonic acid and Hauser et al (Organic Reactions 1954, 8 59) review the preparation of diketones by acylation of ketones.

Interconversion of compounds of general formula III are possible by the application or adaptation of known methods as described for example in Comprehensive Heterocyclic Chemistry Vol 6. For example compounds of general formula III which cannot be made directly from compounds of general formula IV or general formula V may be made by interconversion of substituents R¹.
Examples of interconversions are indicated hereafter.

Compounds in which R¹ is a cyano group may be prepared from compounds in which R¹ is an ester by acidic hydrolysis of the carboxylic acid for example in aqueous acetic/hydrochloric acid at the reflux temperature of the mixture followed by conversion to the acid chloride using for example thionyl chloride at reflux. The acid chloride may be converted to the amide by treatment with aqueous ammonia at a temperature between 0°C and room temperature and the amide may be converted to the cyano group by dehydration using for example phosphorus oxychloride at a temperature between room temperature and 100°C.

Compounds in which R¹ is an acyl group may be prepared by the reaction of compounds in which R¹ is a cyano group with an organometallic reagent such as a Grignard reagent in an inert solvent such as ether or tetrahydrofuran at temperatures between room temperature and the reflux temperature of the solvent.

Compounds in which R¹ is a nitro group may be prepared by the oxidation of compounds in which R¹ is an unsubstituted amino group using for example trifluoroperacetic acid prepared in situ from trifluoroacetic acid and aqueous hydrogen peroxide.

Compounds in which R¹ is a halogen may be prepared from compounds in which R¹ is an unsubstituted amino group by diazotization. This may be carried out using sodium nitrite in the presence of an acid such as hydrochloric acid or hydrobromic acid followed by treatment with for example copper (I) chloride or copper (I) bromide at a temperature between room temperature and 80°C.
Alternatively diazotization may be carried out using an alkyl nitrite such as t-butyl nitrite in the presence of a halogenating agent such as copper (II) chloride or bromoform in an inert solvent such as tetrahydrofuran at a temperature between room temperature and the reflux temperature of the solvent.

Compounds in which R¹ is a substituted amino group may be prepared from compounds in which R¹ is a halogen atom by displacement using the appropriate amine in an inert solvent such as toluene at a temperature between 0°C and room temperature.

Compounds containing sulphinyl or sulphonyl groups may be prepared by oxidation of the compounds containing thioether groups using for example 3-chloroperbenzoic acid in an inert solvent such as dichloromethane at a temperature between -30°C and the reflux temperature of the solvent.

The following Examples illustrate the preparation of compounds of general formula III and the Reference Examples illustrate the preparation of intermediates.

In the present specification, bp means boiling point, mp means melting point. When the letters NMR are present it means that just thereafter the characteristics of the spectrum of the nuclear magnetic resonance of the proton are given. If not otherwise specified the percentages are by weight.

### EXAMPLE 1

### Compound 1

A mixture of crude 2-ethoxymethylene-1-(2-nitro-4-trifluoromethylphenyl)-butan-1,3-dione (13.25g) and hydroxylamine hydrochloride (3.7g) in ethanol (80ml) was stirred for 5h. The solution was evaporated almost to dryness and the resultant solution was diluted with ethyl acetate (100ml). The solution was washed with water (3 x 40ml), dried (anhydrous sodium sulphate) and filtered. The filtrate was evaporated to dryness. The residue was triturated with a mixture of ether and petroleum spirit (bp 60-80°C) (1:1) and the resultant buff solid was filtered off. The solid was dissolved in dichloromethane (100ml) and filtered through silica. The silica was washed with dichloromethane (200ml) and the combined filtrates were evaporated to dryness to give 4-(2-nitro-4-trifluoromethylbenzoyl)-5-methylisoxazole (4.5g) as an off-white solid mp 85-86°C.

By proceeding in a similar manner, the compounds described in the following table were prepared:

| | | | |
|---|---|---|---|
| **Compound N°** | 5 | 8 | 11 |
| **Form** | White solid | White flakes | White solid |
| **mp (°C)** | 159-160 | 138-139 | 150-151 |
| **NMR (where applicable)** | - | - | - |
| **Chromatography solvent** | - | Ethyl acetate/petroleum spirit (bp 60-80°C) 1:2 | - |
| **Recrystallization solvent** | Ethyl acetate/petroleum spirit (bp 60-80°C) 1:1 | Cyclohexane | Triturated ethanol |
| **Starting Material** | 3-(4 Chlorophenyl)-2-ethoxymethylene-1-(2-nitro-4-trifluoromethylphenyl)-propan-1,3 dione | 2-Ethoxymethylene-4-methyl-1-(2-nitro-4-trifluoromethylphenyl)-pentan-1,3 dione | 2-Ethoxymethylene-3-(4-fluorophenyl)-1-(2-nitro-4-trifluoromethylphenyl)-propan-1,3 dione |

| | | | |
|---|---|---|---|
| **Compound N°** | 12 | 14 | 15 |
| **Form** | Brown solid | White solid | Off-white solid |
| **mp (°C)** | 86-87 | 102-103 | 124.5-125 |
| **NMR (where applicable)** | **-** | **-** | **-** |
| **Chromatography solvent** | **-** | **-** | **-** |
| **Recrystallization solvent** | Ether petroleum spirit (bp 60-80°C) 1:20 | - | Cyclohexane |
| **Starting Material** | 2-Ethoxymethylene-1-(2-nitro-4-trifluoromethylphenyl)-pentan-1,3-dione | 2-Ethoxymethylene-1-(2-nitro-4-trifluoromethylphenyl)-hexan-1,3-dione | 3-Cyclopropyl-2-ethoxymethylene-1-(2-nitro-4-trifluoromethylphenyl)-propan-1,3-dione |

| | | | |
|---|---|---|---|
| **Compound N°** | 17 | 22 | 24 |
| **Form** | Buff solid | White flakes | White solid |
| **mp (°C)** | 169-170 | 86.5-87.5 | 128-129 |
| **NMR (where applicable)** | **-** | **-** | **-** |
| **Chromatography solvent** | Ethyl acetate/petroleum spirit (bp 60-80°C) 1:5 | - | Ethyl acetate/petroleum spirit (bp 60-80°C) 1:20 |
| **Recrystallization solvent** | | Petroleum spirit (bp 60-80°C) | Toluene |
| **Starting Material** | 4,4-Dimethyl-2-ethoxymethylene-1-(2-nitro-4-trifluoromethylphenyl)-pentan-1,3-dione | 2-Ethoxymethylene-1-(2-nitro-4-trifluoromethylphenyl)-4-phenylbutan-1,3-dione | 2-Ethoxymethylene-1-(2-nitro-4-pentafluoroethylphenyl)-butan-1,3-dione |

| | | | |
|---|---|---|---|
| **Compound N°** | 27 | 39 | 40 |
| **Form** | White solid | Off-white solid | Orange flakes |
| **mp (°C)** | 136.8 | 155-156 | 165-166 |
| **NMR (where applicable)** | **-** | **-** | **-** |
| **Chromatography solvent** | Ethyl acetate/petroleum spirit (bp 60-80°C) 1:5 | - | - |
| **Recrystallization solvent** | Cyclohexane | Triturated Ethyl acetate | Toluene |
| **Starting Material** | 3-Cyclopentyl-2-ethoxymethylene-1-(2-nitro-4-trifluoromethylphenyl)-propan-1,3-dione | 1-(4-Cyano-2-nitrophenyl)-2-ethoxy-methylenebutan-1,3-dione | 2-Ethoxymethylene-3-(2-nitro-4-trifluoromethylphenyl)-3-oxo-propionitrile |

| | | | |
|---|---|---|---|
| **Compound N°** | 42 | 45 | 60 |
| **Form** | White solid | Pale yellow solid | Viscous orange gum |
| **mp (°C)** | 139-140.5 | 108.6-110 | - |
| **NMR (where applicable)** | **-** | - | 1.1(t,3H)1.4(m,2H) 1.6(m,2H)4.0(q,2H) 7.6(d,1H)8.0(d,1H) 8.1(s,1H)8.4(s,1H) |
| **Chromatography solvent** | - | - | Ether |
| **Recrystallization solvent** | n-hexane | Ethanol | - |
| **Starting Material** | 2-Ethoxymethylene-4-methyl-1-(2-nitro-4-pentafluoroethylphenyl)-pentan-1,3-dione | 3-Cyclopropyl-2-ethoxymethylene-1-(2-nitro-4-pentafluoroethylphenyl)-propan-1,3-dione | 3-(1-Ethoxycarbonylcyclopropyl)-2-ethoxymethylene-1-(2-nitro-4-trifluoromethylphenyl)-propan-1,3-dione |

| | |
|---|---|
| **Compound N°** | 62 |
| **Form** | White solid |
| **mp (°C)** | 06-107.5 |
| **NMR (where applicable)** | **-** |
| **Chromatography solvent** | Ethyl acetate/petroleum spirit (bp 60-80°C) 1:2 |
| **Recrystallization solvent** | - |
| **Starting Material** | 2-Ethoxymethylene-3-(2-methylcyclopropyl)-1-(2-nitro-4-trifluoromethylphenyl)-propan-1,3-dione |

### EXAMPLE 2.

### Compound 2

Triethylamine (1.9g) was added to a mixture of 2-ethoxymethylene-1-(2-nitrophenyl)-butan-1,3-dione (4.9g) and hydroxylamine hydrochloride (1.3g) in acetonitrile (100ml) with stirring. The mixture was stirred for 2h and left to stand overnight. The mixture was evaporated almost to dryness and water (100ml) was added. The mixture was extracted with ethyl acetate (2 x 75ml) dried (anhydrous magnesium sulphate) and filtered. The filtrate was evaporated to dryness to give 4-(2-nitrobenzoyl)-5-methylisoxazole (2.4g) as a brown solid, mp 104.5-105.5°C.

By proceeding in a similar manner the compounds described in the following table were prepared:

| | | | |
|---|---|---|---|
| **Compound N°** | 3 | 4 | 6 |
| **Form** | Off-white solid | Brown solid | Yellow oil |
| **mp (°C)** | 149.5-150.5 | 133-135 | - |
| **NMR (where applicable)** | **-** | - | 2.62(s,3H)7.4 (m,2H)7.49(m,2H) 8.29(s,1H) |
| **Chromatography solvent** | Ethyl acetate/petroleum spirit (bp 60-80°C) 1:5 | - | Ethyl acetate/petroleum spirit (bp 60-80°C) 1:5 |
| **Recrystallization solvent** | -- | Triturated ethyl acetate | - |
| **Starting Material** | 2-Ethoxymethylene-1-(2-nitro-4-trifluoromethylphenyl)-3-phenyl)-propan-1,3-dione | 1-(2,4-Dinitrophenyl)-2-ethoxymethylenebutan-1,3-dione | 1-(2-Chlorophenyl)-2-ethoxymethylenebutan-1,3-dione |

| | | | |
|---|---|---|---|
| **Compound N°** | 7 | 13 | 16 |
| **Form** | White solid | Brown solid | White solid |
| **mp (°C)** | 159-160 | 98-99 | 128-129 |
| **NMR (where applicable)** | - | - | - |
| **Chromatography solvent** | Ethyl acetate/dichloromethane 1:5 | Ethyl acetate/n-hexane 1:10 | Ethyl acetate/n-hexane 1:5 |
| **Recrystallization solvent** | - | - | - |
| **Starting Material** | 2-Ethoxymethylene-1-(4-methyl-sulphonyl-2-nitro-phenyl)-butan-1,3-dione | 1-(4-Chloro-2-nitro-phenyl)-2-ethoxymethylenebutan-1,3-dione | 1-(2,3-Dichloro-4-methylsulphonyl-phenyl)-2-ethoxymethylenebutan-1,3-dione |

| | | | |
|---|---|---|---|
| **Compound N°** | 19 | 20 | 21 |
| **Form** | Orange solid | White solid | White solid |
| **mp (°C)** | 69-70 | 104-106 | 127.6-128.8 |
| **NMR (where applicable)** | **-** | **-** | **-** |
| **Chromatography solvent** | Ethyl acetate/cyclohexane 1:10 | - | Ethyl acetate/cyclohexane 1:10 |
| **Recrystallization solvent** | **-** | Triturated ether | - |
| **Starting Material** | 2-Ethoxymethylene-1-(4-methyl-2-nitrophenyl)-butan-1,3-dione | 1-(2,3-Dichloro-4-methylsulphonylphenyl)-2-ethoxymethylene-4-methylpentan-1,3-dione | 3-Cyclopropyl-1-(2,3-dichloro-4-methylsulphonylphenyl)-2-ethoxy-methylenepropan-1,3-dione |

| | | | |
|---|---|---|---|
| **Compound N°** | 23 | 25 | 26 |
| **Form** | White solid | Brown solid | White solid |
| **mp (°C)** | 39-40 | 87-89 | 124.8-125 |
| **NMR (where applicable)** | **-** | **-** | **-** |
| **Chromatography solvent** | Ethyl acetate/cyc lohexane 1:20 | Ethyl acetate/cyc lohexane 1:5 | - |
| **Recrystallization solvent** | **-** | **-** | Cyclohexane |
| **Starting Material** | 1-(2-Chloro-4-trifluoromethylphenyl)-3-cyclopropyl-2-ethoxymethylene-propan-1,3-dione | 3-Cyclopropyl-1-[4-(1,1-dimethylethyl)-2-nitrophenyl]-2-ethoxymethylenepropan-1,3-dione | 1-[4-(1,1-Dimethylethyl)-2-nitro-phenyl]-2-ethoxymethylene butan-1,3-dione |

| | | | |
|---|---|---|---|
| **Compound N°** | 28 | 29 | 30 |
| **Form** | Yellow oil | Orange solid | Brown solid |
| **mp (°C)** | - | 113.8 | 52.1 |
| **NMR (where applicable)** | 2.6(s,3H)7.3 (m,2H)7.4(s,1H) 8.2 (s,1H) | - | - |
| **Chromatography solvent** | Ethyl acetate/cyclohexane 1:50 | - | - |
| **Recrystallization solvent** | - | Triturated ether | Cyclohexane |
| **Starting Material** | 1-(2,4-Dichlorophenyl)-2-ethoxymethylenebutan-1,3-dione | 1-(2-Chloro-4-methylsulphonylphenyl)-2-ethoxymethylene-butan-1,3-dione | 1-(2-Chloro-4-trifluoromethylphenyl)-2-ethoxymethylene-butan-1,3-dione |

| | | | |
|---|---|---|---|
| **Compound N°** | 31 | 32 | 33 |
| **Form** | Yellow oil | Pale yellow oil | White solid |
| **mp (°C)** | **-** | **-** | 115-117 |
| **NMR (where applicable)** | 2.5(s,3H)7.4 (m,1H)7.6(m,2H) 7.7(m,1H)8.15 (s,1H) | 2.6(s,3H)7.5 (d,1H)7.9(d,1H) 8.0(s,1H)8.1 (s,1H) | - |
| **Chromatography solvent** | Ethyl acetate/cyclohexane 1:50 | Ethyl acetate/cyclohexane 1:20 | Ethyl acetate/toluene 1:20 |
| **Recrystallization solvent** | | | Ethyl acetate/cyclohexane 1:10 |
| **Starting Material** | 2-Ethoxymethylene-1-(2-trifluoromethylphenyl)-butan-1,3-dione | 1-(2,4-Bis-trifluoromethylphenyl)-2-ethoxymethylenebutan-1,3-dione | 1-(2-Chloro-4-methylsulphonylphenyl)-3-cyclopropyl-2-ethoxymethylenepropan-1,3-dione |

| | | | |
|---|---|---|---|
| **Compound N°** | 34 | 35 | 36 |
| **Form** | Viscous clear oil | Pale yellow oil | Cream solid |
| **mp (°C)** | **-** | **-** | 126-128 |
| **NMR (where applicable)** | 1.2(m,2H)1.3 (m,2H)2.55(m,1H) 7.5(m,1H)7.7 (m,2H)7.8(m,1H) 8.15(s,1H) | 1.15(m,2H)1.25 (m,2H)2.6(m,1H) 7.3(s,2H)7.4 (s,1H)8.1(s,1H) | - |
| **Chromatography solvent** | Ethyl acetate/cyclohexane 1:20 | Ethyl acetate/cyclohexane 1:50 | Ethyl acetate/cyclohexane 1:10 |
| **Recrystallization solvent** | | | Ethyl acetate/cyclohexane 1:1 |
| **Starting Material** | 3-Cyclopropyl-2-ethoxymethylene-1-(2-trifluoromethylphenyl)-propan-1,3-dione | 3-Cyclopropyl-1-(2,4-dichlorophenyl)-2-ethoxymethylenepropan-1,3-dione | 1-[2,3-Dichloro-4-(methylthio)-phenyl]-2-ethoxymethylenebutan-1,3-dione |

| | | | |
|---|---|---|---|
| **Compound N°** | 37 | 38 | 41 |
| **Form** | Pale yellow solid | Pale yellow solid | Pale yellow gum |
| **mp (°C)** | 74-75 | 89-89.5 | - |
| **NMR (where applicable)** | **-** | - | 1.2(m,2H)1.4 (m,2H)2.6(m,1H) 7.4(d,1H)7.7 (d,1H)7.8(s,1H) 8.1(s,1H) |
| **Chromatography solvent** | Ethyl acetate/cyclohexane 1:20 | - | Ethyl acetate/cyclohexane 1:20 |
| **Recrystallization solvent** | - | Cyclohexane | - |
| **Starting Material** | 1-(2,4-Bis-trifluoromethylphenyl)-3-cyclopropyl-2-ethoxymethylenepropan-1,3-dione | 1-(4-Chloro-2-trifluoromethylphenyl)-2-ethoxymethylenebutan-1,3-dione | 1-(4-Chloro-2-trifluoromethylphenyl)-3-cyclopropyl-2-ethoxymethylenepropan-1,3-dione |

| | | | |
|---|---|---|---|
| **Compound N°** | 43 | 44 | 47 |
| **Form** | Cream solid | Beige solid | Yellow solid |
| **mp (°C)** | 126-127 | 113.8-114.2 | 147-148 |
| **NMR (where applicable)** | **-** | **-** | **-** |
| **Chromatography solvent** | - | - | - |
| **Recrystallization solvent** | Ethyl acetate/n-hexane 1:5 | Ethyl acetate | Triturated ether/petroleum spirit (bp 60-80°C) 1:5 |
| **Starting Material** | 1-(2-Chloro-4-methylsulphonylphenyl)-2-ethoxymethylene -4-methylpentan-1,3-dione | 3-Cyclopropyl-2-ethoxymethylene-1-(4-fluoro-2-nitrophenyl)-propan 1,3-dione | 3-Cyclobutyl-2-ethoxymethylene-1-(2-nitro-4-trifluoromethylphenyl)-propan-1,3-dione |

| | | | |
|---|---|---|---|
| **Compound N°** | 48 | 49 | 50 |
| **Form** | White solid | Off-white solid | Yellow powder |
| **mp (°C)** | 85-87 | 150-152 | 170-171 |
| **NMR (where applicable)** | - | - | - |
| **Chromatography solvent** | Ethyl acetate/cyclohexane 1:10 | - | - |
| **Recrystallization solvent** | Ethyl acetate/cyclohexane 1:10 | Triturated ether | Toluene petroleum spirit (bp 60-80°C) 1:2 |
| **Starting Material** | 2-Ethoxymethylene-1-(4-fluoro-2-nitrophenyl)-butan-1,3-dione | 2-Ethoxymethylene-3-(1-methylcyclopropyl)-1-(2-nitro-4-trifluoromethylphenyl)-propan-1,3-dione | 2-Ethoxymethylene-3-(4-nitrophenyl)-1-(2-nitro-4-trifluoromethylphenyl)-propan-1,3-dione |

| | | | |
|---|---|---|---|
| **Compound N°** | 51 | 52 | 53 |
| **Form** | Yellow crystals | Light brown solid | Cream solid |
| **mp (°C)** | 154-155 | 104-106 | 93.8-94.4 |
| **NMR (where applicable)** | - | - | - |
| **Chromatography solvent** | - | Ethyl acetate/n-hexane 1:4 | Ethyl acetate/cyclohexane 1:5 |
| **Recrystallization solvent** | Toluene | | Ethyl acetate/n-hexane 1:4 |
| **Starting Material** | 2-Ethoxymethylene-3-(4-methoxyphenyl)-1-(2-nitro-4-trifluoromethylphenyl)-propan-1,3-dione | 1-(2-Chloro-3-ethoxy-4-methylsulphonylphenyl)-2-ethoxymethylenebutan-1,3-dione | 1-(3-Cyanophenyl)-2-ethoxymethylenebutan-1,3-dione |

| | | | |
|---|---|---|---|
| **Compound N°** | 54 | 55 | 56 |
| **Form** | Off-white solid | Cream solid | White solid |
| **mp (°C)** | 145.6-146.6 | 154.6-155.2 | 119-120.2 |
| **NMR (where applicable)** | **-** | **-** | **-** |
| **Chromatography solvent** | Ether | Ether | Ethyl acetate/cyclohexane 1:10 |
| **Recrystallization solvent** | - | - | Triturated cyclohexane |
| **Starting Material** | 3-Cyclopropyl-2-ethoxymethylene-1-(4-methylsulphonyl-2-trifluoromethylphenyl)-propan-1,3-dione | 3-Cyclopropyl-2-ethoxymethylene-1-(4-methylsulphonyl-2-nitrophenyl)-propan-1,3-dione | 1-(2-Chloro-3-ethoxy -4-methylsulphonylphenyl)-3-cyclopropyl-2-ethoxymethylenepropan-1,3-dione |

| | | | |
|---|---|---|---|
| **Compound N°** | 57 | 58 | 59 |
| **Form** | Cream solid | White solid | White solid |
| **mp (°C)** | 108.4-109 | 82.5-84.3 | 127.4-128.2 |
| **NMR (where applicable)** | **-** | **-** | **-** |
| **Chromatography solvent** | - | Ethyl acetate/cyclohexane 1:20 | - |
| **Recrystallization solvent** | Ether/cyclohexane 1:2 | | Ether/cyclohexane 1:4 |
| **Starting Material** | 1-(2-Chloro-3-ethoxy-4-ethylsulphonylphenyl)-2-ethoxymethylenebutan-1,3-dione | 1-(2-Chlor-3-ethoxy-4-methylsulphonylphenyl)-2-ethoxymethylene-4-methylpentan-1,3-dione | 1-(2-Chloro-3-ethoxy-4-ethylsulphonylphenyl)-3-cyclopropyl-2-ethoxymethylenepropan-1,3-dione |

| | | | |
|---|---|---|---|
| **Compound N°** | 61 | 63 | 64 |
| **Form** | Yellow solid | White solid | Pale pink solid |
| **mp (°C)** | 54-57 | 119-120.2 | 131-135 |
| **NMR (where applicable)** | - | - | - |
| **Chromatography solvent** | Ether | Ether | Ethyl acetate/cyclohexane 1:3 |
| **Recrystallization solvent** | Ether/cyclohexane 1:2 | - | - |
| **Starting Material** | 2-Ethoxymethylene-1-(3-methoxycarbonyl-2-methyl-4-methylsulphonylphenyl)-butan-1,3-dione | 1-[2-Chlor-3-(1-methylethoxy)-4-methylsulphonylphenyl]-2-ethoxymethylenebutan-1,3-dione | 2-Ethoxymethylene-1-[2-methyl-3-(1-methylethoxycarbonyl)-4-methylsulphonylphenyl]-butan-1,3-dione |

| | | | |
|---|---|---|---|
| **Compound N°** | 65 | 66 | |
| **Form** | White solid | Off-white solid | |
| **mp (°C)** | 164-166 | 105-107 | |
| **NMR (where applicable)** | - | - | |
| **Chromatography solvent** | Ether | Ether/petroleum spirit (bp 60-80°C) 1:9 | - |
| **Recrystallization solvent** | | cyclohexane | |
| **Starting Material** | 3-Cyclopropyl-2-ethoxymethylene-1-[2-methyl-3-(1-methylethoxycarbonyl)-4-methylsuIphonylphenyl]-propan-1,3-dione | 3-Cyclopropyl-2-ethoxymethylene-1-(2,3,4-trichlorophenyl)-propan-1,3-dione | |

### EXAMPLE 3.

### Compound 46

3-Chloroperbenzoic acid (85%, 0.99g) was added portionwise to a stirred solution of 4-[2,3-dichloro-4-(methylthio)-benzoyl]-5-methylisoxazole (1.5g) in dichloromethane (50ml) whilst maintaining the temperature below - 20°C. The mixture was stirred at -20°C for 1h. Dichloromethane (50ml) was added and the solid was removed by filtration. The filtrate was evaporated to dryness and the residue was chromatographed on silica eluted with a mixture of ethyl acetate and cyclohexane (1:5) to give 4-(2,3-dichloro-4-methylsulphinylbenzoyl) -5-methylisoxazole (0.8g) as a white solid, mp 125-126.4°C.

### EXAMPLE 4.

### Compound 9

A mixture of aluminium chloride (16g) and 5-methylisoxazole-4-carbonyl chloride (5.0g) in dry chlorobenzene (50ml) was stirred in an atmosphere of nitrogen for 16h. The mixture was heated to 80°C for 1.5h. The cooled mixture was quenched with excess ice and extracted with ethyl acetate (3 x 200ml). The combined organic layers were washed with water (3 x 500ml), dried (anhydrous magnesium sulphate) and filtered. The filtrate was evaporated to dryness. The residue was purified by chromatography on silica eluted with a mixture of ethyl acetate and cyclohexane (1:20) to give 4-(4-chlorobenzoyl)-5-methylisoxazole (5.2g) as a yellow solid mp 65-66°C.

By proceeding in a similar manner the following compound was prepared : 4-(4-methylbenzoyl)-5-methylisoxazole, NMR (CDCl₃) 2.36(s,3H) 2.59(s,3H) 7.2-7.7(m,4H) 8.36(s,1H), starting from toluene.

### EXAMPLE 5.

### Compound 18

A mixture of aluminium chloride (10g) and 5-methylisoxazole-4-carbonyl chloride (2.7g) in methoxybenzene (50ml) was stirred at room temperature for 16h. The mixture was quenched with excess ice and extracted with ether (3 x 200ml). The combined organic layers were washed with water (3 x 500ml), dried (anhydrous magnesium sulphate) and filtered. The filtrate was evaporated to dryness. The residue was purified by chromatography on silica eluted with a mixture of ethyl acetate and cyclohexane (1:10) followed by HPLC on silica eluted with a mixture of ethyl acetate and hexane (1:20) to give 4-(4-methoxybenzoyl)-5-methylisoxazole, (0.35g) as a white solid mp 78-79°C.

### REFERENCE EXAMPLE 1.

A mixture of 1-(2-nitro-4-trifluoromethylphenyl)-butan-1,3-dione (11.0g), triethyl orthoformate (11.3g) and acetic anhydride (12.3g) was stirred and heated at reflux for 3h. After cooling, the mixture was evaporated to dryness. Toluene (50ml) was added and the mixture was evaporated to dryness to give 2-ethoxymethylene-1-(2-nitro-4-trifluoromethylphenyl)-butan-1,3-dione as a crude brown oil which was not further purified.

By proceeding in a similar manner the following compounds were prepared:
2-Ethoxymethylene-1-(2-nitrophenyl)-butan-1,3-dione, as a crude black gum, starting from 1-(2-nitrophenyl)-butan-1,3-dione.
2-Ethoxymethylene-1-(2-nitro-4-trifluoromethylphenyl)-3-phenylpropan- 1,3-dione, as a crude orange oil, starting from 1-(2-nitro-4-trifluoromethyl- phenyl)-3-phenylpropan-1,3-dione.
1-(2,4-dinitrophenyl)-2-ethoxymethylenebutan-1,3-dione, as a crude black gum, starting from 1-(2,4-dinitrophenyl)-butan-1,3-dione.
3-(4-Chlorophenyl)-2-ethoxymethylene-1-(2-nitro-4-trifluoromethylphenyl)-propan-1,3-dione, as a white solid, by trituration with ether, mp 146-471°C starting from 3-(4-chlorophenyl)-1-(2-nitro-4-trifluoromethylphenyl)-propan- 1,3-dione.
1-(2-Chlorophenyl)-2-ethoxymethylenebutan-1,3-dione, as a crude black gum, starting from 1-(2-chlorophenyl)-butan-1,3-dione.
2-Ethoxymethylene-1-(4-methylsulphonyl-2-nitrophenyl)-butan-1,3-dione as a crude brown gum, starting from 1-(4-methylsulphonyl-2-nitrophenyl)-butan-1,3-dione.
2-Ethoxymethylene-4-methyl-1-(2-nitro-4-trifluoromethylphenyl)-pentan-1,3- dione as a crude red oil, starting from 4-methyl-1-(2-nitro-4-trifluoromethyl-phenyl)-pentan-1,3-dione.
2-Ethoxymethylene-3-(4-fluorophenyl)-1-(2-nitro-4-trifluoromethylphenyl)- propan-1,3-dione, as an orange solid, mp 103-104°C, starting from 3-(4-fluorophenyl)-1-(2-nitro-4-trifluoromethylphenyl)-propan-1,3-dione.
2-Ethoxymethylene-1-(2-nitro-4-trifluoromethylphenyl)-pentan-1,3-dione, as a crude brown oil, starting from 1-(2-nitro-4-trifluoromethylphenyl)-pentan-1,3-dione.
1-(4-Chloro-2-nitrophenyl)-2-ethoxymethylenebutan-1,3-dione, as a crude black gum, starting from 1-(4-chloro-2-nitrophenyl)-butan-1,3-dione.
2-Ethoxymethylene-1-(2-nitro-4-trifluoromethylphenyl)-hexan-1,3-dione, as a crude red oil, starting from 1-(2-nitro-4-trifluoromethylphenyl)-hexan-1,3-dione.
3-Cyclopropyl-2-ethoxymethylene-1-(2-nitro-4-trifluoromethylphenyl)-propan-1,3-dione, as a crude orange oil, starting from 3-cyclopropyl-1-(2-nitro-4-trifluoromethylphenyl)-propan-1,3-dione.
1-(2,3-Dichloro-4-methylsulphonylphenyl)-2-ethoxymethylenebutan-1,3-dione as a crude black gum, starting from 1-(2,3-dichloro-4-methylsulphonylphenyl)- butan-1,3-dione.
4,4-Dimethyl-2-ethoxymethylene-1-(2-nitro-4-trifluoromethylphenyl)-pentan- 1,3-dione, as a white solid by trituration with petroleum spirit (bp 60-80°C) mp 134-135°C, starting from 4,4-dimethyl-1-(2-nitro-4-trifluoromethylphenyl)- pentan-1,3-dione.
2-Ethoxymethylene-1-(4-methyl-2-nitrophenyl)-butan-1,3-dione as a crude black gum, starting from 1-(4-methyl-2-nitrophenyl)butan-1,3-dione.
1-(2,3-Dichloro-4-methylsulphonylphenyl)-2-ethoxymethylene-4-methylpentan-1,3-dione, as a crude black gum, starting from 1-(2,3-dichloro-4-methyl-sulphonylphenyl)-4-methylpentan-1,3-dione.
3-Cyclopropyl-1-(2,3-dichloro-4-methylsulphonylphenyl)-2-ethoxymethylene-propan-1,3-dione, as a crude brown gum, starting from 3-cyclopropyl-1-(2,3-dichloro-4-methylsulphonylphenyl)-propan-1,3-dione.
2-Ethoxymethylene-1-(2-nitro-4-trifluoromethylphenyl)-4-phenylbutan-1,3-dione, as an orange solid by trituration with petroleum spirit (bp 60-80°C) mp 145-147°C, starting from 1-(2,nitro-4-trifluoromethylphenyl)-4-phenylbutan-1,3-dione.
1-(2-Chloro-4-trifluoromethylphenyl)-3-cyclopropyl-2-ethoxymethylene-propan-1,3-dione, as a crude brown gum, starting from 1-(2-chloro-4-trifluoro-methyl-phenyl)-3-cyclopropylpropan-1,3-dione.
2-Ethoxymethylene-1-(2-nitro-4-pentafluoroethylphenyl)-butan-1,3-dione, as a crude brown oil, starting from 1-(2-nitro-4-pentafluoroethylphenyl)butan-1,3-dione.
3-Cyclopropyl-1-[4-(1,1-dimethylethyl)-2-nitrophenyl]-2-ethoxymethylene-propan-1,3-dione as a crude red gum, starting from 3-cyclopropyl-1-[4-(1,1-dimethylethyl)-2-nitrophenyl]-propan-1,3-dione.
1-[4-(1,1-dimethylethyl)-2-nitrophenyl]-2-ethoxymethylenebutan-1,3-dione, as a crude red gum, starting from 1-[4-(1,1-dimethylethyl)-2-nitrophenyl]-butan-1,3-dione.
3-Cyclopentyl-2-ethoxymethylene-1-(2-nitro-4-trifluoromethylphenyl)-propan-1,3-dione, as a crude red oil, starting from 3-cyclopentyl-1-(2-nitro-4-trifluoromethylphenyl)-propan-1,3-dione.
1-(2,4-Dichlorophenyl)-2-ethoxymethylenebutan-1,3-dione, as a crude brown oil, starting from 1-(2,4-dichlorophenyl)-butan-1,3-dione.
1-(2-Chloro-4-methylsulphonylphenyl)-2-ethoxymethylenebutan-1,3-dione as a crude brown oil, starting from 1-(2-chloro-4-methylsulphonylphenyl)-butan-1,3-dione.
1-(2-Chloro-4-trifluoromethylphenyl)-2-ethoxymethylenebutan-1,3-dione, as a crude red oil, starting from 1-(2-chloro-4-trifluoromethylphenyl)-butan-1,3-dione.
2-Ethoxymethylene-1-(2-trifluoromethylphenyl)-butan-1,3-dione as a crude red oil, starting from 1-(2-trifluoromethylphenyl)-butan-1,3-dione.
1-(2,4-Bis-trifluoromethylphenyl)-2-ethoxymethylenebutan-1,3-dione, as a crude red gum, starting from 1-(2,4-bis-trifluoromethylphenyl)-butan-1,3-dione.
1-(2-Chloro-4-methylsulphonylphenyl)-3-cyclopropyl-2-ethoxymethylene-propan-1,3-dione as a crude golden oil, starting from 1-(2-chloro-4-methyl-sulphonyl-phenyl)-3-cyclopropylpropan-1,3-dione.
3-Cyclopropyl-2-ethoxymethylene-1-(2-trifluoromethylphenyl)-propan-1,3-dione, as a crude yellow oil starting from 3-cyclopropyl-1-(2-trifluoromethyl-phenyl)-propan-1,3-dione.
3-Cyclopropyl-1-(2,4-dichlorophenyl)-2-ethoxymethylenepropan- 1,3-dione, as a crude brown oil, starting from 3-cyclopropyl-1-(2,4-dichlorophenyl)-propan-1,3-dione.
1-[2,3-Dichloro-4-(methylthio)-phenyl]-2-ethoxymethylenebutan-1,3-dione, as a crude red oil starting from 1-[2,3-dichloro-4-(methylthio)-phenyl]-butan-1,3-dione.
1-(2,4-Bis-trifluoromethylphenyl)-3-cyclopropyl-2-ethoxymethylenepropan-1,3-dione, as a crude yellow gum, starting from 1-(2,4-bis-trifluoromethyl-phenyl)-3-cyclopropylpropan-1,3-dione.
1-(4-Chloro-2-trifluoromethylphenyl)-2-ethoxymethylenebutan-1,3-dione, as a crude black gum, starting from 1-(4-chloro-2-trifluoromethylphenyl)-butan-1,3-dione.
1-(4-Cyano-2-nitrophenyl)-2-ethoxymethylenebutan-1,3-dione, as a crude brown oil, starting from 1-(4-cyano-2-nitrophenyl)-butan-1,3-dione.
2-Ethoxymethylene-3-(2-nitro-4-trifluoromethylphenyl)-3-oxopropionitrile as a crude yellow oil, starting from 3-(2-nitro-4-trifluoromethylphenyl)-3-oxopropionitrile.
1-(4-Chloro-2-trifluoromethylphenyl)-3-cyclopropyl-2-ethoxymethylene-propan-1,3-dione as a crude yellow oil, starting from 1-(4-chloro-2-trifluoro-methylphenyl)-3-cyclopropylpropan-1,3-dione.
2-Ethoxymethylene-4-methyl-1-(2-nitro-4-pentafluoroethylphenyl)-pentan-1,3-dione, as a crude red oil, starting from 4-methyl-1-(2-nitro-4-pentafluoro-ethylphenyl)-pentan-1,3-dione.
1-(2-Chloro-4-methylsulphonylphenyl)-2-ethoxymethylenebutan-1,3-dione, as a crude brown oil, starting from 1-(2-chloro-4-methylsulphonylphenyl)-butan-1,3-dione.
3-Cyclopropyl-2-ethoxymethylene-1-(4-fluoro-2-nitrophenyl)-propan-1,3-dione as a crude black oil, starting from 3-cyclopropyl-1-(4-fluoro-2-nitrophenyl)-propan-1,3-dione.
3-Cyclopropyl-2-ethoxymethylene-1-(2-nitro-4-pentafluoroethylphenyl)-propan 1,3-dione, as a crude brown oil, starting from 3-cyclopropyl-1-(2-nitro-4-pentafluoroethylphenyl)-propan-1,3-dione.
3-Cyclobutyl-2-ethoxymethylene-1-(2-nitro-4-trifluoromethylphenyl)-propan-1,3-dione, as a crude red oil, starting from 3-cyclobutyl-1-(2-nitro-4-trifluoromethylphenyl)-propan-1,3-dione.
2-Ethoxymethylene-1-(4-fluoro-2-nitrophenyl)-butan-1,3-dione, as a crude black oil, starting from 1-(4-fluoro-2-nitrophenyl)-butan-1,3-dione.
2-Ethoxymethylene-3-(1-methylcyclopropyl)-1-(2-nitro-4-trifluoromethyl-phenyl)-propan-1,3-dione, as a white solid by trituration with petroleum spirit (bp 60-80°C) mp 124-125°C, starting from 3-(1-methylcyclopropyl)-1-(2-nitro-4-trifluoromethylphenyl)-propan-1,3-dione.
2-Ethoxymethylene-3-(4-nitrophenyl)-1-(2-nitro-4-trifluoromethylphenyl)-propan-1,3-dione, as a crude orange semi-solid, starting from 3-(4-nitrophenyl)-1-(2-nitro-4-trifluoromethylphenyl)-propan-1,3-dione.
2-Ethoxymethylene-3-(4-methoxyphenyl)-1-(2-nitro-4-trifluoromethylphenyl)-propan-1,3-dione, as a crude brown solid, starting from 3-(4-methoxyphenyl)-1-(2-nitro-4-trifluoromethylphenyl)-propan-1,3-dione.
1-(2-Chloro-3-ethoxy-4-methylsulphonylphenyl)-2-ethoxymethylenebutan-1,3-dione, as a crude orange oil, starting from 1-(2-chloro-3-ethoxy-4-methyl-sulphonylphenyl)-butan-1,3-dione.
1-(3-Cyanophenyl)-2-ethoxymethylenebutan-1,3-dione, as a crude black oil, starting from 1-(3-cyanophenyl)-butan-1,3-dione.
3-Cyclopropyl-2-ethoxymethylene-1-(4-methylsulphonyl-2-trifluoromethyl-phenyl)-propan-1,3-dione, as a crude brown solid, starting from 3-cyclopropyl-1-(4-methylsulphonyl-2-trifluoromethylphenyl)-propan- 1,3-dione.
3-Cyclopropyl-2-ethoxymethylene-1-(4-methylsulphonyl-2-nitrophenyl)-propan-1,3-dione, as a crude red oil, starting from 3-cyclopropyl-1-(4-methyl-sulphonyl-2-nitrophenyl)-propan-1,3-dione.
1-(2-Chloro-3-ethoxy-4-methylsulphonylphenyl)-3-cyclopropyl-2-ethoxy-methylenepropan-1,3-dione, as a crude red gum, starting from 1-(2-chloro-3-ethoxy-4-methylsulphonylphenyl)-3-cyclopropylpropan-1,3-dione.
1-(2-Chloro-3-ethoxy-4-ethylsulphonylphenyl)-2-ethoxymethylenebutan-1,3-dione, as a crude red oil, starting from 1-(2-chloro-3-ethoxy-4-ethylsulphonyl-phenyl)-butan-1,3-dione.
1-(2-Chloro-3-ethoxy-4-methylsulphonylphenyl)-2-ethoxymethylene-4-methyl-pentan-1,3-dione, as a crude red gum, starting from 1-(2-chloro-3-ethoxy-4-methylsulphonylphenyl)-4-methylpentan-1,3-dione.
1-(2-Chloro-3-ethoxy4-ethylsulphonylphenyl)-3-cyclopropyl-2-ethoxy-methylenepropan-1,3-dione, as a crude red oil, starting from 1-(2-chloro-3-ethoxy-4-ethylsulphonylphenyl)-3-cyclopropylpropan-1,3-dione.
3-(1-Ethoxycarbonylcyclopropyl)-2-ethoxymethylene-1-(2-nitro-4-trifluoro-methylphenyl)-propan-1,3-dione, as a crude brown oil, starting from 3-(1-ethoxycarbonyl-cyclopropyl)-1-(2-nitro-4-trifluoromethylphenyl)-propan-1,3-dione.
2-Ethoxymethylene-1-(3-methoxycarbonyl-2-methyl-4-methylsulphonylphenyl)-butan-1,3-dione, as a crude brown oil, starting from 1-(3-methoxycarbonyl-2-methyl-4-methylsulphonylphenyl)-butan-1,3-dione.
2-Ethoxymethylene-3-(2-methylcyclopropyl)-1-(2-nitro-4-trifluoromethyl-phenyl)-propan-1,3-dione, as a crude red oil, starting from 3-(2-methyl-cyclopropyl)-1-(2-nitro-4-trifluoromethylphenyl)-propan-1,3-dione.
1-[2-Chloro-3-(1-methylethoxy)-4-methylsulphonylphenyl]-2-ethoxymethylene-butan-1,3-dione, as a crude black gum, starting from 1-[2-chloro-3-(1-methyl-ethoxy)-4-methylsulphonylphenyl]-butan-1,3-dione.
2-Ethoxymethylene-1-[2-methyl-3-(1-methylethoxycarbonyl)-4-methyl-sulphonylphenyl]-butan-1,3-dione, as a crude red oil, starting from 1-[2-methyl-3-(1-methylethoxycarbonyl)-4-methylsulphonylphenyl]-butan-1,3-dione.
3-Cyclopropyl-2-ethoxymethylene-1-[2-methyl-3-(1-methylethoxycarbonyl)-4-methylsulphonylphenyl]-propan-1,3-dione, as a crude orange oil, starting from 3-cyclopropyl-1-[2-methyl-3-(1-methylethoxycarbonyl)-4-methylsulphonylphenyl]-propan-1,3-dione.
3-Cyclopropyl-2-ethoxymethylene-1-(2,3,4-trichlorophenyl)-propan-1,3-dione as a crude brown oil, starting from 3-cyclopropyl-1-(2,3,4-trichlorophenyl)-propan-1,3-dione.

### REFERENCE EXAMPLE 2.

A mixture of crude t-butyl 2-(2-nitro-4-trifluoromethylbenzoyl)-3-oxobutanoate (9.1g) and 4-toluenesulphonic acid (0.1g) in dry toluene (100ml) was stirred and heated at reflux for 3h. The cooled mixture was extracted with aqueous sodium hydroxide solution (2M, 2 x 50ml) and water (2 x 50ml). The combined aqueous extracts were acidified to pH 1 and extracted with ether (3 x 100ml). The combined organic layers were washed with water (50ml), dried (anhydrous sodium sulphate) and filtered. The filtrate was evaporated to dryness and the residue was recrystallized from petroleum spirit (bp 60-80°C) to give 1-(2-nitro-4-trifluoromethylphenyl)-butan-1,3-dione (4.6g) as an off-white solid mp 80-81°C.

By proceeding in a similar manner, the following compounds were prepared:
1-(2,4-Dinitrophenyl)-butan-1,3-dione, as a brown solid, NMR (CDCl₃) 2.2 (s,3H) 5.8 (s,1H) 7.7 (d,1H) 8.4 (dd,1H) 8.65 (d,1H), starting from t-butyl 2-(2,4-dinitrobenzoyl)-3-oxobutanoate.
1-(4-Methylsulphonyl-2-nitrophenyl)-butan-1,3-dione, as a light brown solid NMR (DMSO-d⁶) 2.54 (s,3H) 3.7 (s,3H) 6.4 (s,1H) 8.18 (d,1H) 8.58 (d,1H) 8.75 (s,1H), starting from t-butyl 2-(4-methylsulphonyl-2-nitrophenyl)-3-oxobutanoate.
4-Methyl-1-(2-nitro-4-trifluoromethylphenyl)-pentan-1,3-dione, as an off-white solid by chromatography in a mixture of ethyl acetate and n-hexane (1:3) mp 44-45°C, starting from t-butyl 4-methyl-2-(2-nitro-4-trifluoromethylphenyl)-3-oxopentanoate.
1-(2-Nitro-4-trifluoromethylphenyl)-pentan-1,3-dione as an off-white solid by chromatography in a mixture of ethyl acetate and n-hexane (1:5) mp 45-46°C, starting from t-butyl 2-(2-nitro-4-trifluoromethylbenzoyl)-3-oxopentanoate.
1-(4-Chloro-2-nitrophenyl)-butan-1,3-dione, as a light brown solid, NMR (CDCl₃) 2.1 (s,3H) 5.68 (s,1H) 7.2-7.8 (m,3H) starting from t-butyl 2-(4-Chloro -2-nitrobenzoyl)-3-oxobutanoate.
1-(2-Nitro-4-trifluoromethylphenyl)-hexan-1,3-dione as an orange oil NMR (CDCl₃) 0.95 (t,3H) 1.6 (m,2H) 2.3 (t,2H) 5.7 (s,1H) 7.55 (d,1H) 7.8 (d,1H) 8.0 (s,1H) 15.2 (bs,1H) starting from t-butyl 2-(2-nitro-4-trifluoromethylbenzoyl)-3-oxohexanoate.
3-Cyclopropyl-1-(2-nitro-4-trifluoromethylphenyl)-propan-1,3-dione, as an off-white solid, mp 95-96°C starting from t-butyl 3-cyclopropyl-2-(2-nitro-4-trifluoromethylbenzoyl)-3-oxopropionate.
1-(2,3-Dichloro-4-methylsulphonylphenyl)-butan-1,3-dione as a light brown solid, mp 135-136°C starting from t-butyl 2-(2,3-dichloro-4-methylsulphonyl-benzoyl)-3-oxobutanoate.
4,4-Dimethyl-1-(2-nitro-4-trifluoromethylphenyl)-pentan-1,3-dione, as a buff solid, mp 60-61°C, starting from t-butyl 4,4-dimethyl-2-(2-nitro-4-trifluoromethylbenzoyl)-3-oxopentanoate.
1-(4-Methyl-2-nitrophenyl)-butan-1,3-dione, as an orange solid, mp 68-69°C starting from t-butyl 2-(4-methyl-2-nitrobenzoyl)-3-oxobutanoate.
1-(2,3-Dichloro-4-methylsulphonylphenyl)-4-methylpentan-1,3-dione as a white solid, mp 120-121°C, starting from t-butyl 2-(2,3-dichloro-4-methylsulphonylbenzoyl)-4-methyl-3-oxopentanoate.
3-Cyclopropyl-1-(2,3-dichloro-4-methylsulphonylphenyl)-propan-1,3-dione, as an off white solid, mp 132-134°C starting from t-butyl 3-cyclopropyl-2-(2,3-dichloro-4-methylsulphonylbenzoyl)-3-oxopropionate.
1-(2-Nitro-4-trifluoromethylphenyl)-4-phenylbutan-1,3-dione as a brown oil NMR (CDCl₃) 3.7 (s,2H) 5.7 (s,1H) 7.2 (s,5H) 7.45 (d,1H) 7.75 (d,1H) 8.0 (s,1H) 12.4-12.9 (bs,1H) starting from t-butyl 2-(2-nitro-4-trifluoromethylbenzoyl)-3-oxo-4-phenylbutanoate.
1-(2-Chloro-4-trifluoromethylphenyl)-3-cyclopropylpropan-1,3-dione as a crude brown oil which was not further purified, starting from t-butyl 2-(2-chloro-4-trifluoromethylbenzoyl)-3-cyclopropyl-3-oxopropionate.
1-(2-Nitro-4-pentafluoroethylphenyl)-butan-1,3-dione as an off-white solid, mp 103-104.8°C, starting from t-butyl 2-(2-nitro-4-pentafluoroethylbenzoyl)-3-oxobutanoate.
3-Cyclopropyl-1-[4-(1,1-dimethylethyl)-2-nitrophenyl]-propan-1,3-dione as a crude brown solid which was not further purified, starting from t-butyl 3-cyclo-propyl-2-[4-(1,1-dimethylethyl)-2-nitrobenzoyl]-3-oxopropionate.
1-[4-(1,1-dimethylethyl)-2-nitrophenyl]-butan-1,3-dione, as a yellow gum NMR (CDCl₃) 1.4 (s,9H) 2.1 (s,3H) 5.7 (s,1H) 7.35 (d,1H) 7.55 (dd,1H) 7.75 (d,1H) starting from t-butyl 2-[4-(1,1-dimethylethyl)-2-nitrobenzoyl]-3-oxobutanoate.
3-Cyclopentyl-1-(2-nitro-4-trifluoromethylphenyl)-propan-1,3-dione, as an off-white solid, mp 64-65°C starting from t-butyl 3-cyclopentyl-2-(2-nitro-4-trifluoromethylbenzoyl)-3-oxopropionate.
1-(2-Chloro-4-methylsulphonylphenyl)-butan-1,3-dione as a cream solid, mp 125.8°C, starting from t-butyl 2-(2-chloro-4-methylsulphonylbenzoyl)-3-oxobutanoate.
1-(2-Chloro-4-trifluoromethylphenyl)-butan- 1,3-dione, as a yellow gum, NMR (CDCl₃) 2.2 (s,3H) 5.9 (s, 1H) 7.3-7.7 (m,3H) starting from t-butyl 2-(2-chloro-4-trifluoromethylbenzoyl)-3-oxobutanoate.
1-(2-Trifluoromethylphenyl)-butan-1,3-dione, as a yellow oil NMR (CDCl₃) 2.15 (s,3H) 5.7 (s,1H) 7.6-8.4 (m,4H), starting from t-butyl 3-oxo-(2-trifluoromethylbenzoyl)-butanoate.
1-(2,4-Bis-trifluoromethylphenyl)-butan-1,3-dione as a yellow solid, mp 39.6°C, starting from t-butyl 2-(2,4-bis-trifluoromethylbenzoyl)-3-oxobutanoate.
1-(2-Chloro-4-methylsulphonylphenyl)-3-cyclopropylpropan-1,3-dione, as an off-white solid, mp 93.1°C, starting from t-butyl 2-(2-chloro-4-methylsulphonyl-benzoyl)-3-cyclopropyl-3-oxopropionate.
3-Cyclopropyl-1-(2-trifluoromethylphenyl)-propan-1,3-dione as an orange oil NMR (CDCl₃) 0.8-1.4 (m,4H) 1.5-1.9 (m,1H) 5.8 (s,1H) 8.0-8.6 (m,4H) starting from t-butyl 3-cyclopropyl-3-oxo-2-(2-trifluoromethylbenzoyl)-propionate.
3-Cyclopropyl-1-(2,4-dichlorophenyl)-propan-1,3-dione as an off-white solid by recrystallization from cyclohexane mp 62.3°C starting from t-butyl 3-cyclopropyl-2(2,4-dichlorobenzoyl)-3-oxopropionate.
1-[2,3-Dichloro-4-(methylthio)-phenyl]-butan-1,3-dione as a brown solid mp 103.5°C, starting from t-butyl 2-[2,3-dichloro-4-(methylthio)-benzoyl]-3-oxobutanoate.
1-(2,4-Bis-trifluoromethylphenyl)-3-cyclopropylpropan-1,3-dione as a white solid, mp 40°C, starting from t-butyl 2-(2,4-bis-trifluoromethylbenzoyl)-3-cyclopropyl-3-oxopropionate.
1-(4-Chloro-2-trifluoromethylphenyl)-butan-1,3-dione as a crude yellow gum which was not further purified, starting from t-butyl 2-(4-chloro-2-trifluoro-methylbenzoyl)-3-oxobutanoate.
1(4-Cyano-2-nitrophenyl)-butan-1,3-dione as an off-white solid by chromatography in a mixture of ethyl acetate and n-hexane (1:1) NMR (CDCl₃) 2.1 (s,3H) 5.7 (s,1H) 7.6 (d,1H) 7.9 (d,1H) 8.15 (s,1H) 14.6-15.1 (bs,1H), starting from t-butyl 2-(4-cyano-2-nitrobenzoyl)-3-oxobutanoate.
1-(4-Chloro-2-trifluoromethylphenyl)-3-cyclopropylpropan-1,3-dione as a crude yellow gum which was not further purified, starting from t-butyl 2-(4-chloro-2-trifluoromethylbenzoyl)-3-cyclopropyl-3-oxopropionate.
4-Methyl-1-(2-nitro-4-pentafluoroethylphenyl)-pentan-1,3-dione as a crude orange oil which was not further purified, starting from t-butyl 4-methyl-2-(2-nitro-4-pentafluoroethylbenzoyl)-3-oxopentanoate.
1-(2-Chloro-4-methylsulphonylphenyl)-4-methylpentan-1,3-dione as a yellow oil NMR (CDCl₃) 1.15 (d,6H) 2.3-2.8 (m,1H) 3.0 (s,3H) 5.9 (s,1H) 6.7 (m,2H) 6.85 (s,1H) starting from t-butyl 2-(2-chloro-4-methylsulphonylbenzoyl)-4-methyl-3-oxopentanoate.
3-Cyclopropyl-1-(4-fluoro-2-nitrophenyl)-propan-1,3-dione as a brown solid, mp 75.1°C, starting from t-butyl 3-cyclopropyl-2-(4-fluoro-2-nitrobenzoyl)-3-oxopropionate.
3-Cyclopropyl-1-(2-nitro-4-pentafluoroethylphenyl)-propan-1,3-dione as a crude brown solid which was not further purified, starting from t-butyl 3-cyclo-propyl-2-(2-nitro-4-pentafluoroethylbenzoyl)-3-oxopropionate.
3-Cyclobutyl-1-(2-nitro-4-trifluoromethylphenyl)propan-1,3-dione as a crude orange oil which was not further purified, starting from t-butyl 3-cyclobutyl-2-(2-nitro-4-trifluoromethylbenzoyl)-3-oxopropionate.
1-(4-Fluoro-2-nitrophenyl)-butan-1,3-dione, as a tan solid, mp 98.0°C starting from t-butyl 2-(4-fluoro-2-nitrobenzoyl)-3-oxobutanoate.
3-(1-Methylcyclopropyl)-1-(2-nitro-4-trifluoromethylphenyl)-propan-1,3-dione as an off-white solid, mp 124.5-125°C, starting from t-butyl 3-(1-methyl-cyclopropyl)-2-(2-nitro-4-trifluoromethylbenzoyl)-3-oxopropionate.
1-(2-Chloro-3-ethoxy-4-methylsulphonylphenyl)-butan-1,3-dione, as a light brown solid, mp 120-122°C, starting from t-butyl 2-(2-chloro-3-ethoxy-4-methyl-sulphonyl-benzoyl)-3-oxobutanoate.
1-(3-Cyanophenyl)-butan-1,3-dione as an off-white solid, mp 72.8°C, starting from t-butyl 2-(3-cyanobenzoyl)-3-oxobutanoate.
3-Cyclopropyl-1-(4-methylsulphonyl-2-trifluoromethylphenyl)-propan-1,3-dione, as a crude brown solid which was not further purified, starting from t-butyl 3-cyclopropyl-2-(4-methylsulphonyl-2-trifluoromethylbenzoyl)-3-oxopropionate.
3-Cyclopropyl-1-(4-methylsulphonyl-2-nitrophenyl)-propan-1,3-dione, as a cream solid, mp 179.6-181.6°C, starting from t-butyl 3-cyclopropyl-2-(4-methyl-sulphonyl-2-nitrobenzoyl)-3-oxopropionate.
1-(2-chloro-3-ethoxy-4-methylsulphonylphenyl)-3-cyclopropylpropan-1,3-dione as an orange gum, NMR (CDCl₃) 1.25 (m,4H) 1.6 (t,2H) 3.3 (m,3H) 4.35 (q,2H) 6.05 (s,1H) 7.4 (d,1H) 7.9 (d,1H) 14.5-15.5 (bs,1H) starting from t-butyl 2-(2-chloro-3-ethoxy-4-methylsulphonylbenzoyl)-3-cyclopropyl-3-oxopropionate.
1-(2-Chloro-3-ethoxy-4-ethylsulphonylphenyl)-butan-1,3-dione, as a yellow oil NMR (CDCl₃) 1.2-1.9 (m,6H) 2.3 (s,3H) 3.55 (q,2H) 4.4 (q,2H) 6.0 (s,1H) 7.45 (d,1H) 7.95 (d,1H), starting from t-butyl 2-(2-chloro-3-ethoxy-4-ethyl-sulphonylbenzoyl)-3-oxobutanoate.
1-(2-Chloro-3-ethoxy-4-methylsulphonylphenyl)-4-methylpentan-1,3-dione, as a yellow gum, NMR (CDCl₃) 1.1 (d,6H) 1.5 (t,3H) 2.4 (m,1H) 3.2 (s,3H) 4.2 (q,2H) 5.35 (s,1H) 7.25 (d,1H) 7.65 (d,1H), starting from t-butyl 2-(2-chloro-3-ethoxy-4-methylsulphonylbenzoyl)-4-methyl-3-oxopentanoate.
1-(2-Chloro-3-ethoxy-4-ethylsulphonylphenyl)-3-cyclopropylpropan-1,3-dione as a yellow oil NMR (CDCl3) 1.2-2.3 (m,11H) 3.75 (q,2H) 4.6 (q,2H) 6.3 (s,1H) 7.2 (d,1H) 8.2 (d,1H), starting from t-butyl 2-(2-chloro-3-ethoxy-4-ethylsulphonylbenzoyl)-3-cyclopropyl-3-oxopropionate.
3-(1-Ethoxycarbonylcyclopropyl)-1-(2-nitro-4-trifluoromethylphenyl) propan-1,3-dione, as a white solid by chromatography in a mixture of ethyl acetate, n-hexane and acetic acid (20:80:1) NMR (CDCl₃) 1.25 (t,3H) 1.7 (m,4H) 4.1 (q,2H) 6.6 (s,1H) 7.7 (m,2H) 8.0 (s,1H) 14.2-15.0 (bs,1H), starting from t-butyl 3-(1-ethoxycarbonylcyclopropyl)-2-(2-nitro-4-trifluoromethylbenzoyl)-3-oxopropionate.
1-(3-Methoxycarbonyl-2-methyl-4-methylsulphonylphenyl)-butan-1,3-dione, as an orange oil, NMR (CDCl₃) 2.15 (s,3H) 2.4 (s,3H) 3.1 (s,3N) 3.9 (s,3H) 5.7 (s,1H) 7.45 (d,1H) 7.8 (d,1H), starting from t-butyl 2-(3-methoxycarbonyl-2-methyl-4-methylsulphonylbenzoyl)-3-oxobutanoate.
3-(2-methylcyclopropyl)-1-(2-nitro-4-trifluoromethylphenyl)-propan-1,3-dione as a white solid, mp 70.4-72.6, starting from t-butyl 3-(2-methyl-cyclopropyl)-2-(2-nitro-4-trifluoromethylbenzoyl)-3-oxopropionate.
1-[2-Chloro-3-(1-methylethoxy)-4-methylsulphonylphenyl]-butan-1,3-dione as a white solid, NMR (CDCl₃) 1.4 (d,6H) 2.2 (s,3H) 3.2 (s,3H) 5.2 (m,1H) 5.8 (s,1H) 7.2 (d,1H) 8.2 (d,1H) starting from t-butyl 2-[2-chloro-3-(1-methyl-ethoxy)-4-methylsulphonylbenzoyl]-3-oxobutanoate.
1-[2-Methyl-3-(1-methylethoxycarbonyl)-4-methylsulphonylphenyl]-butan-1,3-dione as an orange oil NMR (CDCl₃) 1.65 (d,6H) 2.4 (s,3H) 2.7 (s,3H) 3.4 (s,3H) 5.5 (m,1H) 5.9 (s,1H) 7.65 (d,1H) 8.05 (d,1H), starting from t-butyl 2-[2-methyl-3-(1-methylethoxycarbonyl)-4-methylsulphonylbenzoyl]-3-oxobutanoate.
3-Cyclopropyl-1-[2-methyl-3-(1-methylethoxycarbonyl)-4-methylsulphonyl-phenyl]-propan-1,3-dione as an orange oil NMR (CDCl₃) 1.4 (m,4H) 1.65 (d,6H) 2.7 (s,3H) 3.4 (s,3H) 5.5 (m,1H) 6.0 (s,1H) 7.65 (d,1H) 8.0 (d,1H), starting from t-butyl 3-cyclopropyl-2[2-methyl-3-(1-methylethoxycarbonyl)-4-methyl-sulphonylbenzoyl]-3-oxopropionate.
3-Cyclopropyl-1-(2,3,4-trichlorophenyl)-propan-1,3-dione as a dark brown oil, NMR (CDCl₃) 1.2 (m,4H) 1.8 (s,1H) 5.95 (s,1H) 7.3 (s,2H) 14.6-15.5 (bs,1H) starting from t-butyl 3-cyclopropyl-3-oxo-2-(2,3,4-trichlorophenyl)-propionate.

### REFERENCE EXAMPLE 3.

A mixture of magnesium turnings (4.8g) and carbon tetrachloride (2ml) in pure ethanol (30ml) was stirred and warmed gently to 50°C until the reaction was initiated (effervescence observed). Ether (100ml was added cautiously with stirring. A solution of t-butyl 3-oxobutanoate (31.6g) in ether (100ml) was added dropwise at such a rate as to maintain the mixture at reflux. Stirring and heating at reflux was continued for 2h. A solution of 2-nitro-4-trifluoromethylbenzoyl chloride (50.7g) in ether (100ml) was added dropwise and the resultant solution was stirred and heated at reflux for 2.5h. The cooled reaction mixture was treated with hydrochloric acid (2M, 100ml) with stirring and the two layers were separated. The organic phase was extracted with aqueous sodium hydroxide solution (2M, 2 x 50ml) and water (4 x 50ml). The combined aqueous layers were acidified to pH 1 and extracted with ether (2 x 100ml). The combined organic layers were washed with water (50ml), dried (anhydrous sodium sulphate) and filtered. The filtrate was evaporated to dryness to give t-butyl 2-(2-nitro-4-trifluoromethylbenzoyl)-3-oxobutanoate (62.2g) as a crude red oil which was not further purified.

By proceeding in a similar manner, the following compounds were prepared:
t-butyl 4methyl-2-(2-nitro-4-trifluoromethylbenzoyl)-3-oxopentanoate as a crude orange oil, starting from t-butyl 4-methyl-3-oxopentanoate.
t-butyl 2-(2-nitro-4-trifluoromethylbenzoyl)-3-oxopentanoate as a crude orange oil starting from t-butyl 3-oxopentanoate.
t-butyl 2-(2-nitro-4-trifluoromethylbenzoyl)-3-oxohexanoate as a crude orange oil, starting from t-butyl 3-oxohexanoate.
t-butyl 3-cyclopropyl-2-(2-nitro-4-trifluoromethylbenzoyl)-3-oxopropionate as a crude orange oil, starting from t-butyl 3-cyclopropyl-3-oxopropionate.
t-butyl 4,4-dimethyl-2-(2-nitro-4-trifluoromethylbenzoyl)-3-oxopentanoate, as a crude orange oil, starting from t-butyl 4,4-dimethyl-3-oxopentanoate.
t-butyl 2-(2-nitro-4-fluoromethylbenzoyl)-3-oxo-4-phenylbutanoate as a crude orange oil, starting from t-butyl 3-oxo-4-phenylbutanoate.
t-butyl 2-(2-nitro-4-pentafluoroethylbenzoyl)-3-oxobutanoate as a crude brown oil, starting from t-butyl 3-oxobutanoate and 2-nitro-4-pentafluoroethylbenzoyl chloride.
t-butyl 3-cyclopentyl-2-(2-nitro-4-trifluoromethylbenzoyl)-3-oxopropionate as a crude orange oil, starting from t-butyl 3-cyclopentyl-3-oxopropionate.
t-butyl 2-(4-cyano-2-nitrobenzoyl)-3-oxobutanoate as a crude brown gummy solid, starting from t-butyl 3-oxobutanoate and 4-cyano-2-nitrobenzoyl chloride.
t-butyl 4-methyl-2-(2-nitro-4-pentafluoroethylbenzoyl)-3-oxopentanoate as a crude brown oil, starting from t-butyl 4-methyl-3-oxopentanoate and 2-nitro-4-pentafluoroethylbenzoyl chloride.
t-butyl 3-cyclopropyl-2-(2-nitro-4-pentafluoroethylbenzoyl)-3-oxopropionate, as a crude brown oil, starting from t-butyl 3-cyclopropyl-3-oxopropionate and 2-nitro-4-pentafluoroethylbenzoyl chloride.
t-butyl 3-cyclobutyl-2-(2-nitro-4-trifluoromethylbenzoyl)-3-oxopropionate as a crude orange oil, starting from t-butyl 3-cyclobutyl-3-oxopropionate.
t-butyl 3-(1-methylcyclopropyl)-2-(2-nitro-4-trifluoromethylbenzoyl)-3-oxopropionate, as a crude orange oil, starting from t-butyl 3-(1-methylcyclopropyl)-3-oxopropionate.
t-butyl 3-(1-ethoxycarbonylcyclopropyl)-2-(2-nitro-4-trifluoromethylbenzoyl)-3-oxopropionate as a crude white solid, starting from t-butyl 3-(1-ethoxycarbonylcyclopropyl)-3-oxopropionate.
t-butyl 3-(2-methylcyclopropyl)-2-(2-nitro-4-trifluoromethylbenzoyl)-3-oxopropionate as a crude orange oil, starting from t-butyl 3-(2-methylcyclopropyl)-3-oxopropionate.
t-butyl 3-cyclopropyl-3-oxo-2-(2,3,4-trichlorobenzoyl)-propionate, as a crude brown solid, starting from t-butyl 3-cyclopropyl-3-oxopropionate, and 2,3,4-trichlorobenzoyl chloride.

### REFERENCE EXAMPLE 4.

Carbon tetrachloride (1ml) was added to a stirred mixture of t-butyl 3-oxobutanoate (7.0g) and magnesium (1.0g) in methanol (30ml) causing a vigorous reaction. After subsidence of the reaction the mixture was stirred for 1/4h and evaporated to dryness. The residue was dissolved in dry ether (70ml) and a solution of 2,4-dinitrobenzoyl chloride (10.0g) in dry ether (30ml) was added dropwise. The mixture was stirred and heated at reflux for 2h. After cooling to room temperature, hydrochloric acid (2M, 75ml) was added.

The layers were separated and the aqueous layer was extracted with ether (2 x 50ml). The combined organic layers were extracted into aqueous sodium hydroxide (2M, 2 x 50ml) and water (3 x 50ml). The combined aqueous extracts were acidified to pH 1 and extracted with ether (2 x 75ml). The combined organic extracts were washed with water, dried (anhydrous magnesium sulphate) and filtered. The flltrate was evaporated to dryness to give t-butyl 2-(2,4-dinitrobenzoyl)-3-oxobutanoate (15.4g) as an orange gum which was not further purified.

By proceeding in a similar manner, the following compounds were prepared:
t-butyl 2-(4-methylsulphonyl-2-nitrobenzoyl)-3-oxobutanoate as a crude orange gum, starting from t-butyl 3-oxobutanoate and 4-methylsulphonyl-2-nitro-benzoyl chloride.
t-butyl 2-(4-chloro-2-nitrobenzoyl)-3-oxobutanoate as a crude orange gum, starting from t-butyl 3-oxobutanoate and 4-chloro-2-nitrobenzoyl chloride and replacing the dry ether by acetonitrile.
t-butyl 2-(2,3-dichloro-4-methylsulphonylbenzoyl)-3-oxobutanoate as a crude clear gum, starting from t-butyl 3-oxobutanoate and 2,3-dichloro-4- methyl-sulphonylbenzoyl chloride and replacing the dry ether by dichloromethane.
t-butyl 2-(4-methyl-2-nitrobenzoyl)-3-oxobutanoate as a crude brown oil, starting from t-butyl 3-oxobutanoate and 4-methyl-2-nitrobenzoyl chloride.
t-butyl 2-(2,3-dichloro-4-methylsulphonylbenzoyl)-4-methyl-3-oxopentanoate as a crude white gum, starting from t-butyl 4-methyl-3-oxopentanoate and 2,3-dichloro-4-methylsulphonylbenzoyl chloride.
t-butyl 3-cyclopropyl-2-(2,3-dichloro-4-methylsulphonylbenzoyl)-3-oxo-propionate as a crude orange gum, starting from t-butyl 3-cyclopropyl-3-oxo-propionate and 2,3-dichloro-4-methylsulphonylbenzoyl chloride and replacing the dry ether by dichloromethane.
t-butyl 2-(2-chloro-4-trifluoromethylbenzoyl)-3-cyclopropyl-3-oxopropionate as a crude black gum, starting from t-butyl 3-cyclopropyl-3-oxopropionate and 2-chloro-4-trifluoromethylbenzoyl chloride.
t-butyl 3-cyclopropyl-2-[4-(1,1-dimethylethyl)-2-nitrobenzoyl]-3-oxopropionate as a crude red gum, starting from t-butyl 3-cyclopropyl-3-oxopropionate and 4-(1,1-dimethylethyl)-2-nitrobenzoyl chloride.
t-butyl 2-[4-(1,1-dimethylethyl)-2-nitrobenzoyl]-3-oxobutanoate as a crude yellow gum, starting from t-butyl 3-oxobutanoate and 4-(1,1-dimethylethyl)-2-nitrobenzoyl chloride.
t-butyl 2-(2-chloro-4-methylsulphonylbenzoyl)-3-oxobutanoate as a crude brown oil, starting from t-butyl 3-oxobutanoate and 2-chloro-4-methylsulphonyl-benzoyl chloride and replacing the dry ether by dichloromethane.
t-butyl 2(2-chloro-4-trifluoromethylbenzoyl)-3-oxobutanoate as a crude dark yellow oil, starting from t-butyl 3-oxobutanoate and 2-chloro-4-trifluoro-methylbenzoyl chloride.
t-butyl 3-oxo-2-(2-trifluoromethylbenzoyl)-butanoate as a crude yellow oil, starting from t-butyl 3-oxobutanoate and 2-trifluoromethylbenzoyl chloride.
t-butyl 2-(2,4-bis-trifluoromethylbenzoyl)-3-oxobutanoate as a crude yellow oil, starting from t-butyl 3-oxobutanoate and 2,4-bis-trifluoromethylbenzoyl chloride.
t-butyl 2-(2-chloro-4-methylsulphonylbenzoyl)-3-cyclopropyl-3-oxopropionate as a crude yellow oil, starting from t-butyl 3-cyclopropyl-3-oxopropionate and 2-chloro-4-methylsulphonylbenzoyl chloride and replacing the dry ether by dichloromethane.
t-butyl 3-cyclopropyl-3-oxo-2-(2-trifluoromethylbenzoyl)-propionate as a crude orange gum, starting from t-butyl 3-cyclopropyl-3-oxopropionate and 2-trifluoromethylbenzoyl chloride.
t-butyl 3-cyclopropyl-2-(2,4-dichlorobenzoyl)-3-oxopropionate as a crude brown oil, starting from t-butyl 3-cyclopropyl-3-oxopropionate and 2,4-dichlorobenzoyl chloride.
t-butyl 2-[2,3-dichloro-4-(methylthio)-benzoyl]-3-oxobutanoate as a crude brown oil, starting from t-butyl 3-oxobutanoate and 2,3-dichloro-4-(methyl-thio)-benzoyl chloride.
t-butyl 2-(2,4-bis-trifluoromethylbenzoyl)-3-cyclopropyl-3-oxopropionate as a crude yellow oil, starting from t-butyl 3-cyclopropyl-3-oxopropionate and 2,4-bis-trifluoromethylbenzoyl chloride.
t-butyl 2-(4-chloro-2-trifluoromethylbenzoyl)-3-oxobutanoate as a crude yellow gum, starting from t-butyl 3-oxobutanoate and 4-chloro-2-trifluoro-methylbenzoyl chloride.
t-butyl 2-(4-chloro-2-trifluoromethylbenzoyl)-3-cyclopropyl-3-oxopropionate as a crude yellow solid starting from t-butyl 3-cyclopropyl-3-oxopropionate and 4-chloro-2-trifluoromethylbenzoyl chloride.
t-butyl 2-(2-chloro-4-methylsulphonylbenzoyl)-4-methyl-3-oxopentanoate, as a crude brown oil, starting from t-butyl 4-methyl-3-oxopentanoate and 2-chloro-4-methylsulphonylbenzoyl chloride, and replacing the dry ether by dichloro-methane.
t-butyl 3-cyclopropyl-2-(4-fluoro-2-nitrobenzoyl)-3-oxopropionate as a crude brown oil starting from t-butyl 3-cyclopropyl-3-oxopropionate and 4-fluoro-2-nitrobenzoyl chloride.
t-butyl 2-(4-fluoro-2-nitrobenzoyl)-3-oxobutanoate as a crude brown oil, starting from t-butyl 3-oxobutanoate and 4-fluoro-2-nitrobenzoyl chloride.
t-butyl 2-(2-chloro-3-ethoxy-4-methylsulphonylbenzoyl)-3-oxobutanoate as a crude orange oil starting from t-butyl 3-oxobutanoate and 2-chloro-3-ethoxy-4-methylsulphonylbenzoyl chloride.
t-butyl 2-(3-cyanobenzoyl)-3-oxobutanoate as a crude orange gum, starting from t-butyl 3-oxobutanoate and 3-cyanobenzoyl chloride.
t-butyl 3-cyclopropyl-2-(4-methylsulphonyl-2-trifluoromethylbenzoyl)-3-oxopropionate as a crude red oil, starting from t-butyl 3-cyclopropyl-3-oxo propionate and 4-methylsulphonyl-2-trifluoromethylbenzoyl chloride and replacing the dry ether by dichloromethane.
t-butyl 3-cyclopropyl-2-(4-methylsulphonyl-2-nitrobenzoyl)-3-oxopropionate as a crude brown oil, starting from t-butyl 3-cyclopropyl-3-oxopropionate and 4-methylsulphonyl-2-nitrobenzoyl chloride and replacing the dry ether by acetonitrile.
t-butyl 2-(2-chloro-3-ethoxy-4-methylsulphonylbenzoyl)-3-cyclopropyl-3-oxopropionate as a crude orange gum starting from t-butyl 3-cyclopropyl-3-oxopropionate and 2-chloro-3-ethoxy-4-methylsulphonylbenzoyl chloride and replacing the dry ether by dichloromethane.
t-butyl 2-(2-chloro-3-ethoxy-4-ethylsulphonylbenzoyl)-3-oxobutanoate as a crude brown gum starting from t-butyl 3-oxobutanoate and 2-chloro-3-ethoxy-4-ethylsulphonylbenzoyl chloride and replacing the dry ether by acetonitrile.
t-butyl 2-(2-chloro-3-ethoxy-4-methylsulphonylbenzoyl)-4-methyl-3-oxo-pentanoate as a crude yellow oil, starting from t-butyl 4-methyl-3-oxopentanoate and 2-chloro-3-ethoxy-4-methylsulphonylbenzoyl chloride and replacing the dry ether by acetonitrile.
t-butyl 2-(2-chloro-3-ethoxy-4-ethylsulphonylbenzoyl)-3-cyclopropyl-3-oxopropionate as a crude brown oil, starting from t-butyl 3-cyclopropyl-3-oxopropionate and 2-chloro-3-ethoxy-4-ethylsulphonylbenzoyl chloride and replacing the dry ether by acetonitrile.
t-butyl 2-(3-methoxycarbonyl-2-methyl-4-methylsulphonylbenzoyl)-3-oxobutanoate as a crude orange oil, starting from t-butyl 3-oxobutanoate and 3-methoxycarbonyl-2-methyl-4-methylsulphonylbenzoyl chloride and replacing the dry ether by toluene.
t-butyl 2-[2-chloro-3-(1-methylethoxy)-4-methylsulphonylbenzoyl]-3-oxobutanoate as a crude orange gum, starting from t-butyl 3-oxobutanoate and 2-chloro-3-(1-methylethoxy)-4-methylsulphonylbenzoyl chloride and replacing the dry ether by acetonitrile.
t-butyl 2-[2-methyl-3-(1-methylethoxycarbonyl)-4-methylsulphonylbenzoyl]-3-oxobutanoate as a crude orange oil, starting from t-butyl 3-oxobutanoate and 2-methyl-3-(1-methylethoxycarbonyl)-4-methylsulphonylbenzoyl chloride and replacing the dry ether by acetonitrile.
t-butyl-3-cyclopropyl-2-[2-methyl-3-(methylethoxycarbonyl)-4-methylsulphonylbenzoyl]-3-oxopropionate as a crude orange oil, starting from t-butyl 3-cyclopropyl-3-oxopropionate and 2-methyl-3-(1-methylethoxycarbonyl)-4-methylsulphonylbenzoyl chloride and replacing the dry ether by acetonitrile.

### REFERENCE EXAMPLE 5

A mixture of 5-cyclopropylcarbonyl-2,2-dimethyl-1,3-dioxan-4,6-dione (23.9g) and t-butanol (25g) in dry toluene (80ml) was stirred and heated at 80°C for 4h. The cooled mixture was washed with water (3 x 30ml), dried (anhydrous sodium sulphate), treated with decolourizing charcoal and filtered. The filtrate was evaporated to dryness and the residue was distilled to give t-butyl 3-cyclopropyl-3-oxopropionate (20.2g) as a clear oil bp 80-84°C/8mmHg.

By proceeding in a similar manner the following compounds were prepared:
t-butyl 3-cyclopentyl-3-oxopropionate as an orange oil NMR (CDCl₃) 1.5 (s,9H) 1.7 (m,8H) 2.9 (m,1H) 3.3 (s,2H), starting from 5-cyclopentylcarbonyl-2,2-dimethyl-1,3-dioxan-4,6-dione.
t-butyl 3-cyclobutyl-3-oxopropionate as a clear oil bp 66-80°C/12mmHg, starting from 5-cyclobutylcarbonyl-2,2-dimethyl-1,3-dioxan-4,6-dione.
t-butyl 3-(2-methylcyclopropyl)-3-oxopropionate as a clear oil bp 100-110°C/16mmHg, starting from 2,2-dimethyl-5-(2-methylcyclopropylcarbonyl)-1,3-dioxan-4,6-dione.

### REFERENCE EXAMPLE 6

A mixture of 2,2-dimethyl-1,3-dioxan-4,6-dione (20.0g) and pyridine (22.0g) in dichloromethane (200ml) was stirred and cooled to 0°C in an ice bath. A solution of cyclopropylcarbonyl chloride (16.0g) in dichloromethane (50ml) was added dropwise with stirring in an atmosphere of nitrogen whilst maintaining the temperature below 3°C by external cooling. The mixture was stirred at 0°C for 1h and at ambient temperature for 2h. The resultant orange suspension was washed with hydrochloric acid (2M, 2 x 50ml), water (2 x 50ml), dried (anhydrous sodium sulphate) and filtered. The filtrate was evaporated to dryness. The residue was dissolved in ether (150ml) and the solution was treated with decolourizing charcoal and filtered. The filtrate was evaporated to dryness to give 5-cyclopropylcarbonyl-2,2-dimethyl-1,3-dioxan-4,6-dione (24.2g) as a yellow solid mp 44-46°C.

By proceeding in a similar manner the following compounds were prepared:
5-cyclopentylcarbonyl-2,2-dimethyl-1,3-dioxan-4,6-dione as an orange solid, mp 75-76°C, starting from cyclopentylcarbonyl chloride.
5-cyclobutylcarbonyl-2,2-dimethyl-1,3-dioxan-4,6-dione as an orange oil which was not further purified, starting from cyclobutylcarbonyl chloride.
2,2-dimethyl-5-(2-methylcyclopropylcarbonyl)-1,3-dioxan-4,6-dione as an orange oil which was not further purified, starting from 2-methylcyclopropylcarbonyl chloride.

### REFERENCE EXAMPLE 7

A solution of n-butyllithium (2.5M in hexane, 36ml) was added dropwise with stirring to a cooled solution of diisopropylamine (9.09g) in dry tetrahydrofuran (80ml) in an atmosphere of nitrogen, whilst maintaining the temperature below -70°C. The mixture was stirred for 1/4h and t-butyl trimethylsilylacetate (16.9g) was added dropwise with stirring whilst maintaining the temperature below -70°C. The mixture was stirred at -78°C for 1h. A suspension of 1-(1-methylcyclopropylcarbonyl)-imidazole (13.5g) in dry tetrahydrofuran (120ml) was added dropwise whilst maintaining the temperature below -60°C. The mixture was stirred at -78°C for 3h and the temperature was allowed to rise to room temperature. Hydrochloric acid (2M, 100ml) was added cautiously and the mixture was extracted with ether (2 x 100ml). The combined extracts were washed with hydrochloric acid (2M, 3 x 50ml) and water (3 x 50ml), dried (anhydrous sodium sulphate) and filtered. The filtrate was evaporated to dryness to give t-butyl 3-(1-methylcyclopropyl)- 3-oxopropionate as an orange oil NMR (CDCl₃) 0.65 (m,2H) 1.1 (m,2H) 1.25 (s,3H) 1.4 (s,9H) 3.2(s,2H).

### REFERENCE EXAMPLE 8

A solution of 1-methylcyclopropylcarbonyl chloride (11.8g) in toluene (15ml) was added dropwise to a solution of imidazole (13.6g) in tetrahydrofuran (100ml) whilst maintaining the temperature below 25°C. The mixture was stirred for 3h and filtered. The filtrate was evaporated to dryness to give 1-(1-methylcyclopropylcarbonyl)-imidazole (13.6g) as a white solid, mp 34-35°C.

### REFERENCE EXAMPLE 9

A solution of n-butyllithium (2.5M in hexane, 40ml) was added dropwise with stirring to a cooled solution of diisopropylamine (10.1g) in anhydrous ether (100ml) in an atmosphere of nitrogen, whilst maintaining the temperature below -70°C. The mixture was stirred at -78°C for 1/2h and a solution of t-butyl acetate (11.6g) in anhydrous ether (20ml) was added dropwise, whilst maintaining the temperature below -70°C. The mixture was stirred at -78°C for 1h and a solution of diethyl cyclopropane-1,1-dicarboxylate (18.6g) in anhydrous ether (20ml) was added dropwise whilst maintaining the temperature below -70°C. The mixture was stirred at -78°C for 1/2h and the temperature was allowed to rise to room temperature. A saturated solution of ammonium chloride (150ml) was added cautiously and the layers were separated. The organic extract was washed with water (2 x 50ml) hydrochloric acid (2M, 2 x 50ml) and water (2 x 50ml). The solution was dried (anhydrous sodium sulphate) and filtered. The filtrate was evaporated to dryness and the residue was distilled to give t-butyl 3-(1-ethoxycarbonylcyclopropyl)-3-oxopropionate (11.66g) as a clear oil bp 89-90°C/0.5mmHg.

### REFERENCE EXAMPLE 10

A solution of 2-(2-nitro-4-trifluoromethylbenzoyl)-1-phenylbutan-1,3-dione (28.5g) in ethanol (300ml) was added to a mixture of concentrated sulphuric acid (225ml) and ethanol (225ml). The resultant solution was stirred and heated at 100°C for 1h. The cooled solution was poured onto a mixture of ice and water (21) and extracted with dichloromethane (3 x 250ml). The combined organic layers were washed with water (2 x 100ml), dried (anhydrous sodium sulphate) and filtered. The filtrate was evaporated to dryness and the residue was recrystallized from petroleum spirit (bp 60-80°C) to give 1-(2-nitro-4-trifluoromethylphenyl)-3-phenylpropan-1,3-dione (19.6g) as an off-white solid mp 101-103°C.

By proceeding in a similar manner, the following compounds were prepared:
1-(2-Nitro-4-trifluoromethylphenyl)-3-(4-chlorophenyl)-propan-1,3-dione, as a yellow solid mp 133-134°C, starting from 2-(2-nitro-4-trifluoromethyl-benzyl)-1-(4-chlorophenyl)-butan-1,3-dione.
1-(2-Nitro-4-trifluoromethylphenyl)-3-(4-fluorophenyl)-propan-1,3-dione, as a yellow solid mp 128-130°C, starting from 2-(2-nitro-4-trifluoromethyl-benzoyl)-1-(4-fluorophenyl)-butan-1,3-dione.

### REFERENCE EXAMPLE 11

A mixture of magnesium turning (3.0g) and carbon tetrachloride (0.5ml) in ethanol (40ml) was warmed gently to 50°C until the reaction initiated (effervescence observed). Ether (100ml) was added followed by dropwise addition of a solution of 1-phenylbutan-1,3-dione (20,0g) in ether (100ml). The mixture was heated at reflux for 3h. The cooled mixture was evaporated to dryness and the residue was treated with toluene (100ml) and re-evaporated. The residue was suspended in ether (150ml) and a solution of 2-nitro-4-trifluoromethylbenzoyl chloride (32.8g) was added. The mixture was stirred and heated at reflux for 2h. After cooling, hydrochloric acid (2M, 100ml) was added and the two layers were separated. The organic layer was extracted with aqueous sodium bicarbonated (saturated, 5 x 100ml). The combined aqueous layers were acidified to pH1 and extracted with dichloromethane (2 x 150ml). The combined organic layers were washed with water (2 x 50ml), dried (anhydrous sodium sulphate) and filtered. The filtrate was evaporated to dryness to give 2-(2-nitro-4-trifluoromethylbenzoyl)-1-phenylbutan-1,3-dione (35.6g) as a brown oil, NMR (CDCl₃) 2.15 (s,3H) 5.25 (s,1H) 7.1-8.0 (m,7H) 8.2 (s,1H) 16.5 (bs,1H).

By proceeding in a similar manner, the following compound was prepared:
2-(2-Nitro-4-trifluoromethylbenzoyl)-1-(4-fluorophenyl)-butan-1,3-dione, as a white solid mp 90-91°C, starting from 1-(4-fluorophenyl)-butan-1,3-dione.

### REFERENCE EXAMPLE 12

A mixture of magnesium turnings (0.71g) in methanol (20ml) was stirred and heated at reflux for 1h until all of the magnesium was consumed. A solution of 1-(2-nitro-4-trifluoromethylphenyl)-butan-1,3-dione (8.0g) in methanol (100ml) was added dropwise at such a rate as to maintain gentle reflux. The mixture was stirred and heated at reflux for 2h. The cooled, cloudy solution was evaporated to dryness and the residue was dissolved in toluene (50ml) and re-evaporated. The residue was redissolved in toluene (100ml) and a solution of 4-chlorobenzoyl chloride (5.12g) in toluene (20ml) was added. The mixture was stirred at ambient temperature for 16h. Hydrochloric acid (2M, 50ml) was added and the layers were separated. The organic layer was washed with water (50ml), dried (anhydrous sodium sulphate) and filtered. The filtrate was evaporated to dryness. The residue was triturated with a mixture of ether and petroleum spirit (bp 60-80°C) (1:1) and the solid was filtered off to give 2-(2-nitro-4-trifluoromethylbenzoyl)-1-(4-chlorophenyl)-butan-1,3-dione (5.5g) as an off-white solid mp 102.5-103.5°C.

### REFERENCE EXAMPLE 13

A mixture of magnesium turnings (0.62g) and carbon tetrachloride (1ml) in anhydrous methanol (120ml) was stirred and heated at reflux for 1/2h until all of the magnesium was consumed. A solution of 1-(2-nitro-4-trifluoromethylphenyl)-butan-1,3-dione (7.0g) in methanol (90ml) was added dropwise and the mixture was stirred and heated at reflux for 2h. The mixture was cooled and evaporated to dryness. The residue was dissolved in toluene (50ml) and re-evaporated to dryness. The residue was dissolved in acetonitrile (100ml) and to this was added dropwise a solution of 4-nitrobenzoyl chloride (9.28g) in acetonitrile (40ml). The mixture was stirred and heated at 70°C for 48h. After cooling, hydrochloric acid (2M, 50ml) was added and the mixture was extracted with ethyl acetate (50ml). The organic layer was extracted with aqueous sodium hydroxide (2M, 2 x 50ml) and water (5 x 60ml). The combined aqueous extracts were acidified to pH1 and extracted with ethyl acetate (2 x 75ml). The combined organic extracts were washed with water (2 x 50ml), dried (anhydrous magnesium sulphate) and filtered. The filtrate was evaporated to dryness and triturated with ether (20ml), to give 3-(4-nitrophenyl)-1-(2-nitro-4-trifluoromethyl-phenyl)-propan-1,3-dione (2.89g) as a yellow solid, mp 139-140°C.

By proceeding in a similar manner the following compound was prepared:
3-(4-Methoxyphenyl)-1-(2-nitro-4-trifluoromethylphenyl)-propan-1,3-dione, as a yellow solid mp 127-129°C, starting from 4-methoxybenzoyl chloride.

### REFERENCE EXAMPLE 14

A solution of 5-(2-nitro-4-trifluoromethylphenyl)-isoxazole (4.91g) in ethanol (35ml) was added dropwise to a solution of sodium ethoxide in ethanol (prepared from 0.6g sodium in 25ml ethanol). The mixture was stirred at room temperature for 3h, poured into water (150ml) and acidified to pH1. It was extracted with ether (2 x 75ml) and the combined extracts were washed with water, dried (anhydrous sodium sulphate) and filtered. The filtrate was evaporated to dryness and the residue was triturated with petroleum spirit (bp 60-80°C) and filtered to give 3-(2-nitro-4-trifluoromethylphenyl)-3-oxo-propionitrile (4.9g) as an off-white solid mp 79.5-80.5°C.

### REFERENCE EXAMPLE 15

5-Hydroxy-5-(2-nitro-4-trifluoromethylphenyl)-isoxazoline (8.47g) was added in portions to concentrated sulphuric acid (85ml). The mixture was stirred for 2h and poured into a mixture of ice and water (200ml). It was extracted with ether (2 x 100ml) and the combined organic extracts were washed with water (2 x 75ml), dried (anhydrous sodium sulphate) and filtered. The filtrate was evaporated to dryness and the residue was triturated with petroleum spirit (bp 60-80°C) and filtered to give 5-(2-nitro-4-trifluoro-methylphenyl)-isoxazole (7.45g) as a white solid, mp 62-63°C.

### REFERENCE EXAMPLE 16

A mixture of 3-dimethylamino-1-(2-nitro-4-trifluoromethylphenyl)-prop-2-en-1-one (41.0g) and hydroxylamine hydrochloride (11.9g) in dry ethanol (280ml) was stirred at room temperature overnight. It was evaporated to dryness and the residue was dissolved in a mixture of ether (150ml) and water (100ml). The layers were separated and the organic layer was washed with water (100ml), dried (anhydrous sodium sulphate) and filtered. The filtrate was evaporated to dryness and the residue was triturated with petroleum spirit (bp 60-80°C) (20ml) and filtered to give 5-hydroxy-5-(2-nitro-4-trifluoromethylphenyl)-isoxazoline (33.9g) as a pale yellow solid mp 122-123°C.

### REFERENCE EXAMPLE 17

A mixture of 2-nitro-4-trifluromethylacetophenone (40.0g) and dimethyl-formamide dimethyl acetal (89.0g) was stirred and heated at reflux for 2 1/2h. The cooled dark red solution was evaporated to dryness and the residue was triturated with ether (30ml) and filtered to give 3-dimethylamino-1-(2-nitro-4-trifluoromethylphenyl)-prop-2-en-1-one (41.5g) as a bright orange solid mp 100.8-101.3°C.

### REFERENCE EXAMPLE 18

Diethyl (2-nitro-4-trifluoromethylbenzoyl)-malonate (90.75g) was added portionwise to a mixture of concentrated sulphuric acid (38ml), glacial acetic acid (315ml) and water (215ml) and the resultant mixture was stirred and heated at reflux for 2h. After cooling the mixture was basified by the addition of aqueous sodium hydroxide (2M) and extracted with ether (3 x 250ml). The combined organic extracts were washed with water, dried (anhydrous sodium sulphate) and filtered. The filtrate was evaporated to dryness and the residue was triturated with petroleum spirit (bp 60-80°C) (50ml) and filtered to give 2-nitro-4-trifluoroacetophenone (48.6g) as an off-white solid mp 67.2-67.7°C.

### REFERENCE EXAMPLE 19

A mixture of magnesium turnings (6.1g) and carbon tetrachloride (2ml) in ethanol (40ml) was stirred and warmed gently until the reaction initiated (effervescence observed). Ether (150ml) was added cautiously followed by dropwise addition of a solution of diethyl malonate (40.2g) in ether (200ml). The mixture was stirred and heated at reflux for 1h until all of the magnesium was consumed. The solution was cooled to room temperature and a solution of 2-nitro-4-trifluoromethylbenzoyl chloride (63.4g) in ether (200ml) was added dropwise. The mixture was stirred and heated at reflux for 2h. The cooled suspension was treated with hydrochloric acid (2M, 200ml) and the layers were separated. The organic layer was extracted with aqueous sodium hydroxide solution (2M, 2 x 100ml) and water (3 x 100ml). The combined aqueous layers were acidified to pH1 and extracted with ether (2 x 150ml). The combined organic layers were washed with water (2 x 75ml) dried (anhydrous sodium sulphate) and filtered. The filtrate was evaporated to give diethyl 2-nitro-4-trifluoromethylbenzoylmalonate (90.75g) as a white solid mp 59-60°C.

Most of the benzoyl chlorides used in the reference examples above are described in the literature, however those which are not, are prepared by the standard reaction of the benzoic acids with thionyl chloride. The solutions were evaporated to dryness and the residual benzoyl chlorides used without purification.

### REFERENCE EXAMPLE 20

1-Nitro-4-pentafluoroethyltoluene (56.6g) and pyridine (170ml) were added to a stirred solution of sodium hydroxide (10.0g) in water (500ml). The mixture was stirred and heated at reflux and potassium permanganate (174g) was added portionwise. The mixture was stirred and heated at reflux for 1h until the purple colouration had disappeared. After cooling, the mixture was filtered and the solid was washed with water (3 x 100ml) and ether (300ml). The layers were separated and the aqueous layer was washed with ether and acidified to pH1. It was extracted with ether (3 x 300ml) and the combined extracts were washed with water, dried (anhydrous magnesium sulphate) and filtered. The filtrate was evaporated to dryness to give 2-nitro-4-penta-fluoroethylbenzoic acid (51.6g) as a white solid mp 124-133°C.

### REFERENCE EXAMPLE 21

A mixture of concentrated nitric acid (1ml) and concentrated sulphuric acid (2ml) was added to a cooled stirred suspension of 4-pentafluoroethyltoluene (2.1 g) in concentrated sulphuric acid (5ml) whilst maintaining the temperature below 5°C. The mixture was stirred at 0°C for 10 min, allowed to warm to room temperature and stirred for 1 1/2h. It was poured onto ice (30ml) and extracted with ether (2 x 15ml). The combined organic extracts were washed with aqueous sodium carbonate (2M, 2 x 25ml), water (25ml), saturated aqueous sodium chloride solution (25ml), dried (anhydrous magnesium sulphate) and filtered. The filtrate was evaporated to dryness to give 2-nitro-4-penta-fluoroethyltoluene (2.04g) as a pale yellow oil NMR (CDCl₃) 2.7 (s,3H) 7.45 (d,1H) 7.7 (d,1H) 8.1 (s,1H).

The preparation of 4-pentafluoroethyltoluene is described by J N Freskos, Synth. Comm. 1988,18 965. He states that the product cannot be separated from toluene by distillation. However distillation through a packed column gives pure pentafluoroethyltoluene bp 137-141°C.

### REFERENCE EXAMPLE 22

Hydrogen peroxide (30%, 12.5ml) was added dropwise with stirring to a cooled solution of 3-methoxycarbonyl-1-methyl-4-(methylthio)-benzoic acid (4.2g) in a mixture of acetic anhydride (2.5ml) and acetic acid (10ml) whilst maintaining the temperature below 5°C. The mixture was stirred at 0°C for 20 min and allowed to warm to room temperature. The mixture was stirred at room temperature for 1/2h and heated at 65°C for 2h. The cooled solution was diluted with water (50ml) and extracted with ethyl acetate (3 x 50ml). The combined extracts were washed with saturated aqueous sodium chloride solution (50ml), aqueous ferrous sulphate solution (3 x 50ml) and saturated aqueous sodium chloride solution (2 x 50ml). The organic layer was extracted into aqueous sodium carbonate solution (1M, 3 x 50ml). The combined aqueous extracts were acidified to pH1 and extracted with ether (3 x 50ml). The combined organic extracts were washed with water (2 x 50ml), dried (anhydrous magnesium sulphate) and filtered. The filtrate was evaporated to dryness to give 3-methoxycarbonyl-2-methyl-4-methylsulphonylbenzoic acid (2.0g) as a pale yellow solid, mp 113-118°C.

By proceeding in a similar manner the following compound was prepared:
2-methyl-3-(1-methylethoxycarbonyl)-4-methylsulphonylbenzoic acid as a cream solid NMR (CD₃CN) 1.6 (d,6H) 2.7 (s,3H) 3.3 (s,3H) 5.4 (m,1H) 8.0 (m,2H), starting from 2-methyl-3-(1-methylethoxycarbonyl)-4-(methylthio)benzoic acid.

### REFERENCE EXAMPLE 23

Sodium nitrite (2.4g) was added dropwise to a stirred solution of 3-methoxycarbonyl-2-methyl-4-(methylthio)-benzamide (4.2g) in a mixture of concentrated sulphuric acid (50ml), water (40ml) and glacial acetic acid (70ml) whilst maintaining the temperature below 5°C. The mixture was stirred without cooling for 1/2 h. The brown solution was recooled to 0°C and water (200ml) was added. The mixture was extracted with ether (3 x 150ml). The combined organic extracts were washed with water (2 x 150ml), dried (anhydrous magnesium sulphate) and filtered. The filtrate was evaporated to dryness to give 3-methoxycarbonyl-2-methyl-4-(methylthio)-benzoic acid (4.6g) as a sticky orange solid NMR (CDCl₃) 2.1 (s,3H) 2.5 (s,3H) 3.9 (s,3H) 7.0 (d,1H) 7.85 (d,1H) 9.0 (bs,1H).

By proceeding in a similar manner the following compound was prepared:
2-methyl-3-(1-methylethoxycarbonyl)-4-(methylthio)-benzoic acid as an orange gum which was not further purified, starting from 2-methyl-3-(1-methyl-ethoxycarbonyl)-4-(methylthio)-benzamide.

### REFERENCE EXAMPLE 24

Hydrogen peroxide (30%, 18ml) was added dropwise with stirring to a solution of methyl 3-cyano-2-methyl-6-(methylthio)-benzoate (10.0g) in ethanol (100ml) in an atmosphere of nitrogen. A solution of sodium hydroxide (0.43g) in water (2ml) was added dropwise and the resultant rapid exotherm was controlled by ice cooling. After the exotherm had ceased the mixture was stirred and heated at 65°C for 1/2 h. The orange solution was poured into a mixture of ice and water (400ml) and it was extracted with ether (3 x 100ml). The combined organic extracts were washed with water (100ml), aqueous ferrous sulphate solution (100ml), water (2 x 100ml), dried (anhydrous magnesium sulphate) and filtered. The filtrate was evaporated to dryness to give 3-methoxycarbonyl-2-methyl-4-(methylthio)-benzamide (4.2g) as an orange oil, NMR (CDCl₃) 2.3 (s,3H) 2.4 (s,3H) 3.9 (s,3H) 6.5 (bs,2H) 6.95 (d,1H) 7.25 (d,1H).

By proceeding in a similar manner, the following compound was prepared:
2-methyl-3-(1-methylethoxycarbonyl)-4-(methylthio)-benzamide, as an orange oil which was not further purified, starting from 1-methylethyl 3-cyano-2-methyl-6-(methylthio)-benzoate.

### REFERENCE EXAMPLE 25

A mixture of methyl 3-iodo-2-methyl-6-(methylthio)-benzoate (16.6g) and cuprous cyanide (4.4g) in dimethyl formamide (50ml) was stirred and heated at 150°C for 1h. The mixture was cooled to 90°C and a solution of ferric chloride (18g) in water (28ml) and concentrated hydrochloric acid (5ml) was added. The mixture was stirred and heated at 90°C for 1h. The cooled mixture was filtered and the solid was washed with ether. The layers in the filtrate were separated and the aqueous layer was extracted with ether (100ml). The combined organic layers were washed with water (2 x 100ml) aqueous sodium sulphate solution (10%, 2 x 100ml) water (2 x 100ml) aqueous sodium hydroxide solution (2 x 100ml), water (3 x 100ml), dried (anhydrous magnesium sulphate) and filtered. The filtrate was evaporated to dryness to give methyl 3-cyano-2-methyl-6-(methylthio)-benzoate (10.0g) as a brown oil NMR (CDCl₃) 2.5 (s,6H) 3.9 (s,3H) 7.05 (d,1H) 7.45 (d,1H).

By proceeding in a similar manner, the following compound was prepared:
1-methylethyl 3-cyano-2-methyl-6-(methylthio)-benzoate as an orange gum NMR (CDCl₃) 1.3 (d,6H) 2.35 (s,6H) 5.0 (m,1H) 6.7-7.2 (m,2H) starting from 1-methylethyl 3-iodo-2-methyl-6-(methylthio)-benzoate.

According to a feature of the present invention, there is provided a method for controlling the growth of weeds (i.e. undesired vegetation) at a locus which comprises applying to the locus a herbicidally effective amount of at least one isoxazole derivative of general formula (III). For this purpose, the isoxazole derivatives are normally used in the form of herbicidal compositions (i.e. in association with compatible diluents or carriers and/or surface active agents suitable for use in herbicidal compositions), for example as hereinafter described, and those compositions are also part of the invention.

The compounds of general formula (III) show herbicidal activity against dicotyledonous (ie. broad-leafed) and monocotyledonous (e.g. grass) weeds by pre- and/or, post-emergence application.

By the term "pre-emergence application" is meant application to the soil in which the weed seeds or seedlings are present before emergence of the weeds above the surface of the soil. By the term "post-emergence application" is meant application to the aerial or exposed portions of the weeds which have emerged above the surface of the soil. For example, the compounds of general formula (III) may be used to control the growth of broad-leafed weeds, for example, Aethusa cynapium, Abutilon theophrasti, Amaranthus retraflexus, Amsinckia intermedia, Anagallis arvensis, Anthemis arvensis, Atriplex patula, Bidens pilosa, Brassica nigra, Capsella bursa-pastoris, Chenopodium album, Chrysanthemum segetum, Cirsium arvense, Datura stramonium, Desmodium tortuosum, Emex australia, Euphorbia helioscopia, Fumaria officinalis, Galeopsis tetrahit, Galium aparine, Geranium dissectum, Ipomea purpurea, Lamium purpureum, Lapsana communis, Matricaria inodora, Monochoria vaginalis, Papaver rhoeas, Physalis longifolia, Plantago lanceolata, Polygonum spp., (e.g. Polygonum lapathifolium, Polygonum aviculare, Polygonum convolvulus and Polygonum persicaria), Portulaca oleracea, Raphanus raphanistrum, Rotala indica, Rumex obtusifolius, Saponaria vaccaria, Scandix pecten-veneris, Senecio vulgaris, Sesbania florida, Sida spinosa, Silene alba, Sinapis arvensis, Solanum nigrum, Sonchus arvensis, Spergula arvensis, Stellaria media, Thlaspi arvense, Tribulus terrestria, Urtica urens, Veronica hederifolia, Veronica persica, Viola arvensis and Xanthium strumarium,and grass weeds, for example, Alopecurus myosuroides, Apera spica-venti, Agrostis stolonifera, Avena fatua, Avena ludoviciana, Brachiaria spp., Bromus sterilis, Bromus tectorum, Cenchrus spp., Cynodon dactylon, Digitaria sanguinalis, Echinochloa crus-galli, Eleusine indica, Setaria viridis and Sorghum halepense and sedges, for example Cyperus.esculentus, Cyperus iria and Cyperus rotundus, and Eleocharis acicularis.

The amounts of compounds of general formula (III) applied vary with the nature of the weeds, the compositions used, the time of application, the climatic and edaphic conditions and (when used to control the growth of weeds in crop-growing areas), the nature of the crops. When applied to a crop-growing area, the rate of application should be sufficient to control the growth of weeds without causing substantial permanent damage to the crop. In general, taking these factors into account, application rates between 0.01 kg and 20 kg of active material per hectare give good results. However, it is to be understood that higher or lower application rates may be used, depending upon the particular problem of weed control encountered.

The compounds of general formula (III) may be used to control selectively the growth of weeds, for example to control the growth of those species hereinbefore mentioned, by pre- or post-emergence application in a directional or non-directional fashion, e.g. by directional or non-directional spraying, to a locus of weed infestation which is an area used, or to be used, for growing crops, for example cereals, e.g. wheat, barley, oats, maize and rice, soya beans, field and dwarf beans, peas, lucerne, cotton, peanuts, flax, onions, carrots, cabbage, oilseed rape, sunflower, sugar beet, and permanent or sown grassland before or after sowing of the crop or before or after emergence of the crop. For the selective control of weeds at a locus of weed infestation which is an area used, or to be used, for growing of crops, e.g. the crops hereinbefore mentioned, application rates between 0.01 kg and 8.0 kg, and preferably between 0.01 kg and 4.0 kg, of active material per hectare are particularly suitable.

The compounds of general formula (III) may also be used to control the growth of weeds, especially those indicated above, by pre- or post-emergence application in established orchards and other tree-growing areas, for example forests, woods and parks, and plantations e.g.sugar cane, oil palm and rubber plantations. For this purpose they may be applied in a directional or non-directional fashion (e.g. by directional or non-directional spraying) to the weeds or to the soil in which they are expected to appear, before or after planting of the trees or plantations at application rates between 0.25 kg and 10.0 kg, and preferably between 0.5 kg and 8.0 kg, of active material per hectare.

The compounds of general formula (III) may also be used to control the growth of weeds, especially those indicated above, at loci which are not crop-growing areas but in which the control of weeds is nevertheless desirable. Examples of such non-crop-growing areas include airfields, industrial sites, railways, roadside verges, the verges of rivers, irrigation and other waterways, scrublands and fallow or uncultivated land, in particular where it is desired to control the growth of weeds in order to reduce fire risks. When used for such purposes in which a total herbicidal effect is frequently desired, the active compounds are normally applied at dosage rates higher than those used in crop-growing areas as hereinbefore described. The precise dosage will depend upon the nature of the vegetation treated and the effect sought. Pre- or post-emergence application, and preferably pre-emergence application, in a directional or non-directional fashion (e.g. by directional or non-directional spraying) at application rates between 1.0 kg and 20.0 kg, and preferably between 5.0 and 10.0 kg, of active material per hectare are particularly suitable for this purpose.

When used to control the growth of weeds by pre-emergence application, the compounds of general formula (III) may be incorporated into the soil in which the weeds are expected to emerge. It will be appreciated that when the compounds of general formula (III) are used to control the growth of weeds by post-emergence application, i.e. by application to the aerial or exposed portions of emerged weeds, the compounds of general formula (III) will also normally come into contact with the soil and may also then exercise a pre-emergence control on later-germinating weeds in the soil.

Where especially prolonged weed control is required, the application of the compounds of general formula (III) may be repeated if required.

According to a further feature of the present invention, there are provided compositions suitable for herbicidal use comprising one or more of the isoxazole derivatives of general formula (III) in association with, and preferably homogeneously dispersed in, one or more compatible herbicidally-acceptable diluents or carriers and/or surface active agents [i.e. diluents or carriers and/or surface active agents of the type generally accepted in the art as being suitable for use in herbicidal compositions and which are compatible with compounds of general formula (III)]. The term "homogeneously dispersed" is used to include compositions in which the compounds of general formula (III) are dissolved in other components. The term "herbicidal compositions" is used in a broad sense to include not only compositions which are ready for use as herbicides but also concentrates which must be diluted before use. Preferably, the compositions contain from 0.05 to 90% by weight of one or more compounds of general formula (III).

The herbicidal compositions may contain both a diluent or carrier and surface-active (e.g. wetting, dispersing, or emulsifying) agent. Surface-active agents which may be present in herbicidal compositions of the present invention may be of the ionic or non-ionic types, for example sulphoricinoleates, quaternary ammonium derivatives, products based on condensates of ethylene oxide with alkyl and polyaryl phenols, e.g. nonyl-or octyl-phenols, or carboxylic acid esters of anhydrosorbitols which have been rendered soluble by etherification of the free hydroxy groups by condensation with ethylene oxide, alkali and alkaline earth metal salts of sulphuric acid esters and sulphonic acids such as dinonyl-and dioctyl-sodium sulphonosuccinates and alkali and alkaline earth metal salts of high molecular weight sulphonic acid derivatives such as sodium and calcium lignosulphonates and sodium and calcium alkylbenzene sulphonates.

Suitably, the herbicidal compositions according to the present invention may comprise up to 10%, e.g. from 0.05% to 10%, of surface-active agent but, if desired, herbicidal compositions according to the present invention may comprise higher proportions of surface-active agent, for example up to 15% in liquid emulsifiable suspension concentrates and up to 25% in liquid water soluble concentrates.

Examples of suitable solid diluents or carriers are aluminium silicate, talc, calcined magnesia, kieselgur, tricalcium phosphate, powdered cork, adsorbent carbon black and clays such as kaolin and betonite. The solid compositions (which may take the form of dusts, granules or wettable powders) are preferably prepared by grinding the compounds of general formula (III) with solid diluents or by impregnating the solid diluents or carriers with solutions of the compounds of general formula (III) in volatile solvents, evaporating the solvents and, if necessary, grinding the products so as to obtain powders. Granular formulations may be prepared by absorbing the compounds of general formula (III) (dissolved in suitable solvents, which may, if desired, be volatile) onto the solid diluents or carriers in granular form and, if desired, evaporating the solvents, or by granulating compositions in powder form obtained as described above. Solid herbicidal compositions, particularly wettable powders and granules, may contain wetting or dispersing agents (for example of the types described above), which may also, when solid, serve as diluents or carriers.

Liquid compositions according to the invention may take the form of aqueous, organic or aqueous-organic solutions, suspensions and emulsions which may incorporate a surface-active agent. Suitable liquid diluents for incorporation in the liquid compositions include water, glycols, tetrahydrofurfuryl alcohol, acetophenone, cyclohexanone, isophorone, toluene, xylene, mineral, animal and vegetable oils and light aromatic and naphthenic fractions of petroleum (and mixtures of these diluents). Surface-active agents, which may be present in the liquid compositions, may be ionic or non-ionic (for example of the types described above) and may, when liquid, also serve as diluents or carriers.

Powders, dispersible granules and liquid compositions in the form of concentrates may be diluted with water or other suitable diluents, for example mineral or vegetable oils, particularly in the case of liquid concentrates in which the diluent or carrier is an oil, to give compositions ready for use. When desired, liquid compositions of the compound of general formula (III) may be used in the form of self-emulsifying concentrates containing the active substances dissolved in the emulsifying agents or in solvents containing emulsifying agents compatible with the active substances, the simple addition of water to such concentrates producing compositions ready for use.

Liquid concentrates in which the diluent or carrier is an oil may be used without further dilution using the electrostatic spray technique.

Herbicidal compositions according to the present invention may also contain, if desired, conventional adjuvants such as adhesives, protective colloids, thickeners, penetrating agents, stabilisers, sequestering agents, anti-caking agents, colouring agents and corrosion inhibitors. These adjuvants may also serve as carriers or diluents.

Preferred herbicidal compositions according to the present invention are:
. aqueous suspension concentrates which comprise from 10 to 70% of one or more compounds of general formula (III), from 2 to 10% of surface-active agent, from 0.1 to 5% of thickener and from 15 to 87.9% of water;
. wettable powders which comprise from 10 to 90% of one or more compounds of general formula (III), from 2 to 10% of surface-active agent and from 8 to 88% of solid diluent or carrier;
. soluble powders which comprise from 10 to 90% of one or more compounds of general formula (III), from 2 to 40% of sodium carbonate and from 0 to 88% of solid diluent;
. liquid water soluble concentrates which comprise from 5 to 50%, e.g. 10 to 30%, of one or more compounds of general formula (III), from 5 to 25% of surface-active agent and from 25 to 90%, e.g. 45 to 85%, of water miscible solvent, e.g. dimethylformamide, or a mixture of water-miscible solvent and water;
. liquid emulsifiable suspension concentrates which comprise from 10 to 70% of one or more compounds of general formula (III), from 5 to 15% of surface-active agent, from 0.1 to 5% of thickener and from 10 to 84.9% of organic solvent;
. granules which comprise from 1 to 90%, e.g. 2 to 10% of one or more compounds of general formula (III), from 0.5 to 7%, e.g 0.5 to 2%, of surface-active agent and from 3 to 98.5%, e.g 88 to 97.5%, of granular carrier and emulsifiable concentrates which comprise 0.05 to 90%, and preferably from 1 to 60% of one or more compounds of general formula (III), from 0.01 to 10%, and preferably from 1 to 10%, of surface-active agent and from 9.99 to 99.94%, and preferably from 39 to 98.99%, of organic solvent.

Herbicidal compositions according to the present invention may also comprise the compounds of general formula (III) in association with, and preferably homogeneously dispersed in, one or more other pesticidally active compounds and, if desired, one or more compatible pesticidally acceptable diluents or carriers, surface-active agents and conventional adjuvants as hereinbefore described. Examples of other pesticidally active compounds which may be included in, or used in conjunction with, the herbicidal compositions of the present invention include herbicides, for example to increase the range of weed species controlled, for example alachlor [2-chloro-2′,6′-diethyl-N-(methoxymethyl)-acetanilide], asulam [methyl(4-aminobenzenesulphonyl)carbamate], alloxydim Na [sodium salt of 2-(1-allyloxy-aminobutylidene)-5,5-dimethyl-4-methoxycarbonylcyclohexane-1,3-dione], atrazine [2-chloro-4-ethylamino-6-isopropylamino-1,3,5-triazine], barban [4-chlorobut-2-ynyl N-(3-chlorophenyl)carbamate], benzoylprop-ethyl [ethyl N-benzoyl-N-(3,4-dichlorophenyl-2-aminopropionate], bromoxynil [3,5-dibromo-4-hydroxybenzonitrile], butachlor [N-(butoxymethyl)-2-chloro-2′,6′-diethylacetanilide], butylate [S-ethyl N,N-diisobutyl(thiocarbamate)], carbetamide [D-N-ethyl-2-(phenylcarbamoxyloxy)propionamide], chlorfenpropmethyl[methyl 2-chloro-3-(4-chloro-phenyl)propionate], chlorpropham [isopropyl N-(3-chlorophenyl)carbamate], chlortoluron [N′-(3-chloro-4-methylphenyl)-N,N-dimethylurea], cyanazine [2-chloro-4-(1-cyano-1-methylethylamino)-6-ethylamino-1,3,5-triazine], cycloate [N′-cyclohexyl-N-ethyl-S-ethyl(thiocarbamate)], 2,4-D [2,4-dichlorophenoxyacetic acid], dalapon [2,2-dichloropropionic acid], 2,4-DB [4-(2,4-dichlorophenoxy)butyric acid], desmedipham [3-(ethoxycarbonylamino)phenyl N-phenyl-carbamate], diallate [S-2,3-dichloroallyl-N,N-di-isopropyl(thiocarbamate)], dicamba [3,6-dichloro-2-methoxybenzoic acid], dichlorprop [(±)-2-(2,4-dichlorophenoxy)propionic acid], difenzoquat [1,2-dimethyl-3,5-diphenyl-pyrazolium salts], dimefuron 4-[2-chloro-4-(3,3-dimethylureiodo)phenyl]-2-t-butyl-1,3,4-oxadiazolin-5-one, dinitramine [N¹,N¹-diethyl-2,6-dinitro-4-trifluoromethyl-m-phenylenediamine], diuron [N′-(3,4-dichlorophenyl)-N,N-dimethylurea], EPTC [S-ethyl N,N-dipropyl(thiocarbamate)], ethofumesate [2-ethoxy-2,3-dihydro-3,3-dimethylbenzofuran-5-yl methylsulphonate], flampropisopropyl [isopropyl (±)-2-(N-benzoyl-3-chloro-4-fluoroanilino)propionate], flampropmethyl [methyl (±)-2-(N-benzoyl-3-chloro-4-fluoroanilino)propionate], fluometuron [N′-(3-trifluoromethylphenyl)-N,N-dimethylurea], ioxynil [4-hydroxy-3,5-diiodobenzo-nitrile], isoproturon [N′-(4-isopropylphenyl)-N,N-dimethylurea], linuron [N-(3,4-dichlorophenyl-N-methoxy-N-methylurea], MCPA [4-chloro-2-methylphenoxyacetic acid, MCPB [4-(4-chloro-2-methylphenoxy)butyric acid], mecoprop [(±)-2-(4-chloro-2-methylphenoxy)propionic acid], metamitron [4-amino-3-methyl-6-phenyl-1,2,4-triazin-5(4H)-one], methabenzthiazuron [N-(benzothiazol-2-yl)-N,N′-dimethylurea], metribuzin [4-amino-6-t-butyl-3-(methylthio)-1,2,4-triazin-5(4H)-one], molinate [S-ethyl N,N-hexamethylene (thiocarbamate)], oxadiazon [3-(2,4-dichloro-5-isopropoxyphenyl)-5-t-butyl-1,3,4-oxadiazolin-2-one], paraquat [1,1′-dimethyl-4,4′-bipyridylium salts], pebulate [S-propyl N-butyl-N-ethyl(thiocarbamate)], phenmedipham [3-(methoxycarbonylamino)phenyl N-(3-methyl-phenyl) carbamate], prometryne [4,6-bisisopropylamino-2-methylthio-1,3,5-triazine], propachlor [2-chloro-N-isopropylacetanilide], propanil [N-(3,4-dichlorophenyl)-propionamide], propham [isopropyl N-phenylcarbamate], pyrazone [5-amino-4-chloro-2-phenylpyridazin-3(2H)-one], simazine [2-chloro-4,6-bisethylamino- 1,3,5-triazine], TCA (trichloroacetic acid), thiobencarb [S-(4-chloro-benzyl)-N,N-diethylthiolcarbamate], tri-allate [S-2,3,3-trichloroallyl N,N-diisopropyl(thiocarbamate)] and trifluralin [2,6-dinitro-N,N-dipropyl-4-trifluoromethylaniline] ; insecticides, e.g. carbaryl [naphth-1-yl N-methylcarbamate]; synthetic pyrethroids, e.g. permethrin and cypermethrin ; and fungicides, e.g. 2,6-dimethyl-4-tridecyl-morpholine, methyl N-(1-butyl-carbamoylbenzimidazol-2-yl)carbamate, 1,2-bis-(3-methoxycarbonyl-2-thioureido)benzene, isopropyl 1-carbamoyl-3-(3,5-dichlorophenyl)hydantoin and 1-(4-chloro-phenoxy)-3,3-dimethyl- 1-(1,2,4-triazol-1-yl)-butan-2-one. Other biologically active materials which may be included in, or used in conjunction with, the herbicidal compositions of the present invention are plant growth regulators, e.g.succinarnic acid, (2-chloroethyl)trimethylammonium chloride and 2-chloroethane-phosphonic acid; or fertilizers, e.g.containing nitrogen, potassium and phosphorus and trace elements known to be essential to successful plant life, e.g. iron, magnesium, zinc, maganese, cobalt and copper.

Pesticidally active compounds and other biologically active materials which may be included in, or used in conjunction with, the herbicidal compositions of the present invention, for example those herein before mentioned, and which are acids, may, if desired, be utilized in the form of conventional derivatives, for example alkali metal and amine salts and esters.

According to a further feature of the present invention there is provided an article of manufacture comprising at least one of the isoxazole derivatives of general formula (III) or, as is preferred, a herbicidal composition as hereinbefore described, and preferably a herbicidal concentrate which must be diluted before use, comprising at least one of the isoxazole derivatives of general formula (III) within a container for the aforesaid derivative or derivatives of general formula (III), or a said herbicidal composition, and instructions physically associated with the aforesaid container setting out the manner in which the aforesaid derivative or derivatives of general formula (III) or herbicidal composition contained therein is to be used to control the growth of weeds. The containers will normally be of the types conventionally used for the storage of chemical substances which are solid at normal ambient temperatures and herbicidal compositions particularly in the form of concentrates, for example cans and drums of metal, which may be internally-lacquered, and plastics materials, bottles of glass and plastics materials and, when the contents of the container is a solid, for example granular, herbicidal compositions, boxes, for example of cardboard, plastics materials and metal, or sacks. The containers will normally be of sufficient capacity to contain amounts of the isoxazole derivative or herbicidal compositions sufficient to treat at least 4,047 square metres (one acre) of ground to control the growth of weeds therein but will not exceed a size which is convenient for conventional methods of handling. The instructions will be physically associated with the container, for example by being printed directly thereon or on a label or tag affixed thereto. The directions will normally indicate that the contents of the container, after dilution if necessary, are to be applied to control the growth of weeds at rates of application between 0.01 kg and 20 kg of active material per hectare in the manner and for the purposes hereinbefore described.

The following Examples illustrate herbicidal compositions according to the present invention. The following trade marks are used in the description: Aerosil, Celite, Sopropon and Rhodigel:

### EXAMPLE C1

A wettable powder was formed from:-

| | |
|---|---|
| active ingredient (compound 1) | 50%w/w |
| Ethylan BCP (a nonylphenol/ethylene oxide condensate containing 9 moles of ethylene oxide per mol of phenol) | 5%w/w |
| Aerosil (silicon dioxide of microfine particle size) | 5%w/w |
| Celite PF (synthetic magnesium silicate carrier) | 40%w/w |

by absorbing the Ethylan BCP onto the Aerosil, mixing with the other ingredients and grinding the mixture in a hammer-mill to give a wettable powder.

Similar wettable powders may be prepared as described above by replacing the isoxazole (compound 1) by other compounds of general formula (III).

### EXAMPLE C2

An aqueous suspension concentrate was formed from:

| | |
|---|---|
| active ingredient (compound 1) | 50%w/v |
| Ethylan BCP | 1.0%w/v |
| Sopropon T36 (sodium salt of polycarboxylic acid) | 0.2%w/v |
| Ethylene glycol | 5%w/v |
| Rhodigel 23 (polysaccharide xanthan gum thickener) | 0.15%w/v |
| distilled water to | 100% by volume |

by intimately mixing the ingredients and grinding in a ball-mill for 24 hours.

Similar aqueous concentrates may be prepared as described above by replacing the isoxazole (compound 1) by other compounds of general formula (III).

Representative compounds of general formula (III) have been used in herbicidal applications or according to the following procedures.

### METHOD OF USE OF HERBICIDAL COMPOUNDS:

### HERBICIDAL ACTIVITY:

### a) General

Appropriate quantities of the test compounds were dissolved in acetone to give solutions equivalent to application rates of 1000 g, 2000 g or 4000 g of test compound per hectare(g/ha). These solutions were applied from a standard laboratory herbicide sprayer using flat fan jet travelling at 2.9 km/h, and delivering the equivalent of 540 litres of spray fluid per hectare.

### b) Weed Control: Pre-emergence application

Weed seeds were sown on the surface of John Innes No. 1 potting compost (7 parts by volume of sterilised loam, 3 parts by volume of peat and 2 parts by volume of fine grit) in 70 mm square, 75 mm deep plastic pots. The quantities of seed per pot were as follows:-

| Weed species | Approximate number seeds/pot |
|---|---|
| | |

| **1) Broad-leafed weeds.** | |
|---|---|
| Abutilon theophrasti | 10 |
| Sinapis arvensis | 20 |
| Chenopodium album | 60 |
| Ipomea purpurea | 10 |

| **2) Grass-weeds** | |
|---|---|
| Avena fatua | 15 |
| Echinochloa crus-galli | 20 |

| **3) Sedges** | |
|---|---|
| Cyperus esculentus | 3 |

The compounds of the invention were applied to the uncovered seeds as described in (a) above and the seeds were covered with 25 ml of sharp sand after spraying. A single pot of each weed species was allocated to each treatment with unsprayed controls and controls sprayed with acetone alone. After treatment, the pots were kept in the greenhouse and were watered overhead. Visual assessment of weed control activity was made 17-20 days after spraying. The results were expressed as the percentage reduction in growth or kill of the weeds, in comparison with the plants in the control pots.

### c) Weed control : Post-emergence application

Weed species were grown and then transplanted at the seedling stage into John Innes No. 1 potting compost in 70 mm square, 75 mm deep plastic pots, except for Avena fatua, which was sown directly in the test pot and not transplanted. The plants were then grown in the greenhouse until ready for spraying with the test compounds. The number of plants per pot, and the growth of the plant at spraying were as follows:-

| Weed Species | Number of plants per pot | Growth stages at spraying |
|---|---|---|
| | | |

| **1) Broad-leafed weeds** | | |
|---|---|---|
| Abutilon theophrasti | 3 | 1-2 leaves |
| Sinapis arvensis | 4 | 2 leaves |
| Chenopodium album | 4 | 2-4 leaves |
| Ipomea purpurea | 3 | 1-2 leaves |

| **2) Grass weeds** | | |
|---|---|---|
| Avena fatua | 15 | 1-2 leaves |
| Echinochloa crus-galli | 4 | 2-3 leaves |

| **3) Sedges** | | |
|---|---|---|
| Cyperus esculentus | 3 | 3 leaves |

The test compounds were applied to the plants as described in (a) above. A single pot of each weed species was allocated to each treatment, with unsprayed controls and controls sprayed with acetone alone. After spraying, the pots were watered overhead, commencing 24 hours after spraying. Visual assessment of the control of the growth of the weeds was made 17-20 days after spraying. The results were expressed as the percentage reduction in growth or kill of the weeds, in comparison with the plants in the control pots.

When applied pre-emergence at 4000g/ha compounds 1, 2, 4, 5, 6, 7, 8, 9, 11, 12, 13, 15, 16, 19, 22, 27, 28, 29, 30, 31, 32, 35, 39, 40, 42, 44 and 49 gave at least 90% reduction in growth of one or more of the weed species.

When applied post-emergence at 4000g/ha compounds 1, 2, 4, 7, 8, 9, 10, 11, 12, 13, 15, 16, 19, 22, 27, 28, 29, 30, 31, 32, 35, 39, 40, 42, 44, 49 and 53 gave at least 70% reduction in growth of one or more of the weed species.

When applied pre-emergence at 2000g/ha compounds 3, 14, 17, 18, 20, 21, 23, 24, 25, 33, 34, 36, 37, 38, 41, 43, 45, 46, 47, 48, 51 and 52 gave at least 90% reduction in growth of one or more of the weed species.

When applied post-emergence at 2000g/ha compounds 3, 14, 17, 18, 20, 21, 23, 24, 25, 26, 33, 34, 36, 37, 38, 41, 43, 45, 46, 48, 50 and 52 gave at least 70% reduction in growth of one or more of the weed species.

When applied pre-emergence at 1000g/ha compounds 54, 55, 56, 57, 58, 59, 60, 61, 62, 63, 64, 65, and 66 gave at least 80% reduction in growth of one or more of the weed species.

When applied post-emergence at 1000g/ha compounds 54, 55, 56, 57, 58, 59, 60, 61, 62, 63, 64, 65 and 66 gave at least 70% reduction in growth of one or more of the weed species.

## Claims (Claims for the following Contracting State(s): AT, BE, CH, DE, DK, FR, GB, GR, IT, LI, LU, NL, SE)

1. An isoxazole derivative characterized by the general formula III : 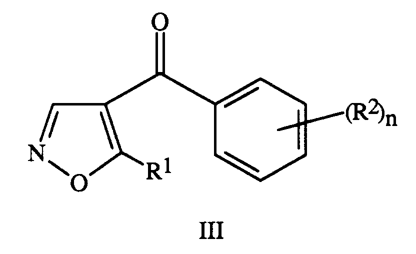 wherein R¹ represents :
a straight- or branched-chain alkyl, alkenyl, or alkynyl group containing up to 6 carbon atoms which is optionally substituted by one or more halogen atoms ;
a cycloalkyl group containing 3 to 6 carbon atoms optionally substituted by one or more R³ groups or one or more halogen atoms or an ester group, CO₂R³¹;
a cycloalkenyl group containing 5 or 6 carbon atoms optionally substituted by one or more R³ groups or one or more halogen atoms or an ester group, CO₂R³¹;
an aryl or aralkyl group;
a group, CO₂R³ ;
an aldehyde or acyl group, COR³;
a cyano group ;
a nitro group ;
an amino group, NR³R⁴ ; or
a halogen atom ;
R² represents :
a nitro group ;
a cyano group ; or
a halogen atom;
a group R⁵ ;
a sulphenyl, sulphinyl or sulphonyl group S(O)ₘR⁵ ;
a sulphamoyl group SO₂NR³R⁴ ;
a group CO₂R³ ;
an aldehyde or acyl group COR³ ;
a carbamoyl or thiocarbamoyl group CONR³R⁴ or CSNR³R⁴ ;
an alkoxy group OR⁵ ; or
an alkyl group (with 1 to 3 carbon atoms) substituted by OR⁵ ;
R³ and R⁴ which may be the same or different, each represents :
a hydrogen atom or a straight- or branched-chain alkyl group containing up to 6 carbon atoms which is optionally substituted by one or more halogen atoms ;
R³¹ is as defined for R³ but does not represent hydrogen;
R⁵ represents :
a straight- or branched-chain alkyl group containing up to 6 carbon atoms which is optionally substituted by one or more halogen atoms ;
m represents zero, 1 or 2;
n represents an integer from 1 to 5;
provided that
(a) when R¹ represents C₁₋₄ alkyl, n is an integer from one to three and R² is selected from the group consisting of halogen, C₁₋₄ alkyl, C₁₋₄ alkoxy, trifluoromethyl and trichloromethyl, the compound is not present in a mixture with a compound of formula:- 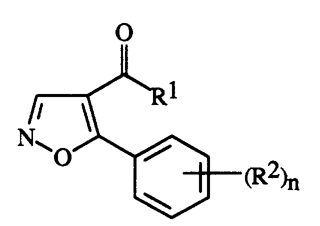 wherein R¹ is C₁₋₄ alkyl, n is an integer from one to three and R² is selected from the group consisting of halogen, C₁₋₄ alkyl, C₁₋₄ alkoxy, trifluoromethyl and trichloromethyl; and
(b) when R¹ is -NH₂, (R²)ₙ is not 4-bromo; (c) when R¹ is methyl, (R²)ₙ is not 4-fluoro; or an agriculturally acceptable salt thereof.

2. A compound according to claim 1 which is a salt.

3. A compound according to claim 1 or 2, wherein aryl or aralkyl groups are of the formula (R²)_{q}-phenyl-[-CR³R⁴-]ₚ- wherein q represents zero or an integer from 1 to 5 and p represents zero or 1.

4. A compound according to claim 1, 2 or 3 wherein R¹ is alkyl or cycloalkyl and n is 2 or 3.

5. A compound according to claim 1, 2, 3 or 4 wherein one of the groups represented by (R²)ₙ is in the ortho position on the phenyl ring.

6. A compound according to any one of claims 1 to 5 wherein R¹ is alkyl optionally substituted by halogen, or cycloalkyl optionally substituted by alkyl or halogen.

7. A compound according to any of claims 1 to 6 wherein R² is halogen, nitro; R⁵; -S(O)ₘR⁵; -OR⁵; alkyl substituted by -OR⁵; or -COOR³.

8. A compound according to any one of claims 1 to 7 wherein R⁵ is alkyl (with 1 to 3 carbon atoms) optionally substituted by F or Cl.

9. A compound according to claim 1 which is :
5-Methyl-4-(2-nitro-4-trifluoromethylbenzoyl)-isoxazole
5-Methyl-4-(2-nitrobenzoyl)-isoxazole
4-(2-Nitro-4-trifluoromethylbenzoyl)-5-phenylisoxazole
4-(2,4-Dinitrobenzoyl)-5-methylisoxazole
5-(4-Chlorophenyl)-4-(2-nitro-4-trifluoromethylbenzoyl)-isoxazole
4-(2-Chlorobenzoyl)-5-methylisoxazole
5-Methyl-4-(2-nitro-4-methylsulphonylbenzoyl)-isoxazole
5-(1-Methylethyl)-4-(2-nitro-4-trifluoromethylbenzoyl)-isoxazole
4-(4-Chlorobenzoyl)-5-methylisoxazole
4-(4-Methylbenzoyl)-5-methylisoxazole
5-(4-Fluorophenyl)-4-(2-nitro-4-trifluoromethylbenzoyl)-isoxazole
5-Ethyl-4-(2-nitro-4-trifluoromethylbenzoyl)-isoxazole
4-(4-Chloro-2-nitrobenzoyl)-5-methylisoxazole
4-(2-Nitro-4-trifluoromethylbenzoyl)-5-propylisoxazole
5-Cyclopropyl-4-(2-nitro-4-trifluoromethylbenzoyl)-isoxazole
4-(2,3-Dichloro-4-methylsulphonylbenzoyl)-5-methylisoxazole
5-(1,1-Dimethylethyl)-4-(2-nitro-4-trifluoromethylbenzoyl)-isoxazole
4-(4-Methoxybenzoyl)-5-methylisoxazole
5-Methyl-4-(4-methyl-2-nitrobenzoyl)-isoxazole
4-(2,3-Dichloro-4-methylsulphonylbenzoyl)-5-(1-methylethyl)-isoxazole
5-Cyclopropyl-4-(2,3-dichloro-4-methylsulphonylbenzoyl)-isoxazole
4-(2-Nitro-4-trifluoromethylbenzoyl)-5-phenylmethylisoxazole
4-(2-Chloro-4-trifluoromethylbenzoyl)-5-cyclopropylisoxazole
5-Methyl-4-(2-nitro-4-pentafluoroethylbenzoyl)-isoxazole
5-Cyclopropyl-4-[4-(1,1-dimethylethyl)-2-nitrobenzoyl]-isoxazole
4-[4-(1,1-Dimethylethyl)-2-nitrobenzoyl]-5-methylisoxazole
5-Cyclopentyl-4-(2-nitro-4-trifluoromethylbenzoyl)-isoxazole
4-(2,4-Dichlorobenzoyl)-5-methylisoxazole
4-(2-Chloro-4-methylsulphonylbenzoyl)-5-methylisoxazole
4-(2-Chloro-4-trifluoromethylbenzoyl)-5-methylisoxazole
5-Methyl-4-(2-trifluoromethylbenzoyl)-isoxazole
5-Methyl-4-(2,4-bis-trifluoromethylbenzoyl)-isoxazole
4-(2-Chloro-4-methylsulphonylbenzoyl)-5-cyclopropylisoxazole
5-Cyclopropyl-4-(2-trifluoromethylbenzoyl)-isoxazole
5-Cyclopropyl-4-(2,4-dichlorobenzoyl)-isoxazole
4-[2,3-Dichloro-4-(methylthio)-benzoyl]-5-methylisoxazole
5-Cyclopropyl-4-(2,4-bis-trifluoromethylbenzoyl)-isoxazole
4-(4-Chloro-2-trifluoromethylbenzoyl)-5-methylisoxazole
4-(4-Cyano-2-nitrobenzoyl)-5-methylisoxazole
5-Amino-4-(2-nitro-4-trifluoromethylbenzoyl)-isoxazole
4-(4-Chloro-2-trifluoromethylbenzoyl)-5-cyclopropylisoxazole
5-(1-Methylethyl)-4-(2-nitro-4-pentafluoroethylbenzoyl)-isoxazole
4-(2-Chloro-4-methylsulphonylbenzoyl)-5-(1-methylethyl)-isoxazole
5-Cyclopropyl-4-(4-fluoro-2-nitrobenzoyl)-isoxazole
5-Cyclopropyl-4-(2-nitro-4-pentafluoroethylbenzoyl)-isoxazole
4-(2,3-Dichloro-4-methylsulphinylbenzoyl)-5-methylisoxazole
5-Cyclobutyl-4-(2-nitro-4-trifluoromethylbenzoyl)-isoxazole
4-(4-Fluoro-2-nitrobenzoyl)-5-methylisoxazole
5-(1-Methylcyclopropyl)-4-(2-nitro-4-trifluoromethylbenzoyl)-isoxazole
5-(4-Nitrophenyl)-4-(2-nitro-4-trifluoromethylbenzoyl)-isoxazole
5-(4-Methoxyphenyl)-4-(2-nitro-4-trifluoromethylbenzoyl)-isoxazole
4-(2-Chloro-3-ethoxy-4-methylsulphonylbenzoyl)-5-methylisoxazole
4-(3-Cyanobenzoyl)-5-methylisoxazole
5-Cyclopropyl-4-(4-methylsulphonyl-2-trifluoromethylbenzoyl)-isoxazole
5-Cyclopropyl-4-(2-nitro-4-methylsulphonylbenzoyl)-isoxazole
4-(2-Chloro-3-ethoxy-4-methylsulphonylbenzoyl)-5-cyclopropylisoxazole
4-(2-Chloro-3-ethoxy-4-ethylsulphonylbenzoyl)-5-methylisoxazole
4-(2-Chloro-3-ethoxy-4-methylsulphonylbenzoyl)-5-(1-methylethyl)-isoxazole
4-(2-Chloro-3-ethoxy-4-ethylsulphonylbenzoyl)-5-cyclopropylisoxazole
5-(1-Ethoxycarbonylcyclopropyl)-4-(2-nitro-4-trifluoromethylbenzoyl)isoxazole
4-(3-Methoxycarbonyl-2-methyl-4-methylsulphonylbenzoyl)-5-methylisoxazole
5-(2-Methylcyclopropyl)-4-(2-nitro-4-trifluoromethylbenzoyl)-isoxazole
4-[2-Chloro-3-(1-methylethoxy)-4-methylsulphonylbenzoyl]-5-methylisoxazole
5-Methyl-4-[2-methyl-3-(1-methylethoxycarbonyl)-4-methylsulphonylbenzoyl]-isoxazole
5-Cyclopropyl-4-[2-methyl-3-(1-methylethoxycarbonyl)-4-methylsulphonylbenzoyl]-isoxazole or
5-Cyclopropyl-4-(2,3,4-trichlorobenzoyl)-isoxazole

10. A herbicidal composition which comprises, as active ingredient, an isoxazole derivative of general formula III 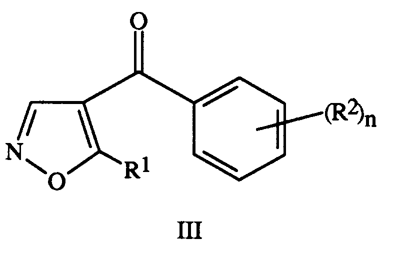 wherein R¹ represents :
a straight- or branched-chain alkyl, alkenyl, or alkynyl group containing up to 6 carbon atoms which is optionally substituted by one or more halogen atoms ;
a cycloalkyl group containing 3 to 6 carbon atoms optionally substituted by one or more R³ groups or one or more halogen atoms or an ester group, CO₂R³¹;
a cycloalkenyl group containing 5 or 6 carbon atoms optionally substituted by one or more R³ groups or one or more halogen atoms or an ester group, CO₂R³¹;
an aryl or aralkyl group;
a group, CO₂R³ ;
an aldehyde or acyl group, COR³ ;
a cyano group ;
a nitro group ;
an amino group, NR³R⁴ ; or
a halogen atom ;
R² represents :
a nitro group ;
a cyano group ;
a halogen atom ;
a group R⁵ ;
a sulphenyl, sulphinyl or sulphonyl group S(O)ₘR⁵ ;
a sulphamoyl group SO₂NR³R⁴ ;
a group CO₂R³ ;
an aldehyde or acyl group COR³ ;
a carbamoyl or thiocarbamoyl group CONR³R⁴ or CSNR³R⁴ ;
an alkoxy group OR⁵ ; or
an alkyl group (with 1 to 3 carbon atoms) substituted by OR⁵ ;
R³ and R⁴ which may be the same or different, each represents :
a hydrogen atom or a straight- or branched-chain alkyl group containing up to 6 carbon atoms which is optionally substituted by one or more halogen atoms ;
R³¹ is as defined for R³ but does not represent hydrogen;
R⁵ represents :
a straight- or branched-chain alkyl group containing up to 6 carbon atoms which is optionally substituted by one or more halogen atoms ;
m represents zero, 1 or 2; and
n represents an integer from 1 to 5;
in association with an agriculturally acceptable diluent or carrier,
provided that when R¹ represents C₁₋₄ alkyl, n is an integer from one to three and R² is selected from the group consisting of halogen, C₁₋₄ alkyl, C₁₋₄ alkoxy, trifluoromethyl and trichloromethyl, the composition is not present in a single organic solvent with a compound of formula:- 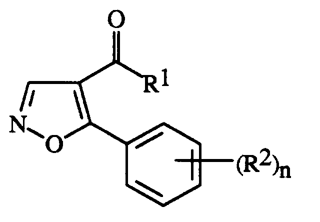 wherein R¹ is C₁₋₄ alkyl, n is an integer from one to three and R² is selected from the group consisting of halogen, C₁₋₄ alkyl, C₁₋₄ alkoxy, trifluoromethyl and trichloromethyl.

11. A herbicidal composition according to claim 10 which comprises a compound according to any one of claims 3 to 9.

12. A herbicidal composition according to claim 10 or 11 which contains from 0.05 to 90% by weight of one or more compounds of general formula III.

13. A herbicidal composition according to any one of claims 10 to 12 which comprises up to 15 % of surface-active agent in liquid emulsifiable suspension concentrates and up to 25 % in liquid water soluble concentrates.

14. A herbicidal composition according to any one of claims 10 to 13 which is in the form of a solid such as a wettable powder or a granule; or a liquid such as a solution or a suspension or an emulsion.

15. A herbicidal composition according to any one of claims 10 to 14 which also contains an adjuvant, a protective colloid, a thickener, a penetrating agent, a stabiliser, a sequestering agent, an anti-caking agent, a colouring agent or a corrosion inhibitor.

16. A herbicidal composition according to any one of claims 10 to 15 which is in the form of:
an aqueous suspension concentrate which comprises from 10 to 70 % of one or more compounds of general formula III, from 2 to 10 % of surface-active agent, from 0.1 to 5 % of thickener and from 15 to 87.9 % of water ;
a wettable powder which comprises from 10 to 90 % of one or more compounds of general formula III, from 2 to 10 % of surface-active agent and from 8 to 88 % of solid diluent or carrier ;
a soluble powder which comprises from 10 to 90 % of one or more compounds of general formula III, from 2 to 40 % of sodium carbonate and from 0 to 88 % of solid diluent ;
a liquid water soluble concentrate which comprises from 5 to 50 %, preferably 10 to 30 %, of one or more compounds of general formula III, from 5 to 25 % of surface-active agent and from 25 to 90 % of water-miscible solvent, or a mixture of water-miscible solvent and water ;
a liquid emulsifiable suspension concentrate which comprises from 10 to 70 % of one or more compounds of general formula III, from 5 to 15 % of surface-active agent, from 0.1 to 5 % of thickener and from 10 to 84.9 % of organic solvent ;
a granule which comprises from 1 to 90 % of one or more compounds of general formula III, from 0.5 to 7 %, preferably 0.5 to 2 % of surface-active agent and from 3 to 98.5 % of granular carrier; or
an emulsifiable concentrate which comprises from 0.05 to 90 % of one or more compounds of general formula III, from 0.01 to 10 %, of surface-active agent and from 9.99 to 99.94 % of organic solvent.

17. An article of manufacture comprising at least one of the isoxazole derivatives of any one of claims 1 to 9 or a herbicidal composition according to any one of claims 10 to 16 comprising at least one of the isoxazole derivatives of general formula III within a container for the aforesaid derivative or derivatives, or aforesaid herbicidal composition, and instructions physically associated with the aforesaid container setting out the manner in which the aforesaid derivative or derivatives or herbicidal composition contained therein is to be used to control the growth of weeds.

18. A method for controlling the growth of weeds at a locus which comprises applying to the locus a herbicidally effective amount of an isoxazole derivative of general formula III 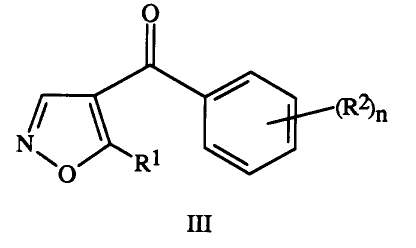 wherein R¹ represents :
a straight- or branched-chain alkyl, alkenyl, or alkynyl group containing up to 6 carbon atoms which is optionally substituted by one or more halogen atoms ; or
a cycloalkyl group containing 3 to 6 carbon atoms optionally substituted by one or more R³ groups or one or more halogen atoms or an ester group, CO₂R³¹; or
a cycloalkenyl group containing 5 or 6 carbon atoms optionally substituted by one or more R³ groups or one or more halogen atoms or an ester group, CO₂R³¹;
an aryl or aralkyl group;
a group, CO₂R³ ;
an aldehyde or acyl group, COR³ ;
a cyano group ;
a nitro group ;
an amino group, NR³R⁴ ; or
a halogen atom ; and
R² represents :
a nitro group ;
a cyano group ;
a halogen atom ;
a group R⁵ ;
a sulphenyl, sulphinyl or sulphonyl group S(O)ₘR⁵ ;
a sulphamoyl group SO₂NR³R⁴ ;
a group CO₂R³ ;
an aldehyde or acyl group COR³ ;
a carbamoyl or thiocarbamoyl group CONR³R⁴ or CSNR³R⁴ ;
an alkoxy group OR⁵ ; or
an alkyl group (with 1 to 3 carbon atoms) substituted by OR⁵ ;
R³ and R⁴ which may be the same or different, each represents :
a hydrogen atom or a straight- or branched-chain alkyl group containing up to 6 carbon atoms which is optionally substituted by one or more halogen atoms ;
R³¹ is as defined for R³ but does not represent hydrogen;
R⁵ represents :
a straight- or branched-chain alkyl group containing up to 6 carbon atoms which is optionally substituted by one or more halogen atoms ;
m represents zero, 1 or 2; and
n represents an integer from 1 to 5;
or an agriculturally acceptable salt thereof.

19. A method according to claim 18 wherein the application rate of isoxazole derivative of general formula III or an agriculturally acceptable salt thereof is from 0.01 kg to 20 kg per hectare.

20. A method according to claim 19 for the selective control of weeds in crops wherein the application rate is from 0.01 kg to 8.0 kg per hectare.

21. A method according to claim 20 for the selective control of weeds in crops wherein the application rate is from 0.1 kg to 4.0 kg per hectare.

22. A method according to claim 18 by pre- or post-emergence application in established orchards or other tree-growing areas or plantations, before or after planting of the trees or plantations at an application rate from 0.25 kg to 10.0 kg.

23. A method according to claim 22 wherein the application rate is from 0.5 kg to 8.0 kg per hectare.

24. A process of the preparation of an isoxazole derivative of general formula III as defined in claim 1 provided that R¹ is not a cyano group, a nitro group, an amino group or a halogen atom, which comprises the reaction of a compound of general formula IV 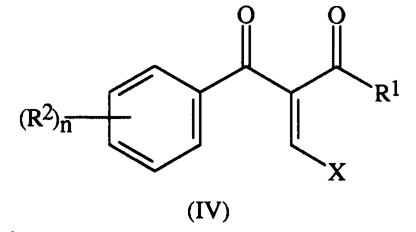 wherein R¹, R² and n are as defined in claim 1 provided that R¹ is not a cyano, nitro, amino or halogen, and X is O-alkyl or N-dialkyl, with a salt of hydroxylamine in a solvent, optionally in the presence of a base.

25. A process according to claim 25 wherein the solvent is ethanol or acetonitrile.

26. A process of the preparation of an isoxazole derivative of general formula III according to any claim 1 which comprises:
(a) where R² is a halogen atom, an alkyl group, an alkylthio group or an alkoxy group, reacting a compound of general formula V 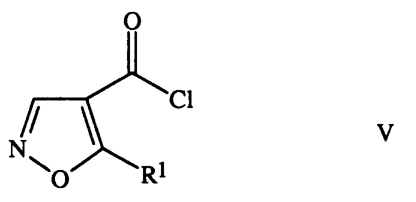 wherein R¹ is as defined in claim 1, with an excess of the appropriately substituted benzene in the presence of a Lewis acid catalyst at a temperature between room temperature and 100°C;
(b) where R¹ is an unsubstituted amino group, reacting a compound of general formula VI 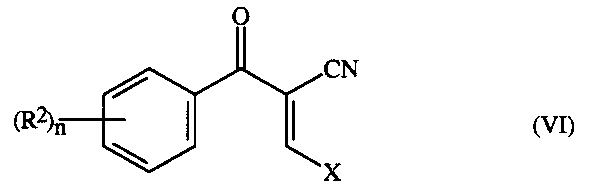 wherein R² and n are as defined in claim 1 and X is O-alkyl or N-dialkyl, with a salt of hydroxylamine in a solvent, optionally in the presence of a base at a temperature between room temperature and the reflux temperature of the solvent;
(c) where R¹ is a cyano group, conversion to cyano of the corresponding compound of general formula III in which R¹ is an ester group by acidic hydrolysis of the carboxylic acid followed by conversion to the acid chloride, the acid chloride being optionally converted to the amide and the amide being optionally converted to the cyano group by dehydration;
(d) where R¹ is an acyl group, the conversion to acyl of the corresponding compound of general formula III in which R¹ is a cyano group by reaction with an organometallic reagent;
(e) where R¹ is a nitro group, oxidising the corresponding compound of general formula III in which R¹ is an unsubstituted amino group;
(f) where R¹ is a halogen, conversion to halogen of the corresponding compound of formula III in which R¹ is unsubstituted amino group by diazotization;
(g) where R¹ is a substituted amino group, displacement of the halogen atom of the corresponding compound of general formula III using the appropriate amine in an inert solvent;
(h) where the compound of general formula III contains a sulphinyl or sulphonyl group, oxidation of the corresponding compound of general formula III containing a thioether group.

27. A compound which may be used as an intermediate in the preparation of a compound of general formula III as defined in any one of claims 1 to 10, characterized by the general formula IV: 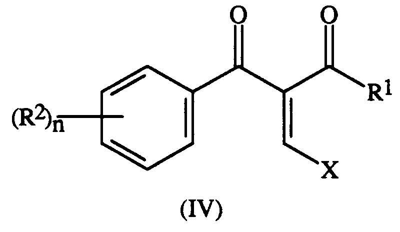 wherein R¹ represents :
a straight- or branched-chain alkyl, alkenyl, or alkynyl group containing up to 6 carbon atoms which is optionally substituted by one or more halogen atoms ;
a cycloalkyl group containing 3 to 6 carbon atoms optionally substituted by one or more R³ groups or one or more halogen atoms or an ester group, CO₂R³¹;
a cycloalkenyl group containing 5 or 6 carbon atoms optionally substituted by one or more R³ groups or one or more halogen atoms or an ester group, CO₂R³¹;
an aryl or aralkyl group;
a group, CO₂R³ ;
an aldehyde or acyl group, COR³ ;
a cyano group ;
a nitro group ;
an amino group, NR³R⁴ ; or
a halogen atom ;
R² represents :
a nitro group ; or
a cyano group ; or
a halogen atom ; or
a group R⁵ ; or
a sulphenyl, sulphinyl or sulphonyl group S(O)ₘR⁵ ; or
a sulphamoyl group SO₂NR³R⁴ ; or
a group CO₂R³ ; or
an aldehyde or acyl group COR³ ; or
a carbamoyl or thiocarbamoyl group CONR³R⁴ or CSNR³R⁴ ; or
an alkoxy group OR⁵ ; or
an alkyl group (with 1 to 3 carbon atoms) substituted by OR⁵ ;
R³ and R⁴ which may be the same or different, each represents :
a hydrogen atom or a straight- or branched-chain alkyl group containing up to 6 carbon atoms which is optionally substituted by one or more halogen atoms ;
R³¹ is as defined for R³ but does not represent hydrogen;
R⁵ represents :
a straight- or branched-chain alkyl group containing up to 6 carbon atoms which is optionally substituted by one or more halogen atoms ;
m represents zero, 1 or 2;
n represents an integer from 1 to 5; and
X represents O-alkyl or N-dialkyl;
with the proviso that
(a) when X is -N(CH₃)₂, R² is selected from the group consisting of halogen, C₁₋₄ alkyl, C₁₋₄ alkoxy, trifluoromethyl and trichloromethyl, and n is an integer from one to three; R¹ is not C₁₋₄ alkyl;
(b) when X and R¹ are -N(CH₃)₂, (R²)ₙ is not 2,4-dichloro-5-fluoro; and
(c) when X is -N(CH₃)₂ and R¹ is methyl, (R²)ₙ is not 4-chloro or 4-methoxy.

## Claims (Claims for the following Contracting State(s): ES)

1. A process of the preparation of an isoxazole derivative of general formula III or of an acriculturally acceptable salt thereof 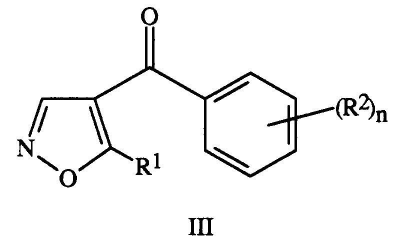 wherein R¹ represents :
a straight- or branched-chain alkyl, alkenyl, or alkynyl group containing up to 6 carbon atoms which is optionally substituted by one or more halogen atoms ;
a cycloalkyl group containing 3 to 6 carbon atoms optionally substituted by one or more R³ groups or one or more halogen atoms or an ester group, CO₂R³¹;
a cycloalkenyl group containing 5 or 6 carbon atoms optionally substituted by one or more R³ groups or one or more halogen atoms or an ester group, CO₂R³¹;
an aryl or aralkyl group;
a group, CO₂R³ ;
an aldehyde or acyl group, COR³;
a cyano group ;
a nitro group ;
an amino group, NR³R⁴ ; or
a halogen atom ;
R² represents :
a nitro group ;
a cyano group ; or
a halogen atom;
a group R⁵ ;
a sulphenyl, sulphinyl or sulphonyl group S(O)ₘR⁵ ;
a sulphamoyl group SO₂NR³R⁴ ;
a group CO₂R³ ;
an aldehyde or acyl group COR³ ;
a carbamoyl or thiocarbamoyl group CONR³R⁴ or CSNR³R⁴ ;
an alkoxy group OR⁵ ; or
an alkyl group (with 1 to 3 carbon atoms) substituted by OR⁵ ;
R³ and R⁴ which may be the same or different, each represents :
a hydrogen atom or a straight- or branched-chain alkyl group containing up to 6 carbon atoms which is optionally substituted by one or more halogen atoms ;
R³¹ is as defined for R³ but does not represent hydrogen;
R⁵ represents :
a straight- or branched-chain alkyl group containing up to 6 carbon atoms which is optionally substituted by one or more halogen atoms ;
m represents zero, 1 or 2;
n represents an integer from 1 to 5;
provided that
(a) when R¹ represents C₁₋₄ alkyl, n is an integer from one to three and R² is selected from the group consisting of halogen, C₁₋₄ alkyl, C₁₋₄ alkoxy, trifluoromethyl and trichloromethyl, the compound is not present in a mixture with a compound of formula:- 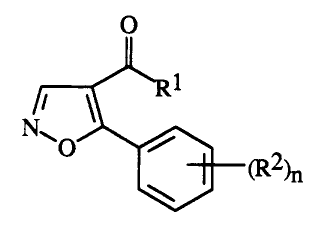 wherein R¹ is C₁₋₄ alkyl, n is an integer from one to three and R² is selected from the group consisting of halogen, C₁₋₄ alkyl, C₁₋₄ alkoxy, trifluoromethyl and trichloromethyl; and
(b) when R¹ is -NH₂, (R²)ₙ is not 4-bromo;
(c) when R¹ is methyl, (R²)ₙ is not 4-fluoro;
or an agriculturally acceptable salt thereof; comprising:
(a) the reaction of a compound of general formula IV 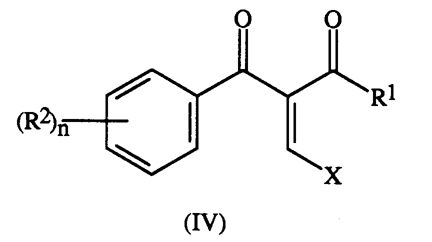 wherein R¹, R² and n are as defined above provided that R¹ is not a cyano, nitro, amino or halogen, and X is O-alkyl or N-dialkyl, with a salt of hydroxylamine in a solvent, optionally in the presence of a base;
(b) where R² is a halogen atom, an alkyl group, an alkylthio group or an alkoxy group, reacting a compound of general formula V 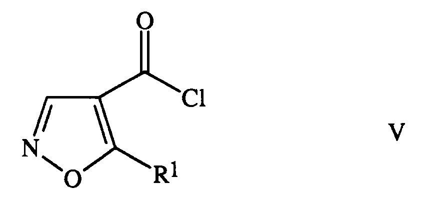 wherein R¹ is as defined above, with an excess of the appropriately substituted benzene in the presence of a Lewis acid catalyst at a temperature between room temperature and 100°C;
(c) where R¹ is an unsubstituted amino group, reacting a compound of general formula VI 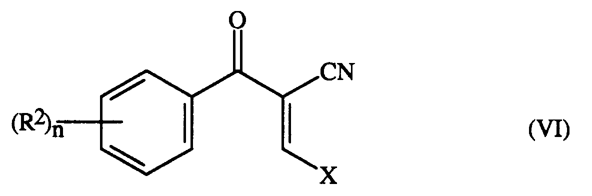 wherein R² and n are as defined above and X is O-alkyl or N-dialkyl, with a salt of hydroxylamine in a solvent, optionally in the presence of a base at a temperature between room temperature and the reflux temperature of the solvent;
(d) where R¹ is a cyano group, conversion to cyano of the corresponding compound of general formula III in which R¹ is an ester group by acidic hydrolysis of the carboxylic acid followed by conversion to the acid chloride, the acid chloride being optionally converted to the amide and the amide being optionally converted to the cyano group by dehydration;
(e) where R¹ is an acyl group, the conversion to acyl of the corresponding compound of general formula III in which R¹ is a cyano group by reaction with an organometallic reagent;
(f) where R¹ is a nitro group, oxidising the corresponding compound of general formula III in which R¹ is an unsubstituted amino group;
(g) where R¹ is a halogen, conversion to halogen of the corresponding compound of formula III in which R¹ is unsubstituted amino group by diazotization;
(h) where R¹ is a substituted amino group, displacement of the halogen atom of the corresponding compound of general formula III using the appropriate amine in an inert solvent;
(i) where the compound of general formula III contains a sulphinyl or sulphonyl group, oxidation of the corresponding compound of general formula III containing a thioether group.

2. A process according to claim 1 for preparing a salt.

3. A process according to claim 1 or 2, wherein aryl or aralkyl groups are of the formula R²)_{q}-phenyl-[-CR³R⁴-]ₚ- wherein q represents zero or an integer from 1 to 5 and p represents zero or 1.

4. A process according to claim 1, 2 or 3 wherein R¹ is alkyl or cycloalkyl and n is 2 or 3.

5. A process according to claim 1, 2, 3 or 4 wherein one of the groups represented by (R²)ₙ is in the ortho position on the phenyl ring.

6. A process according to any one of claims 1 to 5 wherein R¹ is alkyl optionally substituted by halogen, or cycloalkyl optionally substituted by alkyl or halogen.

7. A process according to any of claims 1 to 6 wherein R² is halogen, nitro; R⁵; -S(O)ₘR⁵; -OR⁵; alkyl substituted by -OR⁵; or -COOR³.

8. A process according to any one of claims 1 to 7 wherein R⁵ is alkyl (with 1 to 3 carbon atoms) optionally substituted by F or Cl.

9. A process according to claim 1 for the preparation of:
5-Methyl-4-(2-nitro-4-trifluoromethylbenzoyl)-isoxazole
5-Methyl-4-(2-nitrobenzoyl)-isoxazole
4-(2-Nitro-4-trifluoromethylbenzoyl)-5-phenylisoxazole
4-(2,4-Dinitrobenzoyl)-5-methylisoxazole
5-(4-Chlorophenyl)-4-(2-nitro-4-trifluoromethylbenzoyl)-isoxazole
4-(2-Chlorobenzoyl)-5-methylisoxazole
5-Methyl-4-(2-nitro-4-methylsulphonylbenzoyl)-isoxazole
5-(1-Methylethyl)-4-(2-nitro-4-trifluoromethylbenzoyl)-isoxazole
4-(4-Chlorobenzoyl)-5-methylisoxazole
4-(4-Methylbenzoyl)-5-methylisoxazole
5-(4-Fluorophenyl)-4-(2-nitro-4-trifluoromethylbenzoyl)-isoxazole
5-Ethyl-4-(2-nitro-4-trifluoromethylbenzoyl)-isoxazole
4-(4-Chloro-2-nitrobenzoyl)-5-methylisoxazole
4-(2-Nitro-4-trifluoromethylbenzoyl)-5-propylisoxazole
5-Cyclopropyl-4-(2-nitro-4-trifluoromethylbenzoyl)-isoxazole
4-(2,3-Dichloro-4-methylsulphonylbenzoyl)-5-methylisoxazole
5-(1,1-Dimethylethyl)-4-(2-nitro-4-trifluoromethylbenzoyl)-isoxazole
4-(4-Methoxybenzoyl)-5-methylisoxazole
5-Methyl-4-(4-methyl-2-nitrobenzoyl)-isoxazole
4-(2,3-Dichloro-4-methylsulphonylbenzoyl)-5-(1-methylethyl)-isoxazole
5-Cyclopropyl-4-(2,3-dichloro-4-methylsulphonylbenzoyl)-isoxazole
4-(2-Nitro-4-trifluoromethylbenzoyl)-5-phenylmethylisoxazole
4-(2-Chloro-4-trifluoromethylbenzoyl)-5-cyclopropylisoxazole
5-Methyl-4-(2-nitro-4-pentafluoroethylbenzoyl)-isoxazole
5-Cyclopropyl-4-[4-(1,1-dimethylethyl)-2-nitrobenzoyl]-isoxazole
4-[4-(1,1-Dimethylethyl)-2-nitrobenzoyl]-5-methylisoxazole
5-Cyclopentyl-4-(2-nitro-4-trifluoromethylbenzoyl)-isoxazole
4-(2,4-Dichlorobenzoyl)-5-methylisoxazole
4-(2-Chloro-4-methylsulphonylbenzoyl)-5-methylisoxazole
4-(2-Chloro-4-trifluoromethylbenzoyl)-5-methylisoxazole
5-Methyl-4-(2-trifluoremethylbenzoyl)-isoxazole
5-Methyl-4-(2,4-bis-trifluoromethylbenzoyl)-isoxazole
4-(2-Chloro-4-methylsulphonylbenzoyl)-5-cyclopropylisoxazole
5-Cyclopropyl-4-(2-trifluoromethylbenzoyl)-isoxazole
5-Cyclopropyl-4-(2,4-dichlorobenzoyl)-isoxazole
4-[2,3-Dichloro-4-(methylthio)-benzoyl]-5-methylisoxazole
5-Cyclopropyl-4-(2,4-bis-trifluoromethylbenzoyl)-isoxazole
4-(4-Chloro-2-trifluoromethylbenzoyl)-5-methylisoxazole
4-(4-Cyano-2-nitrobenzoyl)-5-methylisoxazole
5-Amino-4-(2-nitro-4-trifluoromethylbenzoyl)-isoxazole
4-(4-Chloro-2-trifluoromethylbenzoyl)-5-cyclopropylisoxazole
5-(1-Methylethyl)-4-(2-nitro-4-pentafluoroethylbenzoyl)-isoxazole
4-(2-Chloro-4-methylsulphonylbenzoyl)-5-(1-methylethyl)-isoxazole
5-Cyclopropyl-4-(4-fluoro-2-nitrobenzoyl)-isoxazole
5-Cyclopropyl-4-(2-nitro-4-pentafluoroethylbenzoyl)-isoxazole
4-(2,3-Dichloro-4-methylsulphinylbenzoyl)-5-methylisoxazole
5-Cyclobutyl-4-(2-nitro-4-trifluoromethylbenzoyl)-isoxazole
4-(4-Fluoro-2-nitrobenzoyl)-5-methylisoxazole
5-(1-Methylcyclopropyl)-4-(2-nitro-4-trifluoromethylbenzoyl)-isoxazole
5-(4-Nitrophenyl)-4-(2-nitro-4-trifluoromethylbenzoyl)-isoxazole
5-(4-Methoxyphenyl)-4-(2-nitro-4-trifluoromethylbenzoyl)-isoxazole
4-(2-Chloro-3-ethoxy-4-methylsulphonylbenzoyl)-5-methylisoxazole
4-(3-Cyanobenzoyl)-5-methylisoxazole
5-Cyclopropyl-4-(4-methylsulphonyl-2-trifluoromethylbenzoyl)-isoxazole
5-Cyclopropyl-4-(2-nitro-4-methylsulphonylbenzoyl)-isoxazole
4-(2-Chloro-3-ethoxy-4-methylsulphonylbenzoyl)-5-cyclopropylisoxazole
4-(2-Chloro-3-ethoxy-4-ethylsulphonylbenzoyl)-5-methylisoxazole
4-(2-Chloro-3-ethoxy-4-methylsulphonylbenzoyl)-5-(1-methylethyl)-isoxazole
4-(2-Chloro-3-ethoxy-4-ethylsulphonylbenzoyl)-5-cyclopropylisoxazole
5-(1-Ethoxycarbonylcyclopropyl)-4-(2-nitro-4-trifluoromethylbenzoyl)isoxazole
4-(3-Methoxycarbonyl-2-methyl-4-methylsulphonylbenzoyl)-5-methylisoxazole
5-(2-Methylcyclopropyl)-4-(2-nitro-4-trifluoromethylbenzoyl)-isoxazole
4-[2-Chloro-3-(1-methylethoxy)-4-methylsulphonylbenzoyl]-5-methylisoxazole
5-Methyl-4-[2-methyl-3-(1-methylethoxycarbonyl)-4-methylsulphonylbenzoyl]-isoxazole
5-Cyclopropyl-4-[2-methyl-3-(1-methylethoxycarbonyl)-4-methylsulphonylbenzoyl]-isoxazole or
5-Cyclopropyl-4-(2,3,4-trichlorobenzoyl)-isoxazole.

10. A process for preparing a herbicidal composition comprising formulating an isoxazole derivative of general formula III 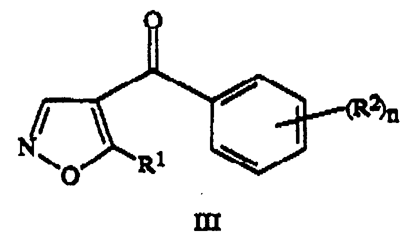 wherein R¹ represents :
a straight- or branched-chain alkyl, alkenyl, or alkynyl group containing up to 6 carbon atoms which is optionally substituted by one or more halogen atoms ;
a cycloalkyl group containing 3 to 6 carbon atoms optionally substituted by one or more R³ groups or one or more halogen atoms or an ester group, CO₂R³¹;
a cycloalkenyl group containing 5 or 6 carbon atoms optionally substituted by one or more R³ groups or one or more halogen atoms or an ester group, CO₂R³¹;
an aryl or aralkyl group;
a group, CO₂R³ ;
an aldehyde or acyl group, COR³ ;
a cyano group ;
a nitro group ;
an amino group, NR³R⁴ ; or
a halogen atom ;
R² represents :
a nitro group ;
a cyano group ;
a halogen atom ;
a group R⁵ ;
a sulphenyl, sulphinyl or sulphonyl group S(O)ₘR⁵ ;
a sulphamoyl group SO₂NR³R⁴ ;
a group CO₂R³ ;
an aldehyde or acyl group COR³ ;
a carbamoyl or thiocarbamoyl group CONR³R⁴ or CSNR³R⁴ ;
an alkoxy group OR⁵ ; or
an alkyl group (with 1 to 3 carbon atoms) substituted by OR⁵ ;
R³ and R⁴ which may be the same of different, each represents :
a hydrogen atom or a straight- or branched-chain alkyl group containing up to 6 carbon atoms which is optionally substituted by one or more halogen atoms ; R³¹ is as defined for R³ but does not represent hydrogen;
R⁵ represents :
a straight-or branched-chain alkyl group containing up to 6 carbon atoms which is optionally substituted by one or more halogen atoms ;
m represents zero,1 or 2; and
n represents an integer from 1 to 5;
with an agricuturally acceptable carrier or diluent;
provided that when R¹ represents C₁₋₄ alkyl, n is an integer from one to three and R² is selected from the group consisting of halogen, C₁₋₄ alkyl, C₁₋₄ alkoxy, trifluoromethyl and trichloromethyl, the composition is not prepared in a single organic solvent with a compound of formula:- 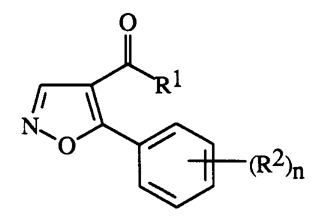 wherein R¹ is C₁₋₄ alkyl, n is an integer from one to three and R² is selected from the group consisting of halogen, C₁₋₄ alkyl, C₁₋₄ alkoxy,trifluoromethyl and trichloromethyl.

11. A process according to claim 10 for preparing a herbicidal composition which contains from 0.05 to 90% by weight of one or more compounds of general formula III.

12. A process according to claim 10 or 11 for preparing a herbicidal composition which comprises up to 15 % of surface-active agent in liquid emulsifiable suspension concentrates and up to 25 % in liquid water soluble concentrates.

13. A process according to claim 10, 11 or 12 for preparing a herbicidal composition which is in the form of a solid such as a wettable powder or a granule; or a liquid such as a solution or a suspension or an emulsion.

14. A process according to any one of claims 10 to 13 for preparing a herbicidal composition which also contains an adjuvant, a protective colloid, a thickener,a penetrating agent, a stabiliser, a sequestering agent, an anti-caking agent, a colouring agent or a corrosion inhibitor.

15. A process according to any one of claims 10 to 14 for preparing a herbicidal composition which is in the form of:
an aqueous suspension concentrate which comprises from 10 to 70 % of one or more compounds of general formula III, from 2 to 10 % of surface-active agent, from 0.1 to 5 % of thickener and from 15 to 87.9 % of water ;
a wettable powder which comprises from 10 to 90 % of one or more compounds of general formula III, from 2 to 10 % of surface-active agent and from 8 to 88 % of solid diluent or carrier ;
compounds of general formula III, from 2 to 40 % of sodium carbonate and from 0 to 88 % of solid diluent ;
a liquid water soluble concentrate which comprises from 5 to 50 %, preferably 10 to 30 %, of one or more compounds of general formula III, from 5 to 25 % of surface-active agent and from 25 to 90 % of water-miscible solvent, or a mixture of water-miscible solvent and water ;
a liquid emulsifiable suspension concentrate which comprises from 10 to 70 % of one or more compounds of general formula III, from 5 to 15 % of surface-active agent, from 0.1 to 5 % of thickener and from 10 to 84.9 % of organic solvent ;
a granule which comprises from 1 to 90 % of one or more compounds of general formula III, from 0.5 to 7 %, preferably 0.5 to 2 % of surface-active agent and from 3 to 98.5 % of granular carrier ; or
an emulsifiable concentrate which comprises from 0.05 to 90 % of one or more compounds of general formula III, from 0.01 to 10 %, of surface-active agent and from 9.99 to 99.94 % of organic solvent.

16. A method for controlling the growth of weeds at a locus which comprises applying to the locus a herbicidally effective amount of an isoxazole derivative of general formula III 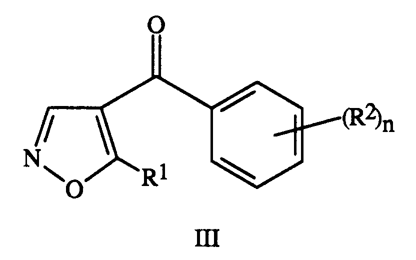 wherein R¹ represents :
a straight- or branched-chain alkyl, alkenyl, or alkynyl group containing up to 6 carbon atoms which is optionally substituted by one or more halogen atoms ; or
a cycloalkyl group containing 3 to 6 carbon atoms optionally substituted by one or more R³ groups or one or more halogen atoms or an ester group, CO₂R³¹; or
a cycloalkenyl group containing 5 or 6 carbon atoms optionally substituted by one or more R³ groups or one or more halogen atoms or an ester group, CO₂R³¹;
an aryl or aralkyl group;
a group, CO₂R³ ;
an aldehyde or acyl group, COR³ ;
a cyano group ;
a nitro group ;
an amino group, NR³R⁴ ; or
a halogen atom ; and
R² represents :
a nitro group ;
a cyano group ;
a halogen atom ;
a group R⁵ ;
a sulphenyl, sulphinyl or sulphonyl group S(O)ₘR⁵ ;
a sulphamoyl group SO₂NR³R⁴ ;
a group CO₂R³ ;
an aldehyde or acyl group COR³ ;
a carbamoyl or thiocarbamoyl group CONR³R⁴ or CSNR³R⁴ ;
an alkoxy group OR⁵ ; or
an alkyl group (with 1 to 3 carbon atoms) substituted by OR⁵ ;
R³ and R⁴ which may be the same or different, each represents :
a hydrogen atom or a straight- or branched-chain alkyl group containing up to 6 carbon atoms which is optionally substituted by one or more halogen atoms ;
R³¹ is as defined for R³ but does not represent hydrogen;
R⁵ represents :
a straight- or branched-chain alkyl group containing up to 6 carbon atoms which is optionally substituted by one or more halogen atoms ;
m represents zero, 1 or 2; and
n represents an integer from 1 to 5;
or an agriculturally acceptable salt thereof.

17. A method according to claim 16 wherein the application rate of isoxazole derivative of general formula III or an agriculturally acceptable salt thereof is from 0.01 kg to 20 kg per hectare.

18. A method according to claim 17 for the selective control of weeds in crops wherein the application rate is from 0.01 kg to 8.0 kg per hectare.

19. A method according to claim 18 for the selective control of weeds in crops wherein the application rate is from 0.1 kg to 4.0 kg per hectare.

20. A method according to claim 16 by pre- or post-emergence application in established orchards or other tree-growing areas or plantations, before or after planting of the trees or plantations at an application rate from 0.25 kg to 10.0 kg.

21. A method according to claim 20 wherein the application rate is from 0.5 kg to 8.0 kg per hectare.

22. A process for preparing an intermediate of formula (IV): 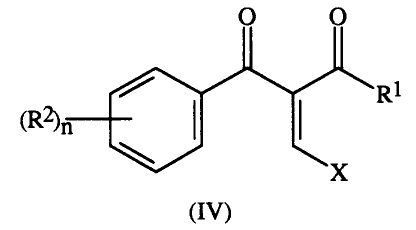 wherein R¹ represents :
a straight- or branched-chain alkyl, alkenyl, or alkynyl group containing up to 6 carbon atoms which is optionally substituted by one or more halogen atoms ;
a cycloalkyl group containing 3 to 6 carbon atoms optionally substituted by one or more R³ groups or one or more halogen atoms or an ester group, CO₂R³¹;
a cycloalkenyl group containing 5 or 6 carbon atoms optionally substituted by one or more R³ groups or one or more halogen atoms or an ester group, CO₂R³¹;
an aryl or aralkyl group;
a group, CO₂R³ ;
an aldehyde or acyl group, COR³ ;
a cyano group ;
a nitro group ;
an amino group, NR³R⁴ ; or
a halogen atom ;
R² represents :
a nitro group ; or
a cyano group ; or
a halogen atom ; or
a group R⁵ ; or
a sulphenyl, sulphinyl or sulphonyl group S(O)ₘR⁵ ; or
a sulphamoyl group SO₂NR³R⁴ ; or
a group CO₂R³ ; or
an aldehyde or acyl group COR³ ; or
a carbamoyl or thiocarbamoyl group CONR³R⁴ or CSNR³R⁴ ; or
an alkoxy group OR⁵ ; or
an alkyl group (with 1 to 3 carbon atoms) substituted by OR⁵ ; R³ and R⁴ which may be the same or different, each represents :
a hydrogen atom or a straight- or branched-chain alkyl group containing up to 6 carbon atoms which is optionally substituted by one or more halogen atoms ;
R³¹ is as defined for R³ but does not represent hydrogen;
R⁵ represents :
a straight- or branched-chain alkyl group containing up to 6 carbon atoms which is optionally substituted by one or more halogen atoms ;
m represents zero, 1 or 2;
n represents an integer from 1 to 5; and
X represents O-alkyl or N-dialkyl;
with the proviso that
(a) when X is -N(CH₃)₂, R² is selected from the group consisting of halogen, C₁₋₄ alkyl, C₁₋₄ alkoxy, trifluoromethyl and trichloromethyl, and n is an integer from one to three; R¹ is not C₁₋₄ alkyl;
(b) when X and R¹ are -N(CH₃)₂, (R²)ₙ is not 2,4-dichloro-5-fluoro; and
(c) when X is -N(CH₃)₂ and R¹ is methyl, (R²)ₙ is not 4-chloro or 4-methoxy
comprising the reaction of a diketone of general formula VII 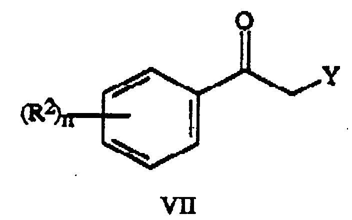 wherein R² and n are as defined above, Y is COR¹ and R¹ is as defined above, with
(i) an ortho ester in the presence of an acid catalyst; or
(ii) an amide acetal in an inert solvent.

## Patentansprüche (Patentansprüche für folgende(n) Vertragsstaat(en): AT, BE, CH, DE, DK, FR, GB, GR, IT, LI, LU, NL, SE)

1. Ein Isoxazol-Derivat, gekennzeichnet durch die allgemeine Formel III: 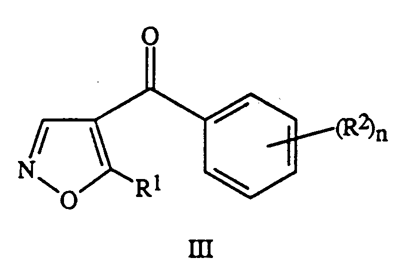 worin R¹ bedeutet:
eine bis zu 6 Kohlenstoffatome enthaltende gerad- oder verzweigtkettige Alkyl-, Alkenyl- oder Alkinylgruppe, welche wahlweise durch ein oder mehrere Halogenatome substituiert ist;
eine 3 bis 6 Kohlenstoffatome enthaltende Cycloalkylgruppe, welche wahlweise durch eine oder mehrere R³-Gruppen oder ein oder mehrere Halogenatome oder eine Estergruppe, CO₂R³¹, substituiert ist;
eine 5 bis 6 Kohlenstoffatome enthaltende Cycloalkenylgruppe, die wahlweise durch eine oder mehrere R³-Gruppen oder ein oder mehrere Halogenatome oder eine Estergruppe, CO₂R³¹, substituiert ist;
eine Aryl- oder Aralkylgruppe;
eine Gruppe CO₂R³;
eine Aldehyd- oder Acylgruppe COR³;
eine Cyanogruppe;
eine Nitrogruppe;
eine Aminogruppe NR³R⁴; oder
ein Halogenatom;
R² bedeutet:
eine Nitrogruppe;
eine Cyanogruppe; oder
ein Halogenatom;
eine Gruppe R⁵;
eine Sulfenyl-, Sulfinyl- oder Sulfonylgruppe S(O)ₘR⁵;
eine Sulfamoylgruppe SO₂NR³R⁴;
eine Gruppe CO₂R³;
eine Aldehyd- oder Acylgruppe COR³;
eine Carbamoyl- oder Thiocarbamoylgruppe CONR³R⁴ oder CSNR³R⁴;
eine Alkoxygruppe OR⁵; oder
eine Alkylgruppe (mit 1 bis 3 Kohlenstoffatomen), die durch OR⁵ substituiert ist;
R³ und R⁴, die gleich oder verschieden sein können, jeweils bedeuten:
ein Wasserstoffatom oder eine bis zu 6 Kohlenstoffatome enthaltende gerad- oder verzweigtkettige Alkylgruppe, die wahlweise durch ein oder mehrere Halogenatome substituiert ist, R³¹ die für R³ angegebene Bedeutung besitzt, aber nicht Wasserstoff darstellt;
R⁵ bedeutet:
eine bis zu 6 Kohlenstoffatome enthaltende gerad- oder verzweigtkettige Alkylgruppe, die wahlweise durch ein oder mehrere Halogenatome substituiert ist;
m den Wert Null, 1 oder 2 hat;
n eine ganze Zahl von 1 bis 5 ist;
mit der Maßgabe, daß
(a) wenn R¹ ein C₁₋₄-Alkylrest ist, n eine ganze Zahl von 1 bis 2 ist und R² aus der Gruppe ausgewählt ist, die aus einem Halogenatom, einer C₁₋₄-Alkyl-, C₁₋₄₋Alkoxy-, Trifluormethyl- und Trichlormethylgruppe besteht, die Verbindung nicht in einer Mischung mit einer Verbindung der Formel: 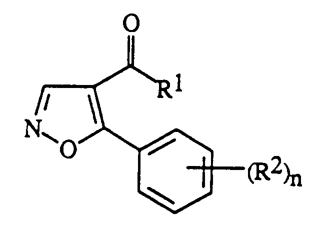 vorliegt,
worin R¹ ein C₁₋₄-Alkylrest ist, n eine ganze Zahl von 1 bis 3 ist und R² aus der Gruppe ausgewählt ist, die aus einem Halogenatom, einer C₁₋₄-Alkyl-, C₁₋₄-Alkoxy-, Trifluormethyl- und Trichlormethylgruppe besteht und
(b) wenn R¹ eine -NH₂-Gruppe ist, (R²)ₙ nicht die 4-Brom-Stellung bedeutet;
(c) wenn R¹ eine Methylgruppe ist, (R²)ₙ nicht eine 4-Fluor-Stellung bedeutet;
oder ein landwirtschaftlich annehmbares Salz davon.

2. Verbindung nach Anspruch 1, die eine Salz ist.

3. Verbindung nach Anspruch 1 oder 2, worin die Aryl- oder Aralkylgruppen der Formel (R²)_{q}-phenyl-[-CR³R⁴-]ₚ- entsprechen, worin q den Wert Null oder eine ganze Zahl von 1 bis 5 bedeutet und p den Wert Null oder 1 hat.

4. Verbindung, gemäß Anspruch 1, 2 oder 3, worin R¹ eine Alkyl- oder Cycloalkylgruppe ist und n den Wert 2 oder 3 hat.

5. Verbindung gemäß Anspruch 1, 2, 3 oder 4, worin eine der durch (R²)ₙ dargestellten Gruppen sich in ortho-Stellung am Phenylring befindet.

6. Eine Verbindung gemäß irgendeinem der Ansprüche 1 bis 5, worin R¹ eine wahlweise durch ein Halogenatom substituierte Alkylgruppe oder eine wahlweise durch Alkyl oder Halogen substituierte Cycloalkylgruppe ist.

7. Verbindung gemäß irgendeinem der Ansprüche 1 bis 6, worin R² Halogen, Nitro, R⁵, -S(O)ₘR⁵, -OR⁵, durch -OR⁵ substituiertes Alkyl oder -COOR³ bedeutet.

8. Verbindung gemäß irgendeinem der Ansprüche 1 bis 7, worin R⁵ eine wahlweise durch F oder Cl substituierte Alkylgruppe (mit 1 bis 3 Kohlenstoffatomen) bedeutet.

9. Verbindung nach Anspruch 1, welche ist:
5-Methyl-4-(2-nitro-4-trifluormethylbenzoyl)-isoxazol
5-Methyl-4-(2-nitrobenzoyl)-isoxazol
4-(2-Nitro-4-trifluormethylbenzoyl)-5-phenylisoxazol
4-(2,4-Dinitrobenzoyl)-5-methylisoxazol
5-(4-Chlorphenyl)-4-(2-nitro-4-trifluormethylbenzoyl)-isoxazol
4-(2-Chlorbenzoyl)-5-methylisoxazol
5-Methyl-4-(2-nitro-4-methylsulfonylbenzoyl)-isoxazol
5-(1-Methylethyl)-4-(2-nitro-4-trifluormethylbenzoyl)-isoxazol
4-(4-Chlorbenzoyl)-5-methylisoxazol
4-(4-Methylbenzoyl)-5-methylisoxazol
5-(4-Fluorphenyl)-4-(2-nitro-4-trifluormethylbenzoyl)-isoxazol
5-Ethyl-4-(2-nitro-4-trifluormethylbenzoyl)-isoxazol
4-(4-Chlor-2-nitrobenzoyl)-5-methylisoxazol
4-(2-Nitro-4-trifluormethylbenzoyl)-5-propylisoxazol
5-Cyclopropyl-4-(2-nitro-4-trifluormethylbenzoyl)-isoxazol
4-(2,3-Dichlor-4-methylsulfonylbenzoyl)-5-methylisoxazol
5-(1,1-Dimethylethyl)-4-(2-nitro-4-trifluormethylbenzoyl)-isoxazol
4-(4-Methoxybenzoyl)-5-methylisoxazol
5-Methyl-4-(4-methyl-2-nitrobenzoyl)-isoxazol
4-(2,3-Dichlor-4-methylsulfonylbenzoyl)-5-(1-methylethyl)-isoxazol
5-Cyclopropyl-4-(2,3-dichlor-4-methylsulfonylbenzoyl)-isoxazol
4-(2-Nitro-4-trifluormethylbenzoyl)-5-phenylmethylisoxazol
4-(2-Chlor-4-trifluormethylbenzoyl)-5-cyclopropylisoxazol
5-Methyl-4-(2-nitro-4-pentafluorethylbenzoyl)-isoxazol
5-Cyclopropyl-4-[4-(1,1-dimethylethyl)-2-nitrobenzoyl]-isoxazol
4-[4-(1,1-Dimethylethyl)-2-nitrobenzoyl]-5-methylisoxazol
5-Cylopentyl-4-(2-nitro-4-trifluormethylbenzoyl)isoxazol
4-(2,4-Dichlorbenzoyl)-5-methylisoxazol
4-(2-Chlor-4-methylsulfonylbenzoyl)-5-methylisoxazol
4-(2-Chlor-4-trifluormethylbenzoyl)-5-methylisoxazol
5-Methyl-4-(2-trifluormethylbenzoyl)-isoxazol
5-Methyl-4-(2,4-bis-trifluormethylbenzoyl)-isoxazol
4-(2-Chlor-4-methylsulfonylbenzoyl)-5-cyclopropylisoxazol
5-Cyclopropyl-4-(2-trifluormethylbenzoyl)isoxazol
5-Cycloproply-4-(2,4-dichlorbenzoyl)-isoxazol
4-[2,3-Dichlor-4-(methylthio)-benzoyl]-5-methylisoxazol
5-Cyclopropyl-4-(2,4-bis-trifluormethylbenzoyl)-isoxazol
4-(4-Chlor-2-trifluormethylbenzoyl)-5-methylisoxazol
4-(4-Cyano-2-nitrobenzoyl)-5-methylisoxazol
5-Amino-4-(2-nitro-4-trifluormethylbenzoyl)-isoxazol
4-(4-Chlor-2-trifluormethylbenzoyl)-5-cyclopropylisoxazol
5-(1-Methylethyl)-4-(2-nitro-4-pentafluorethylbenzoyl)-isoxazol
4-(2-Chlor-4-methylsulfonylbenzoyl)-5-(1-methylethyl)-isoxazol
5-Cyclopropyl-4-(4-fluor-2-nitrobenzoyl)-isoxazol
5-Cyclopropyl-4-(2-nitro-4-pentafluorethylbenzoyl)-isoxazol
4-(2,3-Dichlor-4-methylsulfinylbenzoyl)-5-methylisoxazol
5-Cyclobutyl-4-(2-nitro-4-trifluormethylbenzoyl)-isoxazol
4-(4-Fluor-2-nitrobenzoyl)-5-methylisoxazol
5-(1-Methylcyclopropyl)-4-(2-nitro-4-trifluormethylbenzoyl)-isoxazol
5-(4-Nitrophenyl)-4-(2-nitro-4-trifluormethylbenzoyl)-isoxazol
5-(4-Methoxyphenyl)-4-(2-nitro-4-trifluormethylbenzoyl)-isoxazol
4-(2-Chlor-3-ethoxy-4-methylsulfonylbenzoyl)-5-methylisoxazol
4-(3-Cyanobenzoyl)-5-methylisoxazol
5-Cyclopropyl-4-(4-methylsulfonyl-2-trifluormethylbenzoyl)-isoxazol
5-Cyclopropyl-4-(2-nitro-4-methylsulfonylbenzoyl)-isoxazol
4-(2-Chlor-3-ethoxy-4-methylsulfonylbenzoyl)-5-cyclopropylisoxazol
4-(2-Chlor-3-ethoxy-4-ethylsulfonylbenzoyl)-5-methylisoxazol
4-(2-Chlor-3-ethoxy-4-methylsulfonylbenzoyl)-5-(1-methylethyl)-isoxazol
4-(2-Chlor-3-ethoxy-4-ethylsulfonylbenzoyl)-5-cyclopropylisoxazol
5-(1-Ethoxycarbonylcyclopropyl)-4-(2-nitro-4-trifluormethylbenzoyl)-isoxazol
4-(3-Methoxycarbonyl-2-methyl-4-methylsulfonylbenzoyl)-5-methylisoxazol
5-(2-Methylcyclopropyl)-4-(2-nitro-4-trifluormethylbenzoyl)-isoxazol
4-[2-Chlor-3-(1-methylethoxy)-4-methylsulfonylbenzoyl]-5-methylisoxazol
5-Methyl-4-[2-methyl-3-(1-methylethoxycarbonyl)-4-methylsulfonylbenzoyl]-isoxazol
5-Cyclopropyl-4-[2-methyl-3-(1-methylethoxycarbonyl)-4-methylsulfonylbenzoyl]-isoxazol oder
5-Cyclopropyl-4-(2,3,4-trichlorbenzoyl)-isoxazol.

10. Eine herbizide Zusammensetzung, welche als Wirkstoff ein Isooxazol-Derivat der allgemeinen Formel III 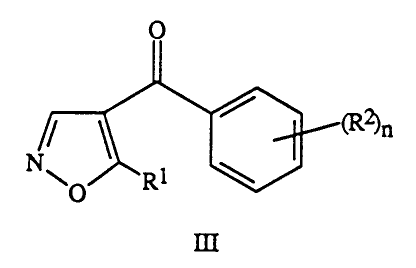 umfaßt, worin R¹ bedeutet:
eine bis zu 6 Kohlenstoffatome enthaltende gerad- oder verzweigtkettige Alkyl-, Alkenyl- oder Alkinylgruppe, welche wahlweise durch ein oder mehrere Halogenatome substituiert ist;
eine 3 bis 6 Kohlenstoffatome enthaltende Cycloalkylgruppe, welche wahlweise durch eine oder mehrere R³-Gruppen oder ein oder mehrere Halogenatome oder eine Estergruppe, CO₂R³¹, substituiert ist;
eine 5 bis 6 Kohlenstoffatome enthaltende Cycloalkenylgruppe, die wahlweise durch eine oder mehrere R³-Gruppen oder ein oder mehrere Halogenatome oder eine Estergruppe, CO₂R³¹, substituiert ist;
eine Aryl- oder Aralkylgruppe;
eine Gruppe CO₂R³;
eine Aldehyd- oder Acylgruppe COR³;
eine Cyanogruppe;
eine Nitrogruppe;
eine Aminogruppe NR³R⁴; oder
ein Halogenatom;
R² bedeutet:
eine Nitrogruppe;
eine Cyanogruppe; oder
ein Halogenatom;
eine Gruppe R⁵;
eine Sulfenyl-, Sulfinyl- oder Sulfonylgruppe S(O)ₘR⁵;
eine Sulphamoylgruppe SO₂NR³R⁴;
eine Gruppe CO₂R³;
eine Aldehyd- oder Acylgruppe COR³;
eine Carbamoyl- oder Thiocarbamoylgruppe CONR³R⁴ oder CSNR³R⁴;
eine Alkoxygruppe OR⁵; oder
eine Alkylgruppe (mit 1 bis 3 Kohlenstoffatomen), die durch OR⁵ substituiert ist;
R³ und R⁴, die gleich oder verschieden sein können, jeweils bedeuten:
ein Wasserstoffatom oder eine bis zu 6 Kohlenstoffatome enthaltende gerad- oder verzweigtkettige Alkylgruppe, die wahlweise durch ein oder mehrere Halogenatome substituiert ist, R³¹ die für R³ angegebene Bedeutung besitzt, aber nicht Wasserstoff darstellt;
R⁵ bedeutet:
eine bis zu 6 Kohlenstoffatome enthaltende gerad- oder verzweigtkettige Alkylgruppe, die wahlweise durch ein oder mehrere Halogenatome substituiert ist;
m den Wert Null, 1 oder 2 hat;
n eine ganze Zahl von 1 bis 5 ist;
in Verbindung mit einem landwirtschaftlich annehmbaren Verdünner oder Träger;
mit der Maßgabe, daß
wenn R¹ ein C₁₋₄-Alkylrest ist, n eine ganze Zahl von 1 bis 2 ist und R² aus der Gruppe ausgewählt ist, die aus einem Halogenatom, einer C₁₋₄-Alkyl-, C₁₋₄₋Alkoxy-, Trifluormethyl- und Trichlormethylgruppe besteht, die Verbindung nicht in einer Mischung mit einer Verbindung der Formel: 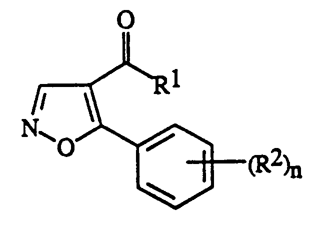 vorliegt, worin R¹ ein C₁₋₄-Alkylrest ist, n eine ganze Zahl von 1 bis 3 ist und R² aus der Gruppe ausgewählt ist, die aus einem Halogenatom, einer C₁₋₄-Alkyl-, C₁₋₄-Alkoxy-, Trifluormethyl- und Trichlormethylgruppe besteht.

11. Herbizide Zusammensetzung nach Anspruch 10, welche eine Verbindung nach irgendeinem der Ansprüche 3 bis 9 umfaßt.

12. Herbizide Zusammensetzung nach Anspruch 10 oder 11, welche von einschließlich 0,05 bis 90 Gew.% einer oder mehrerer Verbindung der allgemeinen Formel III enthält.

13. Herbizide Zusammensetzung nach irgendeinem der Ansprüche 10 bis 12, welche bis zu 15 % eines oberflächenaktiven Stoffes in flüssigen, emulgierbaren Suspensionskonzentraten und bis zu 25 % in flüssigen wasserlöslichen Konzentraten umfaßt.

14. Herbizide Zusammensetzung nach irgendeinem der Ansprüche 10 bis 13, die in Form eines Feststoffes, wie eines benetzbaren Pulvers oder Granulates, oder einer Flüssigkeit, wie einer Lösung oder einer Suspension oder einer Emulsion, vorliegt.

15. Herbizide Zusammensetzung nach irgendeinem der Ansprüche 10 bis 14, die des weiteren einen Hilfsstoff, ein Schutzkolloid, ein Verdickungsmittel, ein Durchdringungsmittel, einen Stabilisator, ein Sequestriermittel, ein das Zusammenbacken verhinderndes Mittel, ein Färbemittel oder einen Korrosionshemmstoff enthält.

16. Herbizide Zusammensetzung nach irgendeinem der Ansprüche 10 bis 15, die in Form
eines wäßrigen Suspensionskonzentrates, welches von 10 bis 70 % einer oder mehrerer Verbindungen der allgemeinen Formel III, von 2 bis 10 % eines oberflächenaktiven Stoffes, von 0,1 bis 5 % eines Verdickungsmittels und von 15 bis 87,9 % Wasser umfaßt,
eines benetzbaren Pulvers, welches von 10 bis 90 % einer oder mehrerer Verbindungen der allgemeinen Formel III, von 2 bis 10 % eines oberflächenaktiven Stoffes und von 8 bis 88 % eines festen Verdünners oder Trägers umfaßt,
eines löslichen Pulvers, welches von 10 bis 90 % einer oder mehrerer Verbindungen der allgemeinen Formel III, von 2 bis 40 % Natriumcarbonat und von 0 bis 88 % festen Verdünner umfaßt,
eines flüssigen, wasserlöslichen Konzentrats, welches von 5 bis 50 %, bevorzugt 10 bis 30 %, einer oder mehrerer Verbindungen der allgemeinen Formel III, von 5 bis 25 % eines oberflächenaktiven Stoffes und von 25 bis 90 % eines wassermischbaren Lösungsmittels oder einer Mischung aus wassermischbarem Lösungsmittel und Wasser umfaßt,
eines flüssigen emulgierbaren Suspensionskonzentrats, welches von 10 bis 70 % einer oder mehrerer Verbindungen der allgemeinen Formel III, von 5 bis 15 % eines oberflächenaktiven Stoffes, von 0,1 bis 5 % eines Verdickungsmittels und von 10 bis 84,9 % eines organischen Lösungsmittels umfaßt,
eines Granulates, welches von 1 bis 90 % einer oder mehrerer Verbindungen der allgemeinen Formel III, von 0,5 bis 7 %, bevorzugt 0,5 bis 2 %, eines oberflächenaktiven Stoffes und von 3 bis 98,5 % eines körnigen Trägers umfaßt, oder
eines emulgierbaren Konzentrates, welches von 0,05 bis 90 % einer oder mehrerer Verbindungen der allgemeinen Formel III, von 0,01 bis 10 % eines oberflächenaktiven Stoffes und von 9,99 bis 99,94 % eines organischen Lösungsmittels umfaßt, vorliegt.

17. Herstellungsgegenstand, welcher wenigstens eines der Isoxazol-Derivate irgendeines der Ansprüche 1 bis 9 oder eine herbizide Zusammensetzung gemäß irgendeines der Patentansprüche 10 bis 16, welche wenigstens eines der Isoxazol-Derivate der allgemeinen Formel III umfaßt, in einem Behälter für das vorgenannte Derivat oder die Derivate oder die vorgenannte herbizide Zusammensetzung und Instruktionen, die mit dem vorgenannten Behälter physikalisch verbunden sind, welche die Art und Weise angeben, in welcher das vorgenannte Derivat oder die Derivate oder eine herbizide Zusammensetzung, welche darin enthalten sind, zur Unkrautbekämpfung verwendet werden soll, umfaßt.

18. Verfahren zur Unkrautbekämpfung im Feld, welches die Anwendung einer herbizid wirksamen Menge eines Isoxazol-Derivates der allgemeinen Formel III im Feld umfaßt: 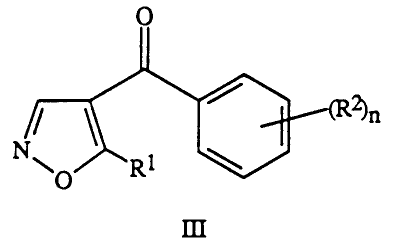 worin R¹ bedeutet:
eine bis zu 6 Kohlenstoffatome enthaltende gerad- oder verzweigtkettige Alkyl-, Alkenyl- oder Alkinylgruppe, welche wahlweise durch ein oder mehrere Halogenatome substituiert ist;
eine 3 bis 6 Kohlenstoffatome enthaltende Cycloalkylgruppe, welche wahlweise durch eine oder mehrere R³-Gruppen oder ein oder mehrere Halogenatome oder eine Estergruppe, CO₂R³¹, substituiert ist;
eine 5 bis 6 Kohlenstoffatome enthaltende Cycloalkenylgruppe, die wahlweise durch eine oder mehrere R³-Gruppen oder ein oder mehrere Halogenatome oder eine Estergruppe, CO₂R³¹, substituiert ist;
eine Aryl- oder Aralkylgruppe;
eine Gruppe CO₂R³;
eine Aldehyd- oder Acylgruppe COR³;
eine Cyanogruppe;
eine Nitrogruppe;
eine Aminogruppe NR³R⁴; oder
ein Halogenatom;
R² bedeutet:
eine Nitrogruppe;
eine Cyanogruppe; oder
ein Halogenatom;
eine Gruppe R⁵;
eine Sulfenyl-, Sulfinyl- oder Sulfonylgruppe S(O)ₘR⁵;
eine Sulphamoylgruppe SO₂NR³R⁴;
eine Gruppe CO₂R³;
eine Aldehyd- oder Acylgruppe COR³;
eine Carbamoyl- oder Thiocarbamoylgruppe CONR³R⁴ oder CSNR³R⁴;
eine Alkoxygruppe OR⁵; oder
eine Alkylgruppe (mit 1 bis 3 Kohlenstoffatomen), die durch OR⁵ substituiert ist;
R³ und R⁴, die gleich oder verschieden sein können, jeweils bedeuten:
ein Wasserstoffatom oder eine bis zu 6 Kohlenstoffatome enthaltende gerad- oder verzweigtkettige Alkylgruppe, die wahlweise durch ein oder mehrere Halogenatome substituiert ist, R³¹ die für R³ angegebene Bedeutung besitzt, aber nicht Wasserstoff darstellt;
R⁵ bedeutet:
eine bis zu 6 Kohlenstoffatome enthaltende gerad- oder verzweigtkettige Alkylgruppe, die wahlweise durch ein oder mehrere Halogenatome substituiert ist;
m den Wert Null, 1 oder 2 hat;
n eine ganze Zahl von 1 bis 5 ist,
oder ein landwirtschaftlich annehmbares Salz davon.

19. Verfahren nach Anspruch 18, worin die Auftragsrate des Isoxazol-Derivates der allgemeinen Formel III oder eines landwirtschaftlich annehmbaren Salzes davon von 0,01 kg bis 20 kg pro Hektar beträgt.

20. Verfahren nach Anspruch 19 zur selektiven Unkrautbekämpfung bei Feldfrüchten, worin die Auftragsrate von 0,01 kg bis 8,0 kg pro Hektar beträgt.

21. Verfahren nach Anspruch 20, zur selektiven Unkrautbekämpfung bei Feldfrüchten, worin die Auftragsrate von 0,1 kg bis 4 kg pro Hektar beträgt.

22. Verfahren nach Anspruch 18, durch Vor- oder Nachauflauf-Auftrag in eingerichteten Obstgärten oder anderen Baumzucht-Anlagen oder Pflanzungen, vor oder nach einem Pflanzen der Bäume oder Pflanzungen, in einer Auftragsrate von 0,25 kg bis 10 kg.

23. Verfahren nach Anspruch 22, worin die Auftragsrate von 0,5 kg bis 8,0 kg pro Hektar beträgt.

24. Verfahren zur Herstellung eines wie in Anspruch 1 definierten Isoxazol-Derivates der allgemeinen Formel III, mit der Maßgabe, daß R¹ nicht eine Cyanogruppe, eine Nitrogruppe, eine Aminogruppe oder ein Halogenatom ist, welches die Umsetzung einer Verbindung der allgemeinen Formel IV 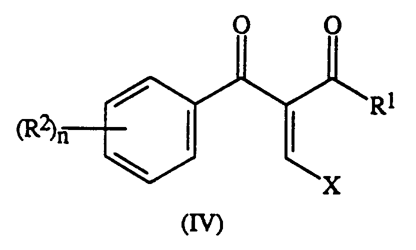 worin R¹, R² und n die in Anspruch 1 angegebene Bedeutung besitzen, mit der Maßgabe, daß R¹ nicht eine Cyano-, Nitro-, Aminogruppe oder ein Halogenatom ist und X ein O-Alkyl oder N-Dialkylrest ist, mit einem Salz von Hydroxylamin in einem Lösungsmittel, wahlweise in Gegenwart einer Base, umfaßt.

25. Verfahren nach Anspruch 24, worin das Lösungsmittel Ethanol oder Acetonitril ist.

26. Verfahren zur Herstellung eines wie in Anspruch 1 definierten Isoxazol-Derivates der allgemeinen Formel III, welches umfaßt:
(a) wenn R² ein Halogenatom, eine Alkylgruppe, eine Alkylthiogruppe oder eine Alkoxygruppe ist, Umsetzen einer Verbindung der allgemeinen Formel V 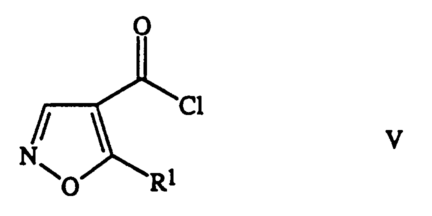 worin R¹ die in Anspruch 1 angegebene Bedeutung besitzt, mit einem Überschuß des geeignet substituierten Benzoles in Gegenwart eines Lewis-Säure-Katalysators, bei einer Temperatur zwischen Raumtemperatur und 100°C;
(b) wenn R¹ eine unsubstituierte Aminogruppe ist, Umsetzen einer Verbindung der allgemeinen Formel VI 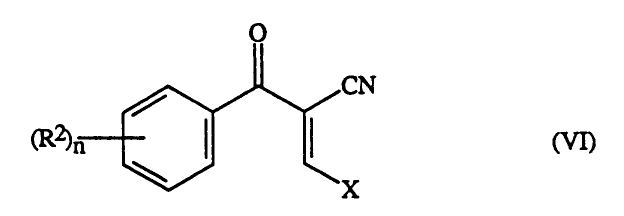 worin R² und n die in Anspruch angegebene Bedeutung besitzen und X ein O-Alkyl- oder N-Dialkylrest ist, mit einem Salz von Hydroxylamin in einem Lösungsmittel, wahlweise in Gegenwart einer Base, bei einer Temperatur zwischen Raumtemperatur und der Rückflußtemperatur des Lösungsmittels;
(c) wenn R¹ eine Cyanogruppe ist, Umwandeln in die Cyanoverbindung der entsprechenden Verbindung der allgemeinen Formel III, in welcher R¹ eine Estergruppe ist, durch saure Hydrolyse der Carbonsäure, gefolgt von einer Umwandlung in das Säurechlorid, wobei das Säurechlorid wahlweise in das Amid umgewandelt wird und das Amid durch Dehydratisierung wahlweise in die Cyanogruppe umgewandelt wird;
(d) wenn R¹ eine Acylgruppe ist, Umwandeln in die Acylverbindung der entsprechenden Verbindung der allgemeinen Formel III, in welcher R¹ eine Cyanogruppe ist, durch Umsetzung mit einem organometallischen Reagens;
(e) wenn R¹ eine Nitrogruppe ist, Oxidieren der entsprechenden Verbindung der allgemeinen Formel III, in welcher R¹ eine unsubstituierte Aminogruppe ist;
(f) wenn R¹ ein Halogenatom ist, Umwandlung in die Halogenverbindung durch Diazotierung der entsprechenden Verbindung der Formel III, in welcher R¹ eine unsubstituierte Aminogruppe ist;
(g) wenn R¹ eine substituierte Aminogruppe ist, Ersatz des Halogenatomes der entsprechenden Verbindung der allgemeinen Formel III, unter Verwendung des geeigneten Amines in einem inerten Lösungsmittel;
(h) wenn die Verbindung der allgemeinen Formel III eine Sulfinyl- oder Sulfonylgrupppe enthält, Oxidieren der entsprechenden Verbindung der allgemeinen Formel III, welche eine Thioethergruppe enthält.

27. Verbindung, die als Zwischenprodukt bei der Herstellung einer in irgendeinem der Ansprüche 1 bis 10 definierten Verbindung der allgemeinen Formel III, verwendet werden kann, gekennzeichnet durch die allgemeine Formel IV: 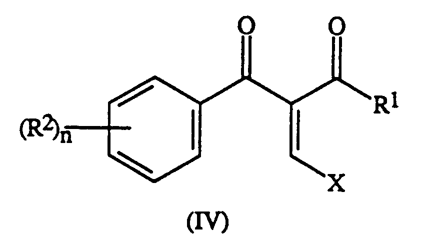 worin R¹ bedeutet:
eine bis zu 6 Kohlenstoffatome enthaltende, gerad- oder verzweigtkettige Alkyl-, Alkenyl- oder Alkinylgruppe, welche wahlweise durch eines oder mehrere Halogenatome substituiert ist;
eine 3 bis 6 Kohlenstoffatome enthaltende Cycloalkylgruppe, welche wahlweise durch eine oder mehrere R³-Gruppen oder eines oder mehrere Halogenatome oder eine Estergruppe CO₂R³¹ substituiert ist;
eine 5 bis 6 Kohlenstoffatome enthaltende Cycloalkenylgruppe, welche wahlweise durch eine oder mehrere R³-Gruppen oder ein oder mehrere Halogenatome oder eine Estergruppe CO₂R³¹ substituiert ist;
eine Aryl- oder Aralkylgruppe;
eine Gruppe CO₂R³;
eine Aldehyd- oder Acylgruppe, COR³;
eine Cyanogruppe;
eine Nitrogruppe;
eine Aminogruppe NR³R⁴ oder
ein Halogenatom;
R² bedeutet:
eine Nitrogruppe; oder
eine Cyanogruppe; oder
ein Halogenatom; oder
eine Gruppe R⁵; oder
eine Sulfenyl-, Sulfinyl- oder Sulfonylgruppe S(O)ₘR⁵;
oder
eine Sulphamoylgruppe SO₂NR³R⁴; oder
eine Gruppe CO₂R³; oder
eine Aldehyd- oder Acylgruppe COR³; oder
eine Carbamoyl- oder Thiocarbamoylgruppe CONR³R⁴ oder
CSNR³R⁴; eine Alkoxygruppe OR⁵; oder
eine durch OR⁵ substituierte Alkylgruppe (mit 1 bis 3 Kohlenstoffatomen),
R³ und R⁴, welche gleich oder verschieden sein können, jeweils bedeuten:
ein Wasserstoffatom oder eine bis zu 6 Kohlenstoffatomen enthaltende gerad- oder verzweigtkettige Alkylgruppe, welche wahlweise durch ein oder mehrere Halogenatome substituiert ist;
R³¹ die für R³ angegebene Bedeutung besitzt, aber nicht Wasserstoff ist;
R⁵ bedeutet:
eine bis zu 6 Kohlenstoffatome enthaltende gerad- oder verzweigtkettige Alkylgruppe, welche wahlweise durch ein oder mehrere Halogenatome substituiert ist,
m die Werte Null, 1 oder 2 besitzt,
n eine ganze Zahl von 1 bis 5 ist, und
X einen O-Alkyl- oder N-Dialklyrest darstellt, mit der Maßgabe, daß
(a) wenn X ein -N(CH₃)₂-Rest ist, R² aus der Gruppe ausgewählt ist, die aus einem Halogenatom, einer C₁₋₄-Alkyl-, C₁₋₄-Alkoxy-, Trifluormethyl- und Trichlormethylgruppe besteht und n eine ganze Zahl von 1 bis 3 ist; R¹ ist keine C₁₋₄-Alkylgruppe;
(b) wenn X und R¹ ein -N(CH₃)₂-Rest sind, (R²)ₙ nicht ein 2,4- Dichlor-5-Fluor-Rest ist und
(c) wenn X ein -N(CH₃)₂-Rest und R¹ ein Methylrest ist, (R²)ₙ nicht ein 4-Chlor- oder 4-Methoxy-Rest ist.

## Patentansprüche (Patentansprüche für folgende(n) Vertragsstaat(en): ES)

1. Ein Verfahren zur Herstellung eines Isoxazol-Derivats der allgemeinen Formel III oder eines landwirtschaftlich annehmbaren Salzes davon, 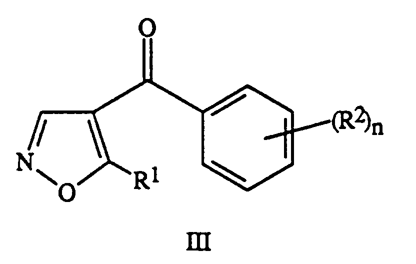 worin R¹ bedeutet:
eine bis zu 6 Kohlenstoffatome enthaltende gerad- oder verzweigtkettige Alkyl-, Alkenyl- oder Alkinylgruppe, welche wahlweise durch ein oder mehrere Halogenatome substituiert ist;
eine 3 bis 6 Kohlenstoffatome enthaltende Cycloalkylgruppe, welche wahlweise durch eine oder mehrere R³-Gruppen oder ein oder mehrere Halogenatome oder eine Estergruppe, CO₂R³¹, substituiert ist;
eine 5 bis 6 Kohlenstoffatome enthaltende Cycloalkenylgruppe, die wahlweise durch eine oder mehrere R³-Gruppen oder ein oder mehrere Halogenatome oder eine Estergruppe, CO₂R³¹, substituiert ist;
eine Aryl- oder Aralkylgruppe;
eine Gruppe CO₂R³;
eine Aldehyd- oder Acylgruppe COR³;
eine Cyanogruppe;
eine Nitrogruppe;
eine Aminogruppe NR³R⁴; oder
ein Halogenatom;
R² bedeutet:
eine Nitrogruppe;
eine Cyanogruppe; oder
ein Halogenatom;
eine Gruppe R⁵;
eine Sulfenyl-, Sulfinyl- oder Sulfonylgruppe S(O)ₘR⁵;
eine Sulfamoylgruppe SO₂NR³R⁴;
eine Gruppe CO₂R³;
eine Aldehyd- oder Acylgruppe COR³;
eine Carbamoyl- oder Thiocarbamoylgruppe CONR³R⁴ oder CSNR³R⁴;
eine Alkoxygruppe OR⁵; oder
eine Alkylgruppe (mit 1 bis 3 Kohlenstoffatomen), die durch OR⁵ substituiert ist;
R³ und R⁴, die gleich oder verschieden sein können, jeweils bedeuten:
ein Wasserstoffatom oder eine bis zu 6 Kohlenstoffatome enthaltende gerad- oder verzweigtkettige Alkylgruppe, die wahlweise durch ein oder mehrere Halogenatome substituiert ist, R³¹ die für R³ angegebene Bedeutung besitzt, aber nicht Wasserstoff darstellt;
R⁵ bedeutet:
eine bis zu 6 Kohlenstoffatome enthaltende gerad- oder verzweigtkettige Alkylgruppe, die wahlweise durch ein oder mehrere Halogenatome substituiert ist;
m den Wert Null, 1 oder 2 hat;
n eine ganze Zahl von 1 bis 5 ist;
mit der Maßgabe, daß
(a) wenn R¹ ein C₁₋₄-Alkylrest ist, n eine ganze Zahl von 1 bis 2 ist und R² aus der Gruppe ausgewählt ist, die aus einem Halogenatom, einer C₁₋₄-Alkyl-, C₁₋₄₋Alkoxy-, Trifluormethyl- und Trichlormethylgruppe besteht, die Verbindung nicht in einer Mischung mit einer Verbindung der Formel: 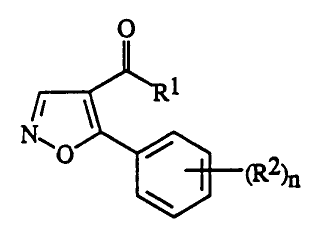 vorliegt,
worin R¹ ein C₁₋₄-Alkylrest ist, n eine ganze Zahl von 1 bis 3 ist und R² aus der Gruppe ausgewählt ist, die aus einem Halogenatom, einer C₁₋₄-Alkyl-, C₁₋₄-Alkoxy-, Trifluormethyl- und Trichlormethylgruppe besteht und
(b) wenn R¹ eine -NH₂-Gruppe ist, (R²)ₙ nicht die 4-Brom-Stellung bedeutet;
(c) wenn R¹ eine Methylgruppe ist, (R²)ₙ nicht eine 4-Fluor-Stellung bedeutet;
oder ein landwirtschaftlich annehmbares Salz davon, umfassend:
(a) the Umsetzung einer Verbindung der allgemeinen Formel IV 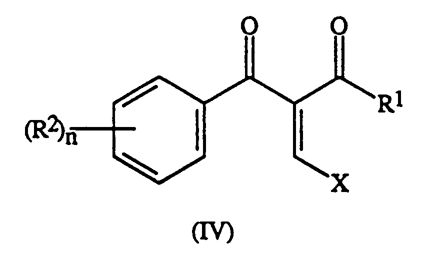 worin R¹, R² und n die oben angegebene Bedeutung besitzen, mit der Maßgabe, daß R¹ keine Cyano, Nitro, Amino-Gruppe oder kein Halogenatom ist, und X ein O-Alkyl- oder N-Dialkylrest ist, mit einem Salz von Hydroxylamin in einem Lösungsmittel, wahlweise in Gegenwart einer Base;
(b) wenn R² ein Halogenatom, eine Alkylgruppe, eine Alkylthiogruppe oder eine Alkoxygruppe ist, Umsetzen einer Verbindung der allgemeinen Formel V 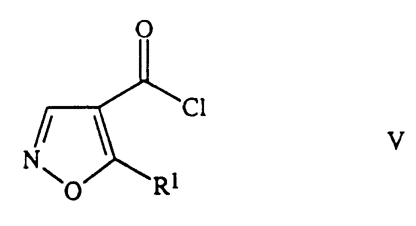 worin R¹ die in Anspruch 1 angegebene Bedeutung besitzt, mit einem Überschuß des geeignet substituierten Benzoles in Gegenwart eines Lewis-Säure-Katalysators, bei einer Temperatur zwischen Raumtemperatur und 100°C;
(c) wenn R¹ eine unsubstituierte Aminogruppe ist, Umsetzen einer Verbindung der allgemeinen Formel VI 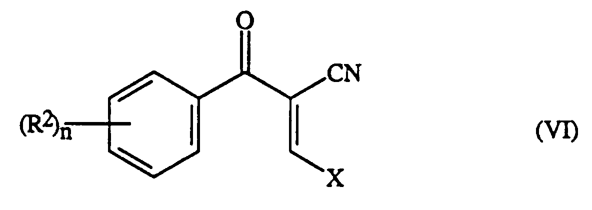 worin R² und n die in Anspruch angegebene Bedeutung besitzen und X ein O-Alkyl- oder N-Dialkylrest ist, mit einem Salz von Hydroxylamin in einem Lösungsmittel, wahlweise in Gegenwart einer Base, bei einer Temperatur zwischen Raumtemperatur und der Rückflußtemperatur des Lösungsmittels;
(d) wenn R¹ eine Cyanogruppe ist, Umwandeln in die Cyanoverbindung der entsprechenden Verbindung der allgemeinen Formel III, in welcher R¹ eine Estergruppe ist, durch saure Hydrolyse der Carbonsäure, gefolgt von einer Umwandlung in das Säurechlorid, wobei das Säurechlorid wahlweise in das Amid umgewandelt wird und das Amid durch Dehydratisierung wahlweise in die Cyanogruppe umgewandelt wird;
(e) wenn R¹ eine Acylgruppe ist, Umwandeln in die Acylverbindung der entsprechenden Verbindung der allgemeinen Formel III, in welcher R¹ eine Cyanogruppe ist, durch Umsetzung mit einem organometallischen Reagens;
(f) wenn R¹ eine Nitrogruppe ist, Oxidieren der entsprechenden Verbindung der allgemeinen Formel III, in welcher R¹ eine unsubstituierte Aminogruppe ist;
(g) wenn R¹ ein Halogenatom ist, Umwandlung in die Halogenverbindung durch Diazotierung der entsprechenden Verbindung der Formel III, in welcher R¹ eine unsubstituierte Aminogruppe ist;
(h) wenn R¹ eine substituierte Aminogruppe ist, Ersatz des Halogenatomes der entsprechenden Verbindung der allgemeinen Formel III, unter Verwendung des geeigneten Amines in einem inerten Lösungsmittel;
(i) wenn die Verbindung der allgemeinen Formel III eine Sulfinyl- oder Sulfonylgrupppe enthält, Oxidieren der entsprechenden Verbindung der allgemeinen Formel III, welche eine Thioethergruppe enthält.

2. Ein Verfahren nach Anspruch 1, zur Herstellung eines Salzes.

3. Ein Verfahren nach Anspruch 1 oder 2, worin die Aryl- oder Aralkylgruppen der Formel (R²)_{q}-phenyl-[-CR³R⁴-]ₚ- entsprechen, worin q den Wert Null oder eine ganze Zahl von 1 bis 5 bedeutet und p den Wert Null oder 1 hat.

4. Verfahren nach Anspruch 1, 2 oder 3, worin R¹ eine Alkyl- oder Cycloalkylgruppe ist und n den Wert 2 oder 3 hat.

5. Verfahren nach Anspruch 1, 2, 3 oder 4, worin eine der durch (R²)ₙ dargestellten Gruppen sich in ortho-Stellung am Phenylring befindet.

6. Verfahren nach irgendeinem der Ansprüche 1 bis 5, worin R¹ eine wahlweise durch ein Halogenatom substituierte Alkylgruppe oder eine wahlweise durch Alkyl oder Halogen substituierte Cycloalkylgruppe ist.

7. Verfahren nach irgendeinem der Ansprüche 1 bis 6, worin R² Halogen, Nitro, R⁵, -S(O)ₘR⁵, -OR⁵, durch -OR⁵ substituiertes Alkyl oder -COOR³ bedeutet.

8. Verfahren nach irgendeinem der Ansprüche 1 bis 7, worin R⁵ eine wahlweise durch F oder Cl substituierte Alkylgruppe (mit 1 bis 3 Kohlenstoffatomen) bedeutet.

9. Verfahren nach Anspruch 1, welche ist:
5-Methyl-4-(2-nitro-4-trifluormethylbenzoyl)-isoxazol
5-Methyl-4-(2-nitrobenzoyl)-isoxazol
4-(2-Nitro-4-trifluormethylbenzoyl)-5-phenylisoxazol
4-(2,4-Dinitrobenzoyl)-5-methylisoxazol
5-(4-Chlorphenyl)-4-(2-nitro-4-trifluormethylbenzoyl)-isoxazol
4-(2-Chlorbenzoyl)-5-methylisoxazol
5-Methyl-4-(2-nitro-4-methylsulfonylbenzoyl)-isoxazol
5-(1-Methylethyl)-4-(2-nitro-4-trifluormethylbenzoyl)-isoxazol
4-(4-Chlorbenzoyl)-5-methylisoxazol
4-(4-Methylbenzoyl)-5-methylisoxazol
5-(4-Fluorphenyl)-4-(2-nitro-4-trifluormethylbenzoyl)-isoxazol
5-Ethyl-4-(2-nitro-4-trifluormethylbenzoyl)-isoxazol
4-(4-Chlor-2-nitrobenzoyl)-5-methylisoxazol
4-(2-Nitro-4-trifluormethylbenzoyl)-5-propylisoxazol
5-Cyclopropyl-4-(2-nitro-4-trifluormethylbenzoyl)-isoxazol
4-(2,3-Dichlor-4-methylsulfonylbenzoyl)-5-methylisoxazol
5-(1,1-Dimethylethyl)-4-(2-nitro-4-trifluormethylbenzoyl)-isoxazol
4-(4-Methoxybenzoyl)-5-methylisoxazol
5-Methyl-4-(4-methyl-2-nitrobenzoyl)-isoxazol
4-(2,3-Dichlor-4-methylsulfonylbenzoyl)-5-(1-methylethyl)-isoxazol
5-Cyclopropyl-4-(2,3-dichlor-4-methylsulfonylbenzoyl)-isoxazol
4-(2-Nitro-4-trifluormethylbenzoyl)-5-phenylmethylisoxazol
4-(2-Chlor-4-trifluormethylbenzoyl)-5-cyclopropylisoxazol
5-Methyl-4-(2-nitro-4-pentafluorethylbenzoyl)-isoxazol
5-Cyclopropyl-4-[4-(1,1-dimethylethyl)-2-nitrobenzoyl]-isoxazol
4-[4-(1,1-Dimethylethyl)-2-nitrobenzoyl]-5-methylisoxazol
5-Cylopentyl-4-(2-nitro-4-trifluormethylbenzoyl)isoxazol
4-(2,4-Dichlorbenzoyl)-5-methylisoxazol
4-(2-Chlor-4-methylsulfonylbenzoyl)-5-methylisoxazol
4-(2-Chlor-4-trifluormethylbenzoyl)-5-methylisoxazol
5-Methyl-4-(2-trifluormethylbenzoyl)-isoxazol
5-Methyl-4-(2,4-bis-trifluormethylbenzoyl)-isoxazol
4-(2-Chlor-4-methylsulfonylbenzoyl)-5-cyclopropylisoxazol
5-Cyclopropyl-4-(2-trifluormethylbenzoyl)isoxazol
5-Cycloproply-4-(2,4-dichlorbenzoyl)-isoxazol
4-[2,3-Dichlor-4-(methylthio)-benzoyl]-5-methylisoxazol
5-Cyclopropyl-4-(2,4-bis-trifluormethylbenzoyl)-isoxazol
4-(4-Chlor-2-trifluormethylbenzoyl)-5-methylisoxazol
4-(4-Cyano-2-nitrobenzoyl)-5-methylisoxazol
5-Amino-4-(2-nitro-4-trifluormethylbenzoyl)-isoxazol
4-(4-Chlor-2-trifluormethylbenzoyl)-5-cyclopropylisoxazol
5-(1-Methylethyl)-4-(2-nitro-4-pentafluorethylbenzoyl)-isoxazol
4-(2-Chlor-4-methylsulfonylbenzoyl)-5-(1-methylethyl)-isoxazol
5-Cyclopropyl-4-(4-fluor-2-nitrobenzoyl)-isoxazol
5-Cyclopropyl-4-(2-nitro-4-pentafluorethylbenzoyl)-isoxazol
4-(2,3-Dichlor-4-methylsulfinylbenzoyl)-5-methylisoxazol
5-Cyclobutyl-4-(2-nitro-4-trifluormethylbenzoyl)-isoxazol
4-(4-Fluor-2-nitrobenzoyl)-5-methylisoxazol
5-(1-Methylcyclopropyl)-4-(2-nitro-4-trifluormethylbenzoyl)-isoxazol
5-(4-Nitrophenyl)-4-(2-nitro-4-trifluormethylbenzoyl)-isoxazol
5-(4-Methoxyphenyl)-4-(2-nitro-4-trifluormethylbenzoyl)-isoxazol
4-(2-Chlor-3-ethoxy-4-methylsulfonylbenzoyl)-5-methylisoxazol
4-(3-Cyanobenzoyl)-5-methylisoxazol
5-Cyclopropyl-4-(4-methylsulfonyl-2-trifluormethylbenzoyl)-isoxazol
5-Cyclopropyl-4-(2-nitro-4-methylsulfonylbenzoyl)-isoxazol
4-(2-Chlor-3-ethoxy-4-methylsulfonylbenzoyl)-5-cyclopropylisoxazol
4-(2-Chlor-3-ethoxy-4-ethylsulfonylbenzoyl)-5-methylisoxazol
4-(2-Chlor-3-ethoxy-4-methylsulfonylbenzoyl)-5-(1-methylethyl)-isoxazol
4-(2-Chlor-3-ethoxy-4-ethylsulfonylbenzoyl)-5-cyclopropylisoxazol
5-(1-Ethoxycarbonylcyclopropyl)-4-(2-nitro-4-trifluormethylbenzoyl)-isoxazol
4-(3-Methoxycarbonyl-2-methyl-4-methylsulfonylbenzoyl)-5-methylisoxazol
5-(2-Methylcyclopropyl)-4-(2-nitro-4-trifluormethylbenzoyl)-isoxazol
4-[2-Chlor-3-(1-methylethoxy)-4-methylsulfonylbenzoyl]-5-methylisoxazol
5-Methyl-4-[2-methyl-3-(1-methylethoxycarbonyl)-4-methylsulfonylbenzoyl]-isoxazol
5-Cyclopropyl-4-[2-methyl-3-(1-methylethoxycarbonyl)-4-methylsulfonylbenzoyl]-isoxazol oder
5-Cyclopropyl-4-(2,3,4-trichlorbenzoyl)-isoxazol.

10. Ein Verfahren zur Herstellung einer herbiziden Zusammensetzung, umfassend eine Formulierung eines Isoxazol-Derivates der allgemeinen Formel III 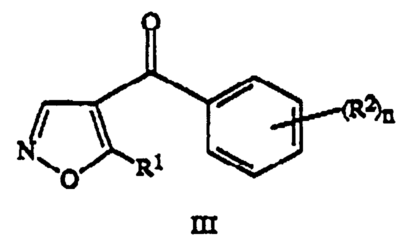 worin R¹ bedeutet:
eine bis zu 6 Kohlenstoffatome enthaltende gerad- oder verzweigtkettige Alkyl-, Alkenyl- oder Alkinylgruppe, welche wahlweise durch ein oder mehrere Halogenatome substituiert ist;
eine 3 bis 6 Kohlenstoffatome enthaltende Cycloalkylgruppe, welche wahlweise durch eine oder mehrere R³-Gruppen oder ein oder mehrere Halogenatome oder eine Estergruppe, CO₂R³¹, substituiert ist;
eine 5 bis 6 Kohlenstoffatome enthaltende Cycloalkenylgruppe, die wahlweise durch eine oder mehrere R³-Gruppen oder ein oder mehrere Halogenatome oder eine Estergruppe, CO₂R³¹, substituiert ist;
eine Aryl- oder Aralkylgruppe;
eine Gruppe CO₂R³;
eine Aldehyd- oder Acylgruppe COR³;
eine Cyanogruppe;
eine Nitrogruppe;
eine Aminogruppe NR³R⁴; oder
ein Halogenatom;
R² bedeutet:
eine Nitrogruppe;
eine Cyanogruppe; oder
ein Halogenatom;
eine Gruppe R⁵;
eine Sulfenyl-, Sulfinyl- oder Sulfonylgruppe S(O)ₘR⁵;
eine Sulphamoylgruppe SO₂NR³R⁴;
eine Gruppe CO₂R³;
eine Aldehyd- oder Acylgruppe COR³;
eine Carbamoyl- oder Thiocarbamoylgruppe CONR³R⁴ oder CSNR³R⁴;
eine Alkoxygruppe OR⁵; oder
eine Alkylgruppe (mit 1 bis 3 Kohlenstoffatomen), die durch OR⁵ substituiert ist;
R³ und R⁴, die gleich oder verschieden sein können, jeweils bedeuten:
ein Wasserstoffatom oder eine bis zu 6 Kohlenstoffatome enthaltende gerad- oder verzweigtkettige Alkylgruppe, die wahlweise durch ein oder mehrere Halogenatome substituiert ist, R³¹ die für R³ angegebene Bedeutung besitzt, aber nicht Wasserstoff darstellt;
R⁵ bedeutet:
eine bis zu 6 Kohlenstoffatome enthaltende gerad- oder verzweigtkettige Alkylgruppe, die wahlweise durch ein oder mehrere Halogenatome substituiert ist;
m den Wert Null, 1 oder 2 hat;
n eine ganze Zahl von 1 bis 5 ist;
in Verbindung mit einem landwirtschaftlich annehmbaren Verdünner oder Träger;
mit der Maßgabe, daß
wenn R¹ ein C₁₋₄-Alkylrest ist, n eine ganze Zahl von 1 bis 2 ist und R² aus der Gruppe ausgewählt ist, die aus einem Halogenatom, einer C₁₋₄-Alkyl-, C₁₋₄₋Alkoxy-, Trifluormethyl- und Trichlormethylgruppe besteht, die Verbindung nicht in einer Mischung mit einer Verbindung der Formel: 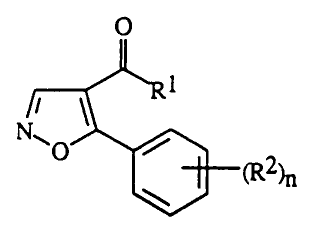 vorliegt, worin R¹ ein C₁₋₄-Alkylrest ist, n eine ganze Zahl von 1 bis 3 ist und R² aus der Gruppe ausgewählt ist, die aus einem Halogenatom, einer C₁₋₄-Alkyl-, C₁₋₄-Alkoxy-, Trifluormethyl- und Trichlormethylgruppe besteht.

11. Verfahren nach Anspruch 10, zur Herstellung einer herbiziden Zusammensetzung, welche von 0,05 bis 90 Gew.% einer oder mehrerer Verbindungen der allgemeinen Formel III enthält.

12. Verfahren nach Anspruch 10 oder 12 zur Herstellung einer herbiziden Zusammensetzung, welche bis zu 15 % eines oberflächenaktiven Stoffes in flüssigen, emulgierbaren Suspensionskonzentraten und bis zu 25 % in flüssigen, wasserlöslichen Konzentraten umfaßt.

13. Verfahren nach Anspruch 10, 11 oder 12 zur Herstellung einer herbiziden Zusammensetzung, die in Form eines Feststoffes, wie einem benetzbaren Pulver oder einem Granulat oder einer Flüssigkeit, wie einer Lösung oder einer Suspension oder einer Emulsion, vorliegt.

14. Ein Verfahren nach irgendeinem der Ansprüche 10 bis 13, zur Herstellung einer herbiziden Zusammensetzung, die des weiteren einen Hilfsstoff, ein Schutzkolloid, ein Verdickungsmittel, ein Durchdringungsmittel, einen Stabilisator, ein Sequestriermittel, ein das Zusammenbacken verhinderndes Mittel, ein Färbemittel oder einen Korrosionshemmstoff enthält.

15. Verfahren nach irgendeinem der Ansprüche 10 bis 14 zur Herstellung einer herbiziden Zusammensetzung, welche in Form:
eines wäßrigen Suspensionskonzentrates, welches von 10 bis 70 % einer oder mehrerer Verbindungen der allgemeinen Formel III, von 2 bis 10 % eines oberflächenaktiven Stoffes, von 0,1 bis 5 % eines Verdickungsmittels und von 15 bis 87,9 % Wasser umfaßt,
eines benetzbaren Pulvers, welches von 10 bis 90 % einer oder mehrerer Verbindungen der allgemeinen Formel III, von 2 bis 10 % eines oberflächenaktiven Stoffes und von 8 bis 88 % eines festen Verdünners oder Trägers umfaßt,
eines löslichen Pulvers, welches von 10 bis 90 % einer oder mehrerer Verbindungen der allgemeinen Formel III, von 2 bis 40 % Natriumcarbonat und von 0 bis 88 % festen Verdünner umfaßt,
eines flüssigen, wasserlöslichen Konzentrats, welches von 5 bis 50 %, bevorzugt 10 bis 30 %, einer oder mehrerer Verbindungen der allgemeinen Formel III, von 5 bis 25 % eines oberflächenaktiven Stoffes und von 25 bis 90 % eines wassermischbaren Lösungsmittels oder einer Mischung aus wassermischbarem Lösungsmittel und Wasser umfaßt,
eines flüssigen emulgierbaren Suspensionskonzentrats, welches von 10 bis 70 % einer oder mehrerer Verbindungen der allgemeinen Formel III, von 5 bis 15 % eines oberflächenaktiven Stoffes, von 0,1 bis 5 % eines Verdickungsmittels und von 10 bis 84,9 % eines organischen Lösungsmittels umfaßt,
eines Granulates, welches von 1 bis 90 % einer oder mehrerer Verbindungen der allgemeinen Formel III, von 0,5 bis 7 %, bevorzugt 0,5 bis 2 %, eines oberflächenaktiven Stoffes und von 3 bis 98,5 % eines körnigen Trägers umfaßt, oder
eines emulgierbaren Konzentrates, welches von 0,05 bis 90 % einer oder mehrerer Verbindungen der allgemeinen Formel III, von 0,01 bis 10 % eines oberflächenaktiven Stoffes und von 9,99 bis 99,94 % eines organischen Lösungsmittels umfaßt, vorliegt.

16. Verfahren zur Unkrautbekämpfung im Feld, welches die Anwendung einer herbizid wirksamen Menge eines Isoxazol-Derivates der allgemeinen Formel III im Feld umfaßt: 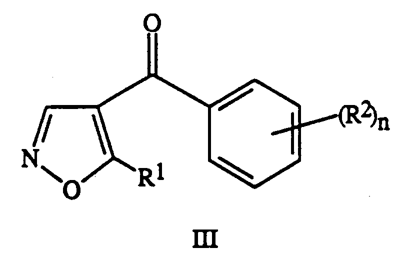 worin R¹ bedeutet:
eine bis zu 6 Kohlenstoffatome enthaltende gerad- oder verzweigtkettige Alkyl-, Alkenyl- oder Alkinylgruppe, welche wahlweise durch ein oder mehrere Halogenatome substituiert ist;
eine 3 bis 6 Kohlenstoffatome enthaltende Cycloalkylgruppe, welche wahlweise durch eine oder mehrere R³-Gruppen oder ein oder mehrere Halogenatome oder eine Estergruppe, CO₂R³¹, substituiert ist;
eine 5 bis 6 Kohlenstoffatome enthaltende Cycloalkenylgruppe, die wahlweise durch eine oder mehrere R³-Gruppen oder ein oder mehrere Halogenatome oder eine Estergruppe, CO₂R³¹, substituiert ist;
eine Aryl- oder Aralkylgruppe;
eine Gruppe CO₂R³;
eine Aldehyd- oder Acylgruppe COR³;
eine Cyanogruppe;
eine Nitrogruppe;
eine Aminogruppe NR³R⁴; oder
ein Halogenatom;
R² bedeutet:
eine Nitrogruppe;
eine Cyanogruppe; oder
ein Halogenatom;
eine Gruppe R⁵;
eine Sulfenyl-, Sulfinyl- oder Sulfonylgruppe S(O)ₘR⁵;
eine Sulphamoylgruppe SO₂NR³R⁴;
eine Gruppe CO₂R³;
eine Aldehyd- oder Acylgruppe COR³;
eine Carbamoyl- oder Thiocarbamoylgruppe CONR³R⁴ oder CSNR³R⁴;
eine Alkoxygruppe OR⁵; oder
eine Alkylgruppe (mit 1 bis 3 Kohlenstoffatomen), die durch OR⁵ substituiert ist;
R³ und R⁴, die gleich oder verschieden sein können, jeweils bedeuten:
ein Wasserstoffatom oder eine bis zu 6 Kohlenstoffatome enthaltende gerad- oder verzweigtkettige Alkylgruppe, die wahlweise durch ein oder mehrere Halogenatome substituiert ist, R³¹ die für R³ angegebene Bedeutung besitzt, aber nicht Wasserstoff darstellt;
R⁵ bedeutet:
eine bis zu 6 Kohlenstoffatome enthaltende gerad- oder verzweigtkettige Alkylgruppe, die wahlweise durch ein oder mehrere Halogenatome substituiert ist;
m den Wert Null, 1 oder 2 hat;
n eine ganze Zahl von 1 bis 5 ist,
oder ein landwirtschaftlich annehmbares Salz davon.

17. Verfahren nach Anspruch 16, worin die Auftragsrate des Isoxazol-Derivates der allgemeinen Formel III oder eines landwirtschaftlich annehmbaren Salzes davon von 0,01 kg bis 20 kg pro Hektar beträgt.

18. Verfahren nach Anspruch 17, zur selektiven Unkrautbekämpfung bei Feldfrüchten, worin die Auftragsrate von 0,01 kg bis 8,0 kg pro Hektar beträgt.

19. Verfahren nach Anspruch 18, zur selektiven Unkrautbekämpfung bei Feldfrüchten, worin die Auftragsrate von 0,1 kg bis 4 kg pro Hektar beträgt.

20. Verfahren nach Anspruch 16, durch Vor- oder Nachauflauf-Auftrag in eingerichteten Obstgärten oder anderen Baumzucht-Anlagen oder Pflanzungen, vor oder nach einem Pflanzen der Bäume oder Pflanzungen, in einer Auftragsrate von 0,25 kg bis 10 kg.

21. Verfahren nach Anspruch 20, worin die Auftragsrate von 0,5 kg bis 8,0 kg pro Hektar beträgt.

22. Ein Verfahren zur Herstellung eines Zwischenproduktes der Formel IV: 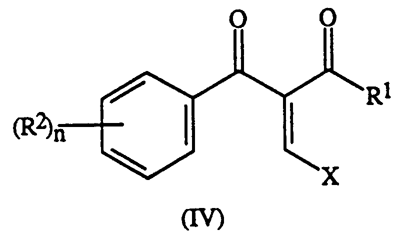 worin R¹ bedeutet:
eine bis zu 6 Kohlenstoffatome enthaltende, gerad- oder verzweigtkettige Alkyl-, Alkenyl- oder Alkinylgruppe, welche wahlweise durch eines oder mehrere Halogenatome substituiert ist;
eine 3 bis 6 Kohlenstoffatome enthaltende Cycloalkylgruppe, welche wahlweise durch eine oder mehrere R³-Gruppen oder eines oder mehrere Halogenatome oder eine Estergruppe CO₂R³¹ substituiert ist;
eine 5 bis 6 Kohlenstoffatome enthaltende Cycloalkenylgruppe, welche wahlweise durch eine oder mehrere R³-Gruppen oder ein oder mehrere Halogenatome oder eine Estergruppe CO₂R³¹ substituiert ist;
eine Aryl- oder Aralkylgruppe;
eine Gruppe CO₂R³;
eine Aldehyd- oder Acylgruppe, COR³;
eine Cyanogruppe;
eine Nitrogruppe;
eine Aminogruppe NR³R⁴ oder
ein Halogenatom;
R² bedeutet:
eine Nitrogruppe; oder
eine Cyanogruppe; oder
ein Halogenatom; oder
eine Gruppe R⁵; oder
eine Sulfenyl-, Sulfinyl- oder Sulfonylgruppe S(O)ₘR⁵; oder
eine Sulphamoylgruppe SO₂NR³R⁴; oder
eine Gruppe CO₂R³; oder
eine Aldehyd- oder Acylgruppe COR³; oder
eine Carbamoyl- oder Thiocarbamoylgruppe CONR³R⁴ oder
CSNR³R⁴; eine Alkoxygruppe OR⁵; oder
eine durch OR⁵ substituierte Alkylgruppe (mit 1 bis 3 Koh lenstoffatomen),
R³ und R⁴, welche gleich oder verschieden sein können, jeweils bedeuten:
ein Wasserstoffatom oder eine bis zu 6 Kohlenstoffatomen enthaltende gerad- oder verzweigtkettige Alkylgruppe, welche wahlweise durch ein oder mehrere Halogenatome substituiert ist;
R³¹ die für R³ angegebene Bedeutung besitzt, aber nicht Wasserstoff ist;
R⁵ bedeutet:
eine bis zu 6 Kohlenstoffatome enthaltende gerad- oder verzweigtkettige Alkylgruppe, welche wahlweise durch ein oder mehrere Halogenatome substituiert ist,
m die Werte Null, 1 oder 2 besitzt,
n eine ganze Zahl von 1 bis 5 ist, und
X einen O-Alkyl- oder N-Dialklyrest darstellt,
mit der Maßgabe, daß
(a) wenn X ein -N(CH₃)₂-Rest ist, R² aus der Gruppe ausgewählt ist, die aus einem Halogenatom, einer C₁₋₄-Alkyl-, C₁₋₄-Alkoxy-, Trifluormethyl- und Trichlormethylgruppe besteht und n eine ganze Zahl von 1 bis 3; R¹ ist keine C₁₋₄-Alkylgruppe;
(b) wenn X und R¹ ein -N(CH₃)₂-Rest sind, (R²)ₙ nicht ein 2,4-Dichlor-5-Fluor-Rest ist und
(c) wenn X ein -N(CH₃)₂-Rest und R¹ ein Methylrest ist, (R²)ₙ nicht ein 4-Chlor- oder 4-Methoxy-Rest ist, umfassend die Umsetzung eines Diketons der allgemeinen Formel VII 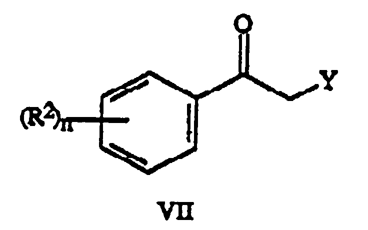 worin R² und n die oben angegebene Bedeutung besitzen, Y COR¹ ist und R¹ die oben angegebenen Bedeutung besitzt, mit
(i) einem Ortho-Ester in Gegenwart eines sauren Katalysators oder
(ii) einem Amidacetal in einem inerten Lösungsmittel.

## Revendications (Revendications pour l'(les) Etat(s) contractant(s) suivant(s): AT, BE, CH, DE, DK, FR, GB, GR, IT, LI, LU, NL, SE)

1. Un dérivé de l'isoxazole caractérisé par la formule générale III 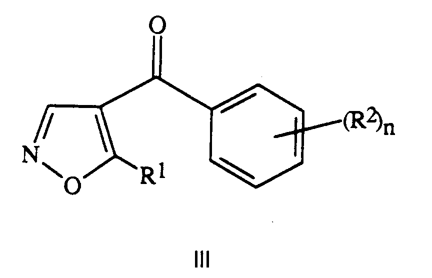 dans laquelle :
R¹ représente :
un radical alcoyle, alcényle ou alcynyle, linéaire ou ramifié, contenant de 1 à 6 atomes de carbone, éventuellement substitué par un ou plusieurs atomes d'halogènes ;
un radical cycloalkyle contenant de 3 à 6 atomes de carbone éventuellement substitué par un ou plusieurs radicaux R³ ou par un ou plusieurs atomes d'halogènes ou par un radical ester CO₂R³¹ ;
un radical cycloalkènyle contenant 5 ou 6 atomes de carbone éventuellement substitué par un ou plusieurs radicaux R³ ou par un ou plusieurs atomes d'halogènes ou par un radical ester CO₂R³¹ un radical aryle ou aralkyle ;
un radical CO₂R³ ;
un aldéhyde ou un radical acyle COR³
un radical cyano ;
un radical nitro ;
un radical amino NR³R⁴ ; ou
un atome d'halogène ;
R² représente
un radical nitro ;
un radical cyano ;
un atome d'halogène ;
un radical R⁵ ;
un radical sulfènyle, sulfinyle ou sulfonyle S(O)ₘR⁵ ;
un radical sulfamoyle SONR³R⁴ ;
un radical CO₂R³ ;
un aldéhyde ou un radical acyle COR³
un radical carbamoyle ou thiocarbamoyle CONR³R⁴ ou CSNR³R⁴ ;
un radical alcoxy OR⁵ ; ou
un radical alkyle contenant de 1 à 3 atomes de carbone substitué par un radical OR⁵ ;
R³ et R⁴ qui peuvent être identiques ou différents représentent chacun :
l'hydrogène ou un radical alkyle linéaire ou ramifié contenant de 1 à 6 atomes de carbone, qui est éventuellement substitué par un ou plusieurs atomes d'halogènes ;
R³¹ a la même définition que R³ mais ne peut pas être l'hydrogène ;
R⁵ représente :
un radical alkyle linéaire ou ramifié contenant de 1 à 6 atomes de carbone éventuellement substitué par un ou plusieurs atomes d'halogènes ;
m représente 0, 1 ou 2
n représente un nombre entier pouvant prendre toutes les valeurs de 1 à 5 ;
à condition que :
a) si R¹ représente un radical alkyle en C₁₋₄, n est un nombre entier égal à 1, 2 ou 3 et R² représente un radical choisi parmi les radicaux suivants, halogène, alkyle en C₁₋₄, alkoxy en C₁₋₄, trifluorométhyle et trichlorométhyle le composé ne soit pas présent dans un mélange avec un composé de formule générale 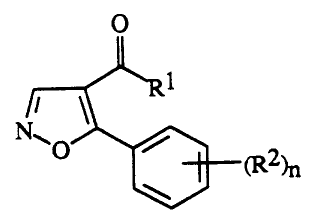 dans laquelle R¹ représente un radical alkyle en C₁₋₄, n est un nombre entier égal à 1, 2 ou 3 et R² représente un radical choisi parmi les radicaux suivants, halogène, alkyle en C₁₋₄, alkoxy en C₁₋₄, trifluorométhyle et trichlorométhyle ; et
(b) si R¹ représente -NH₂, (R²)ₙ ne soit pas un radical 4-bromo ; (c) si R¹ représente un radical méthyle, (R²)ₙ ne soit pas un radical 4-fluoro ; ou des sels de ces composés acceptables pour des usages agricoles.

2. Un composé selon la revendication 1 dans laquelle le composé est un sel.

3. Un composé selon la revendication 1 ou 2 dans laquelle un radical aryle ou aralkyle répond à la formule générale (R²)_{q}-phényl-[-CR³R⁴-]ₚ dans laquelle q représente zéro ou un nombre entier de 1 à 5 et p représente zéro ou 1.

4. Un composé selon l'une des revendications 1, 2 ou 3 dans laquelle R1¹ représente un radical alkyle ou cycloalkyle et n représente 2 ou 3.

5. Un composé selon l'une des revendications 1, 2, 3 ou 4 dans laquelle l'un des radicaux représentés par (R²)ₙ est en position ortho sur le noyau phényle.

6. Un composé selon l'une quelconque des revendications 1 à 5 dans laquelle radical R¹ est un radical alkyle éventuellement substitué par un halogène, ou un radical cycloalkyle éventuellement substitué par un radical alkyle ou un halogène.

7. Un composé selon l'une quelconque des revendications 1 à 6 dans laquelle R² est un halogène , un radical nitro ; R⁵ ; -S(O)ₘR⁵ ; -OR⁵ ou COOR³.

8. Un composé selon l'une quelconque des revendications 1 à 7 dans laquelle R⁵ est un radical alkyle (contenant de 1 à 3 atomes de carbone) éventuellement substitué par F ou Cl.

9. Un composé selon la revendication 1 qui est le :
5-méthyle-4-(2-nitro-4-trifluorométhylbenzoyl)-isoxazole
5-méthyle-4-(2-nitrobenzoyl)-isoxazole
4-(2-nitro-4-trifluorométhylbenzoyl)-5-phénylisoxazole
4-(2,4-dinitrobenzoyl)-5-méthylisoxazole
5-(4-chlorophényle)-4-(2-nitro-4-trifluorométhylbenzoyl)-isoxazole
4-(2-chlorobenzoyl)-5-méthylisoxazole
5-méthyle-4-(2-nitro-4-méthylsulfonylbenzoyl)-isoxazole
5-1-(méthyléthyl)-4-(2-nitro-4-trifluorométhylbenzoyl)-isoxazole
4-(4-chlorobenzoyle)-5-méthylisoxazole
4-(4-méthylbenzoyle)-5-méthylisoxazole
5-(4-fluorophényle)-4-(2-nitro-4-trifluorométhylbenzoyl)-isoxazole
5-éthyle-4-(2-nitro-4-trifluorométhylbenzoyl)-isoxazole
4-(4-chloro-2-nitrobenzoyle)-5-méthylisoxazole
4-(2-nitro-4-trifluorométhylbenzoyle)-5-propylisoxazole
5-cyclopropyl-4-(2-nitro-4-trifluorométhylbenzoyl)-isoxazole
4-(2,3-dichloro-4-méthylsulfonylbenzoyl)-5-méthylisoxazole
5-(1,1diméthyléthyle)-4-(2-nitro-4-trifluorométhylbenzoyl)-isoxazole
4-(4-méthoxybenzoyl)-5-méthylisoxazole
5-méthyle-4-(4-méthyl-2-nitrobenzoyl)-isoxazole
4-(2,3-dichloro-4-méthylsulfonylbenzoyl)-5-(1-méthyléthyl)-isoxazole
5-cyclopropyl-4-(2,3-dichloro-4-méthylsulfonylbenzoyl)-5-isoxazole
4-(2-nitro-4-trifluorométhylbenzoyle)-5-phénylméthylisoxazole
4-(2-chloro-4-trifluorométhylbenzoyle)-5-cyclopropylisoxazole
5-méthyle-4-(2-nitro-4-pentafluoroéthylbenzoyl)-isoxazole
5-cyclopropyl-4-[4-(1,1-diméthyléthyl)-2-nitrobenzoyl]-5-isoxazole
4-[4-(1,1-diméthyléthyl)-2-nitrobenzoyl]-5-méthylisoxazole
5-cyclopentyle-4-(2-nitro-4-trifluorométhylbenzoyl)-isoxazole
4-(2,4-dichlorobenzoyle)-5-méthylisoxazole
4-(2-chloro-4-méthylsulfonylbenzoyle)-5-méthylisoxazole
4-(2-chloro-4-trifluorométhylbenzoyle)-5-méthylisoxazole
5-méthyle-4-(2-trifluorométhylbenzoyl)-isoxazole
5-méthyle-4-(2,4-bis-trifluorométhylbenzoyl)-isoxazole
4-(2-chloro-4-méthylsulfonylbenzoyle)-5-cyclopropylisoxazole
5-cyclopropyl-4-(2-trifluorométhylbenzoyl)-isoxazole
5-cyclopropyl-4-(2,4-dichlorobenzoyl)-isoxazole
4-[2,3-dichloro-4-(méthylthio)-benzoyle]-5-méthylisoxazole
5-cyclopropyle-4-(2,4-bis-trifluorométhylbenzoyl)-isoxazole
4-(4-chloro-2-trifluorométhylbenzoyle)-5-méthylisoxazole
4-(4-cyano-2-nitrobenzoyle)-5-méthylisoxazole
5-amino-4-(2-nitro-4-trifluorométhylbenzoyl)-isoxazole
4-(4-chloro-2-trifluorométhylbenzoyle)-5-cyclopropylisoxazole
5-(1-méthyléthyl)-4-(2-nitro-4-pentafluoroéthylbenzoyl)-isoxazole
4-(2-chloro-4-méthylsulfonylbenzoyle)-5-(1-méthyléthyl)-isoxazole
5-cyclopropyle-4-(4-fluoro-2-nitrobenzoyl)-isoxazole
5-cyclopropyle-4-(2-nitro-4-pentafluoroéthylbenzoyl)-isoxazole
4-(2,3-dichloro-4-méthylsulfinylbenzoyle)-5-méthylisoxazole
5-cyclobutyle-4-(2-nitro-4-trifluorométhylbenzoyl)-isoxazole
4-(4-fluoro-2-nitrobenzoyle)-5-méthylisoxazole
5-(1-méthylcyclopropyl)-4-(2-nitro-4-trifluorométhylbenzoyl)-isoxazole
5-(4-nitrophényl)-4-(2-nitro-4-trifluorométhylbenzoyl)-isoxazole
5-(4-méthoxyphényl)-4-(2-nitro-4-trifluorométhylbenzoyl)-isoxazole
4-(2-chloro-3-éthoxy-4-méthylsulfonylbenzoyle)-5-méthylisoxazole
4-(3-cyanobenzoyle)-5-méthylisoxazole
5-cyclopropyle-4-(4-méthylsulfonyl-2-trifluorométhyle benzoyl)-isoxazole
5-cyclopropyle-4-(2-nitro-4-méthylsulfonylbenzoyl)-isoxazole
4-(2-chloro-3-éthoxy-4-méthylsulfonylbenzoyle)-5-cyclopropylisoxazole
4-(2-chloro-3-éthoxy-4-éthylsulfonylbenzoyle)-5-méthylisoxazole
4-(2-chloro-3-éthoxy-4-méthylsulfonylbenzoyle)-5-(1-méthyléthyl)-isoxazole
4-(2-chloro-3-éthoxy-4-éthylsulfonylbenzoyle)-5-cyclopropylisoxazole
5-(1-éthoxycarbonylcyclopropyl)-4-(2-nitro-4-trifluorométhylbenzoyl)-isoxazole
4-(3-méthoxycarbonyl-2-méthyl-4-méthylsulfonylbenzoyle)-5-méthylisoxazole
5-(2-méthylcyclopropyl)-4-(2-nitro-4-trifluorométhylbenzoyl)-isoxazole
4-[2-chloro-3-(1-méthyléthoxy)-4-méthylsulfonylbenzoyle)-5-méthylisoxazole
5-méthyl-4-[2-méthyl-3-(1-méthyléthoxycarbonyl)-4-méthylsulfonyl benzoyl]-isoxazole
5-cyclopropyl-4-[2-méthyl-3-(1-méthyléthoxycarbonyl)-4-méthylsulfonyl benzoyl]-isoxazole ou
5-cyclopropyl-4-trichlorobenzoyl]-isoxazole

10. Une composition herbicide qui contient, comme matière active, un dérivé de l' isoxazole de formule générale III 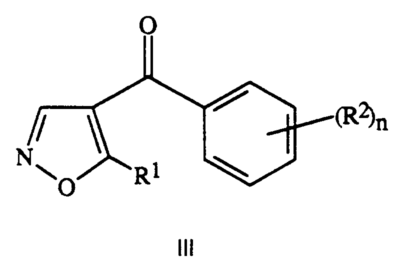 dans laquelle :
R¹ représente : un radical alcoyle, alcényle ou alcynyle, linéaire ou ramifié, contenant
de 1 à 6 atomes de carbone, éventuellement substitué par un ou plusieurs atomes d'halogènes ;
un radical cycloalkyle contenant de 3 à 6 atomes de carbone éventuellement substitué par un ou plusieurs radicaux R³ ou par un ou plusieurs atomes d'halogènes ou par un un radical ester CO₂R³¹ ;
un radical cycloalkènyle contenant 5 ou 6 atomes de carbone éventuellement substitué par un ou plusieurs radicaux R³ ou par un ou plusieurs atomes d' halogènes ou par un radical ester CO₂R³¹ un radical aryle ou aralkyle ;
un radical CO₂R³ ;
un aldéhyde ou un radical acyle COR³
un radical cyano ;
un radical nitro ;
un radical amino NR³R⁴ ; ou
un atome d' halogène ;
R² représente
un radical nitro ;
un radical cyano ;
un atome d' halogène ;
un radical R⁵ ;
un radical sulfènyle, sulfinyle ou sulfonyle S(O)ₘR⁵ ;
un radical sulfamoyle SONR³R⁴ ;
un radical CO₂R³ ;
un aldéhyde ou un radical acyle COR³
un radical carbamoyle ou thiocarbamoyle CONR³R⁴ ou CSNR³R⁴ ;
un radical alcoxy OR⁵ ; ou
un radical alkyle, contenant de 1 à 3 atomes de carbone, substitué par un radical OR⁵ ;
R³ et R⁴ qui peuvent être identiques ou différents représentent chacun :
l'hydrogène ou un radical alkyle linéaire ou ramifié contenant de 1 à 6 atomes de carbone, qui est éventuellement substitué par un ou plusieurs atomes d'halogènes ;
R³¹ a la même définition que R³ mais ne peut pas être l'hydrogène ;
R⁵ représente :
un radical alkyle linéaire ou ramifié contenant de 1 à 6 atomes de carbone éventuellement substitué par un ou plusieurs atomes d' halogènes ;
m représente 0, 1 ou 2
n représente un nombre entier pouvant prendre toutes les valeurs de 1 à 5 ;
en mélange avec une charge ou un diluant acceptable pour des utilisations agricoles,
à condition que si R¹ représente un radical alkyle en C₁₋₄, n est un nombre entier égal à 1, 2 ou 3 et R² représente un radical choisi parmi les radicaux suivants, halogène, alkyle en C₁₋₄, alkoxy en C₁₋₄, trifluorométhyle et trichlorométhyle le composé ne soit pas présent dans un solvant organique unique avec un composé de formule générale 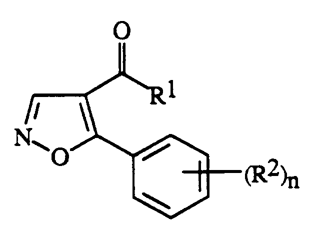 dans laquelle R¹ représente un radical alkyle en C₁₋₄, n est un nombre entier égal à 1, 2 ou 3 et R² représente un radical choisi parmi les radicaux suivants, halogène, alkyle en C₁₋₄, alkoxy en C₁₋₄, trifluorométhyle et trichlorométhyle.

11. Une composition herbicide selon la revendication 10 qui comprend un composé selon l'une quelconque des revendications 3 à 9.

12. Une composition herbicide selon la revendication 10 ou 11 qui comprend de 0,05 à 90% en poids de un ou plusieurs des composés de formule générale III.

13. Une composition herbicide selon l'une quelconque des revendications 10 à 12 qui contient jusqu'à 15% en poids d'un agent tensio-actif dans des suspensions emulsionnables concentrées liquides et jusqu'à 25% dans des concentrés liquides solubles dans l'eau.

14. Une composition herbicide selon l'une quelconque des revendications 10 à 13 qui se présente sous une forme solide telle que une poudre mouillable ou un granulé ; ou sous forme liquide telle qu'une solution, une suspension ou une émulsion.

15. Une composition herbicide selon l'une quelconque des revendications 10 à 14 qui contient aussi un adjuvant, un colloïde protecteur, un agent épaississant, un agent facilitant la pénétration, un stabilisant, un agent séquestrant, un agent anti-mottant, un agent colorant ou un inhibiteur de corrosion.

16. Une composition herbicide selon l'une quelconque des revendications 10 à 15 se présentant sous la forme de :
une suspension aqueuse concentrée qui contient de 10 à 70% de un ou plusieurs composés de formule générale III, de 2 à 10% d'un agent tensio-actif, de 0,1 à 5% d'un agent épaississant et de 15 à 87,9% d'eau ;
une poudre mouillable qui contient de 10 à 90% de un ou plusieurs composés de formule générale III, de 2à 10% d'un agent tensio-actif et de 8 à 88% d'une charge solide ou liquide ;
une poudre soluble qui contient de 10 à 90% de un ou plusieurs composés de formule générale III, de 2 à 40% de carbonate de sodium et de 0 à 88% d'une charge solide ;
un concentré liquide soluble dans l'eau qui contient de 5 à50% et de préférence de 10 à 30% de un ou plusieurs composés de formule générale III, de 5 à 25% d'un agent tensio-actif et de 25 à 90% d'un solvant miscible à l'eau, ou d'un mélange de solvants miscible à l'eau et d'eau ;
une suspension émulsionnable liquide concentrée qui contient de 10 à 70% de un ou plusieurs composés de formule générale III, de 5 à15% d'un agent tensio-actif, de 0,1 à 5% d'un agent épaississant et de 10 à 84,9% de solvant organique ;
un granulé qui contient de 1 à 90% de un ou plusieurs composés de formule générale III, de 0,5 à 7%, et de préférence de 0,5 à 2% d'un agent tensio-actif et de 3 à 98,5% d'une charge granulée; ou
un concentré émulsionnable qui contient de 0,05 à 90% de un ou plusieurs composés de formule générale III, de 0,01 à 10% d'un agent tensio-actif et de 9,99 à 99 ,94% de solvant organique.

17. Un produit manufacturé qui contient au moins un des dérivés de l'isoxazole selon l'une quelconque des revendications 1 à 9 ou une composition herbicide selon l'une quelconque des revendications 10 à 16 contenant au moins un dérivé de l'isoxazole de formule générale III à l'intérieur d'un récipient pour le dit ou les dits dérivés, ou pour la dite composition herbicide, et des instructions, physiquement associée au dit récipient précisant les conditions dans lesquelles le dit ou les dits dérivés ou les compositions herbicides qu'ils contiennent peuvent être utilisées pour contrôler la croissance des mauvaises herbes.

18. Un procédé de contrôle de la croissance des mauvaises herbes en un lieu qui consiste à appliquer sur le dit lieu une quantité herbicide efficace d'un dérivé de l'isoxazole de formule générale III 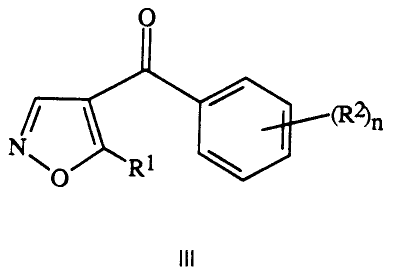 dans laquelle :
R¹ représente :
un radical alcoyle, alcényle ou alcynyle, linéaire ou ramifié, contenant de 1 à 6 atomes de carbone, éventuellement substitué par un ou plusieurs atomes d'halogènes ;
un radical cycloalkyle contenant de 3 à 6 atomes de carbone éventuellement substitué par un ou plusieurs radicaux R³ ou par un ou plusieurs atomes d'halogènes ou par un radical ester CO₂R³¹ ;
un radical cycloalkènyle contenant 5 ou 6 atomes de carbone éventuellement substitué par un ou plusieurs radicaux R³ ou par un ou plusieurs atomes d'halogènes ou par un radical ester CO₂R³¹ un radical aryle ou aralkyle ;
un radical CO₂R³ ;
un aldéhyde ou un radical acyle COR³
un radical cyano ;
un radical nitro ;
un radical amino NR³R⁴ ; ou
un atome d'halogène ;
R² représente
un radical nitro ;
un radical cyano ;
un atome d'halogène ;
un radical R⁵ ;
un radical sulfènyle, sulfinyle ou sulfonyle S(O)ₘR⁵ ;
un radical sulfamoyle SONR³R⁴ ;
un radical CO₂R³ ;
un aldéhyde ou un radical acyle COR³
un radical carbamoyle ou thiocarbamoyle CONR³R⁴ ou CSNR³R⁴ ;
un radical alcoxy OR⁵ ; ou
un radical alkyle contenant de 1 à 3 atomes de carbone substitué par un radical OR⁵ ;
R³ et R⁴ qui peuvent être identiques ou différents représentent chacun :
l'hydrogène ou un radical alkyle linéaire ou ramifié contenant de 1 à 6 atomes de carbone, qui est éventuellement substitué par un ou plusieurs atomes d'halogènes ;
R³¹ a la même définition que R³ mais ne peut pas être l'hydrogène ;
R⁵ représente :
un radical alkyle linéaire ou ramifié contenant de 1 à 6 atomes de carbone éventuellement substitué par un ou plusieurs atomes d'halogènes ;
m représente 0, 1 ou 2
n représente un nombre entier pouvant prendre toutes les valeurs de 1 à 5 ;
ou un sel de ces composés acceptable pour des usages agricoles.

19. Un procédé selon la revendication 18 dans lequel la dose d'application du dérivé de l'isoxazole de formule générale III ou de ses sels acceptables pour des usages agricoles est comprise entre 0,01 kg et 20 kg par hectare.

20. Un procédé selon la revendication 19 pour le contrôle sélectif de la croissance des mauvaises herbes dans les cultures dans lequel la dose d'application est comprise entre 0,01 kg et 8 kg par hectare.

21. Un procédé selon la revendication 20 pour le contrôle sélectif de la croissance des mauvaises herbes dans les cultures dans lequel la dose d'application est comprise entre 0,1 kg et 4 kg par hectare.

22. Un procédé selon la revendication 18 réalisé en application de préparé- ou de post-émergence dans des vergers établis ou d'autres zones ou plantations où poussent des arbres, avant ou après la plantation des arbres à une dose d'emploi comprise entre 0,25 kg et 10,0 kg par hectare.

23. Un procédé selon la revendication 22 dans lequel la dose d'emploi est comprise entre 0,5 kg et 8,0 kg par hectare.

24. Un procédé de préparation d'un dérivé de l'isoxazole de formule générale III tel que défini dans la revendication 1, à condition que R¹ ne soit pas un radical cyano, un radical nitro, un radical amino ou un atome d' halogène, qui comprend la réaction d'un composé de formule générale IV 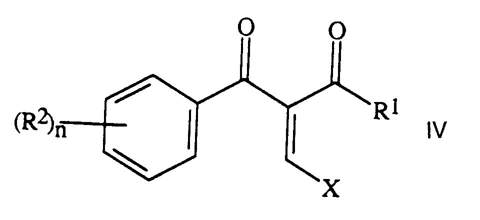 dans laquelle R¹, R² et n ont la définition donnée dans la revendication 1, à condition que R¹ ne soit pas un radical cyano, un radical nitro, un radical amino ou un atome d' halogène, et X est O-alkyle ou N-dialkyle, avec un sel d'hydroxylamine dans un solvant, éventuellement en présence d'une base.

25. Un procédé selon la revendication 25 dans lequel le solvant est l'acétone ou l'acétonitrile.

26. Un procédé de préparation d'un dérivé de l'isoxazole de formule générale III tel que défini dans la revendication 1 qui comprend :
(a) si R² est un halogène un radical alkyle, un radical alkylthio ou un radical alkoxy, la réaction d'un composé de formule générale V 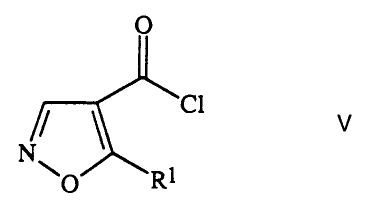 dans laquelle R¹ est défini comme décrit dans la revendication 1, avec un excès d'un benzène substitué approprié en présence d'un catalyseur acide de Lewis à une température comprise entre la température ambiante et 100°C ;
(b) si R¹ est un radical amino non substitué, la réaction d'un composé de formule générale VI 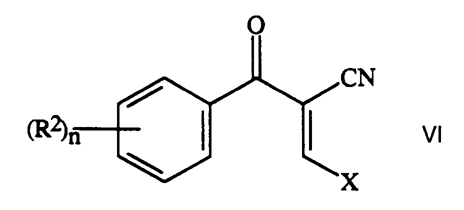 dans laquelle R² et n ont la définition donnée dans la revendication 1 et X est O-alkyle ou N-dialkyle, avec un sel d'hydroxylamine dans un solvant, éventuellement en présence d'une base à une température comprise entre la température ambiante et la température de reflux du solvant ;
(c) si R¹ est un radical cyano, la conversion en dérivé cyano du composé correspondant de formule générale III dans laquelle R¹ est un radical ester par hydrolyse acide de l'acide carboxylique suivi par une conversion en chlorure d'acide, le chlorure d'acide étant éventuellement transformé en amide et l'amide étant éventuellement transformée en un radical cyano par déhydratation ;
(d) si R¹ est un radical acyle, la conversion en dérivé acyle du composé correspondant de formule générale III dans laquelle R¹ est un radical cyano par réaction avec un dérivé organométallique ;
(e) si R¹ est un radical nitro, l'oxydationdu dérivé correspondant de formule générale III dans laquelle R¹ est un radical amino non substitué ;
(f) si R¹ est un halogène, la conversion en dérivé halogéné du composé correspondant de formule générale III dans laquelle R¹ est un radical amino non substitué par diazotation ;
(g) si R¹ est un radical amino substitué, déplacement de l'atome d'halogène du dérivé correspondant de formule générale III en utilisant l'amine appropriée dans un solvant inerte ;
(h) si le composé de formule générale III contient un radical sulfinyle ou sulfonyle, oxydation du dérivé correspondant de formule générale III contenant un radical thioéther.

27. Un composé qui peut être utilisé comme un intermédiaire dans la préparation d'un composé de formule générale III comme défini dans l'une quelconque des revendications 1 à 10, caractérisé en ce qu'il répond à la formule générale IV 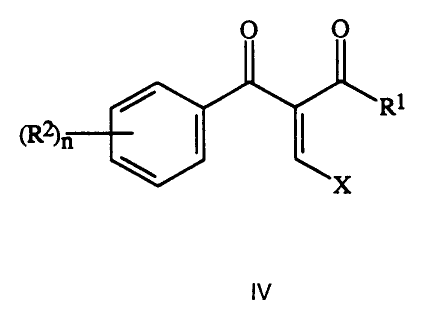 dans laquelle R¹ représente :
un radical alcoyle, alcényle ou alcynyle, linéaire ou ramifié, contenant de 1 à 6 atomes de carbone, éventuellement substitué par un ou plusieurs atomes d'halogènes ;
un radical cycloalkyle contenant de 3 à 6 atomes de carbone éventuellement substitué par un ou plusieurs radicaux R³ ou par un ou plusieurs atomes d'halogènes ou par un radical ester CO₂R³¹ ;
un radical cycloalkènyle contenant 5 ou 6 atomes de carbone éventuellement substitué par un ou plusieurs radicaux R³ ou par un ou plusieurs atomes d'halogènes ou par un radical ester CO₂R³¹
un radical aryle ou aralkyle ;
un radical CO₂R³ ;
un aldéhyde ou un radical acyle COR³
un radical cyano ;
un radical nitro ; ou
un radical amino NR³R⁴ ; ou
un atome d'halogène ;
R² représente
un radical nitro ; ou
un radical cyano ;
un atome d'halogène ;
un radical R⁵ ;
un radical sulfènyle, sulfinyle ou sulfonyle S(O)ₘR⁵ ;
un radical sulfamoyle SONR³R⁴ ;
un radical CO₂R³ ;
un aldéhyde ou un radical acyle COR³
un radical carbamoyle ou thiocarbamoyle CONR³R⁴ ou CSNR³R⁴ ;
un radical alcoxy OR⁵ ; ou
un radical alkyle contenant de 1 à 3 atomes de carbone substitué par un radical OR⁵ ;
R³ et R⁴ qui peuvent être identiques ou différents représentent chacun :
l'hydrogène ou un radical alkyle linéaire ou ramifié contenant de 1 à 6 atomes de carbone, qui est éventuellement substitué par un ou plusieurs atomes d'halogènes ;
R³¹ a la même définition que R³ mais ne peut pas être l'hydrogène ;
R⁵ représente :
un radical alkyle linéaire ou ramifié contenant de 1 à 6 atomes de carbone éventuellement substitué par un ou plusieurs atomes d'halogènes ;
m représente 0, 1 ou 2
n représente un nombre entier pouvant prendre toutes les valeurs de 1 à 5 ;
à condition que :
(a) si X représente -N(CH₃)₂, R² est choisi parmi les radicaux suivants, halogène, C₁₋₄ alkyle, C₁₋₄ alkoxy, trifluorométhyle et trichlorométhyle, et n est un un nombre entier pouvant prendre toutes les valeurs de 1 à 3 ; R¹ ne soit pas un radical alkyle en C₁₋₄ ;
(b) si X et R¹ représente -N(CH₃)₂, (R²)ₙ ne soit pas un radical 2,4-dichloro-5-fluoro ; et
(c) si X représente -N(CH₃)₂ et R¹ représente radical méthyle, (R²)ₙ ne représente pas un radical 4-chloro ou 4-méthoxy.

## Revendications (Revendications pour l'(les) Etat(s) contractant(s) suivant(s): ES)

1. Un procédé de préparation d'un dérivé de l'isoxazole de formule générale III, ou d'un sel de ce composé acceptable pour des applications agricoles : 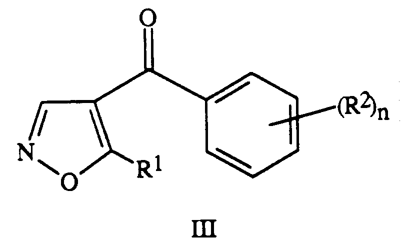 dans laquelle :
R¹ représente :
un radical alcoyle, alcényle ou alcynyle, linéaire ou ramifié, contenant de 1 à 6 atomes de carbone, éventuellement substitué par un ou plusieurs atomes d'halogènes ;
un radical cycloalkyle contenant de 3 à 6 atomes de carbone éventuellement substitué par un ou plusieurs radicaux R³ ou par un ou plusieurs atomes d'halogènes ou par un radical ester CO₂R³¹ ;
un radical cycloalkènyle contenant 5 ou 6 atomes de carbone éventuellement substitué par un ou plusieurs radicaux R³ ou par un ou plusieurs atomes d'halogènes ou par un radical ester CO₂R³¹
un radical aryle ou aralkyle ;
un radical CO₂R³ ;
un aldéhyde ou un radical acyle COR³
un radical cyano ;
un radical nitro ; ou
un radical amino NR³R⁴ ; ou
un atome d'halogène ;
R² représente
un radical nitro ; ou
un radical cyano ;
un atome d'halogène ;
un radical R⁵ ;
un radical sulfènyle, sulfinyle ou sulfonyle S(O)ₘR⁵ ;
un radical sulfamoyle SONR³R⁴ ;
un radical CO₂R³ ;
un aldéhyde ou un radical acyle COR³
un radical carbamoyle ou thiocarbamoyle CONR³R⁴ ou CSNR³R⁴ ;
un radical alcoxy OR⁵ ; ou
un radical alkyle contenant de 1 à 3 atomes de carbone substitué par un radical OR⁵ ;
R³ et R⁴ qui peuvent être identiques ou différents représentent chacun :
l'hydrogène ou un radical alkyle linéaire ou ramifié contenant de 1 à 6 atomes de carbone, qui est éventuellement substitué par un ou plusieurs atomes d'halogènes ;
R³¹ a la même définition que R³ mais ne peut pas être l'hydrogène ;
R⁵ représente :
un radical alkyle linéaire ou ramifié contenant de 1 à 6 atomes de carbone éventuellement substitué par un ou plusieurs atomes d'halogènes ;
m représente 0, 1 ou 2
n représente un nombre entier pouvant prendre toutes les valeurs de 1 à 5 ;
à condition que :
a) si R¹ représente un radical alkyle en C₁₋₄, n est un nombre entier égal à 1, 2 ou 3 et R² représente un radical choisi parmi les radicaux suivants, halogène, alkyle en C₁₋₄, alkoxy en C₁₋₄, trifluorométhyle et trichlorométhyle le composé ne soit pas présent dans un mélange avec un composé de formule générale 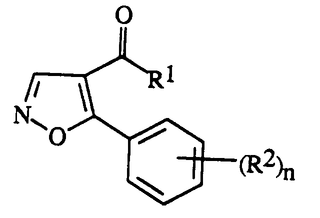 dans laquelle R¹ représente un radical alkyle en C₁₋₄, n est un nombre entier égal à 1, 2 ou 3 et R² représente un radical choisi parmi les radicaux suivants, halogène, alkyle en C₁₋₄, alkoxy en C₁₋₄, trifluorométhyle et trichlorométhyle et ; et
(b) si R¹ représente -NH₂ (R²)ₙ ne soit pas un radical 4-bromo ;
(c) si R¹ représente un radical méthyle, (R²)ₙ ne soit pas un radical 4-fluoro ;
ou des sels de ces composés acceptables pour des usages agricoles, qui comprend
(a) la réaction d'un composé de formule générale IV 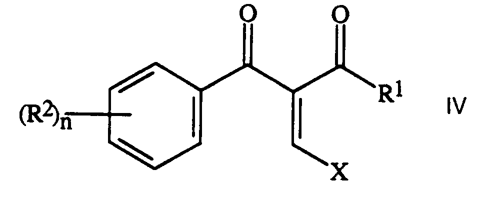 dans laquelle R¹, R² et n ont la définition donnéeci-dessus, à condition que R¹ ne soit pas un radical cyano, un radical nitro, un radical amino ou un atome d' halogène, et X est O-alkyle ou N-dialkyle, avec un sel d'hydroxylamine dans un solvant, éventuellement en présence d'une base.
(b) si R² est un halogène un radical alkyle, un radical alkylthio ou un radical alkoxy, la réaction d'un composé de formule générale V 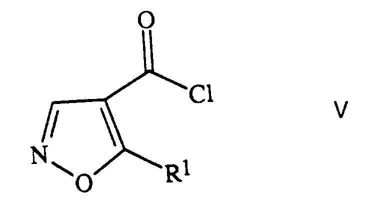 dans laquelle R¹ est défini comme décrit ci-dessus, avec un excès d'un benzène substitué approprié en présence d'un catalyseur acide de Lewis à une température comprise entre la température ambiante et 100°C ;
(c) si R¹ est un radical amino non substitué, la réaction d'un composé de formule générale VI 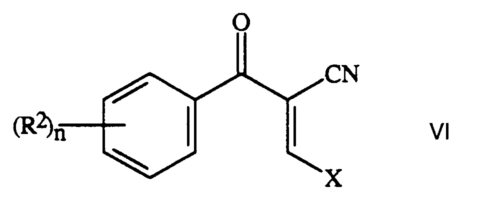 dans laquelle R² et n ont la définition donnée ci-dessus et X est O-alkyle ou N-dialkyle, avec un sel d'hydroxylamine dans un solvant, éventuellement en présence d'une base à une température comprise entre la température ambiante et la température de reflux du solvant ;
(d) si R¹ est un radical cyano, la conversion en dérivé cyano du composé correspondant de formule générale III dans laquelle R¹ est un radical ester par hydrolyse acide de l'acide carboxylique suivi par une conversion en chlorure d'acide, le chlorure d'acide etant éventuellement transformé en amide et l'amide étant éventuellement transformée en un radical cyano par déhydratation ;
(e) si R¹ est un radical acyle, la conversion en dérivé acyle du composé correspondant de formule générale III dans laquelle R¹ est un radical cyano par réaction avec un dérivé organométallique ;
(f) si R¹ est un radical nitro, l'oxydationdu dérivé correspondant de formule générale III dans laquelle R¹ est un radical amino non substitué ;
(g) si R¹ est un halogène, la conversion en dérivé halogéné du composé correspondant de formule générale III dans laquelle R¹ est un radical amino non substitué par diazotation ;
(h) si R¹ est un radical amino substitué, déplacement de l'atome d'halogène du dérivé correspondant de formule générale III en utilisant l'amine appropriée dans un solvant inerte ;
(i) si le composé de formule générale III contient un radical sulfinyle ou sulfonyle, oxydation du dérivé correspondant de formule générale III contenant un radical thioéther.

2. Un procédé selon la revendication 1 pour préparer un sel.

3. Un procédé selon la revendication 3 dans laquelle les radicaux aryle ou aralkyle répondent à la formule générale (R²)_{q}-phényl-[-CR³R⁴-]ₚ dans laquelle q représente zéro ou un nombre entier de 1 à 5 et p est zéro ou 1.

4. Un procédé selon l'une des revendications 1, 2 ou 3 dans lequel R^{1°} représente un radical alkyle ou cycloalkyle et n est 2 ou 3.

5. Un procédé selon l'une des revendications 1, 2, 3 ou 4 dans lequel un des groupes représenté par (R²)ₙ est fixé en position ortho sur le noyau phényle.

6. Un procédé selon l'une quelconque des revendications 1 à 5 dans lequel R¹ est un radical alkyl éventuellement substitué par un halogène, ou un radical cycloalkyl éventuellement substitué par un radical alkyl ou par un halogène .

7. Un procédé selon l'une quelconque des revendications 1 à 6 dans lequel R² est un halogène, un radical nitro ; R⁵ ; -S(O)ₘR⁵ ; -OR⁵ ; un radical alkyle substitué par -OR⁵ ; ou -COOR³.

8. Un procédé selon l'une quelconque des revendications 1à 7 dans lequel R⁵ est un radical alkyle (contenant de 1 à 3 atomes de carbone ) éventuellement substitué par F ou Cl.

9. Un procédé selon la revendication 1 pour la préparation des composés suivants :
5-méthyle-4-(2-nitro-4-trifluorométhylbenzoyl)-isoxazole
5-méthyle-4-(2-nitrobenzoyl)-isoxazole
4-(2-nitro-4-trifluorométhylbenzoyl)-5-phénylisoxazole
4-(2,4-dinitrobenzoyl)-5-méthylisoxazole
5-(4-chlorophényle)-4-(2-nitro-4-trifluorométhylbenzoyl)-isoxazole
4-(2-chlorobenzoyl)-5-méthylisoxazole
5-méthyle-4-(2-nitro-4-méthylsulfonylbenzoyl)-isoxazole
5-1-(méthyléthyl)-4-(2-nitro-4-trifluorométhylbenzoyl)-isoxazole
4-(4-chlorobenzoyle)-5-méthylisoxazole
4-(4-méthylbenzoyle)-5-méthylisoxazole
5-(4-fluorophényle)-4-(2-nitro-4-trifluorométhylbenzoyl)-isoxazole
5-éthyle-4-(2-nitro-4-trifluorométhylbenzoyl)-isoxazole
4-(4-chloro-2-nitrobenzoyle)-5-méthylisoxazole
4-(2-nitro-4-trifluorométhylbenzoyle)-5-propylisoxazole
5-cyclopropyl-4-(2-nitro-4-trifluorométhylbenzoyl)-isoxazole
4-(2,3-dichloro-4-méthylsulfonylbenzoyl)-5-méthylisoxazole
5-(1,1diméthyléthyle)-4-(2-nitro-4-trifluorométhylbenzoyl)-isoxazole
4-(4-méthoxybenzoyl)-5-méthylisoxazole
5-méthyle-4-(4-méthyl-2-nitrobenzoyl)-isoxazole
4-(2,3-dichloro-4-méthylsulfonylbenzoyl)-5-(1-méthyléthyl)-isoxazole
5-cyclopropyl-4-(2,3-dichloro-4-méthylsulfonylbenzoyl)-5-isoxazole
4-(2-nitro-4-trifluorométhylbenzoyle)-5-phénylméthylisoxazole
4-(2-chloro-4-trifluorométhylbenzoyle)-5-cyclopropylisoxazole
5-méthyle-4-(2-nitro-4-pentafluoroéthylbenzoyl)-isoxazole
5-cyclopropyl-4-[4-(1,1-diméthyléthyl)-2-nitrobenzoyl]-5-isoxazole
4-[4-(1,1-diméthyléthyl)-2-nitrobenzoyl]-5-méthylisoxazole
5-cyclopentyle-4-(2-nitro-4-trifluorométhylbenzoyl)-isoxazole
4-(2,4-dichlorobenzoyle)-5-méthylisoxazole
4-(2-chloro-4-méthylsulfonylbenzoyle)-5-méthylisoxazole
4-(2-chloro-4-trifluorométhylbenzoyle)-5-méthylisoxazole
5-méthyle-4-(2-trifluorométhylbenzoyl)-isoxazole
5-méthyle-4-(2,4-bis-trifluorométhylbenzoyl)-isoxazole
4-(2-chloro-4-méthylsulfonylbenzoyle)-5-cyclopropylisoxazole
5-cyclopropyl-4-(2-trifluorométhylbenzoyl)-isoxazole
5-cyclopropyl-4-(2,4-dichlorobenzoyl)-isoxazole
4-[2,3-dichloro-4-(méthylthio)-benzoyle]-5-méthylisoxazole
5-cyclopropyle-4-(2,4-bis-trifluorométhylbenzoyl)-isoxazole
4-(4-chloro-2-trifluorométhylbenzoyle)-5-méthylisoxazole
4-(4-cyano-2-nitrobenzoyle)-5-méthylisoxazole
5-amino-4-(2-nitro-4-trifluorométhylbenzoyl)-isoxazole
4-(4-chloro-2-trifluorométhylbenzoyle)-5-cyclopropylisoxazole
5-(1-méthyléthyl)-4-(2-nitro-4-pentafluoroéthylbenzoyl)-isoxazole
4-(2-chloro-4-méthylsulfonylbenzoyle)-5-(1-méthyléthyl)-isoxazole
5-cyclopropyle-4-(4-fluoro-2-nitrobenzoyl)-isoxazole
5-cyclopropyle-4-(2-nitro-4-pentafluoroéthylbenzoyl)-isoxazole
4-(2,3-dichloro-4-méthylsulfinylbenzoyle)-5-méthylisoxazole
5-cyclobutyle-4-(2-nitro-4-trifluorométhylbenzoyl)-isoxazole
4-(4-fluoro-2-nitrobenzoyle)-5-méthylisoxazole
5-(1-méthylcyclopropyl)-4-(2-nitro-4-trifluorométhylbenzoyl)-isoxazole
5-(4-nitrophényl)-4-(2-nitro-4-trifluorométhylbenzoyl)-isoxazole
5-(4-méthoxyphényl)-4-(2-nitro-4-trifluorométhylbenzoyl)-isoxazole
4-(2-chloro-3-éthoxy-4-méthylsulfonylbenzoyle)-5-méthylisoxazole
4-(3-cyanobenzoyle)-5-méthylisoxazole
5-cyclopropyle-4-(4-méthylsulfonyl-2-trifluorométhyle benzoyl)-isoxazole
5-cyclopropyle-4-(2-nitro-4-méthylsulfonylbenzoyl)-isoxazole
4-(2-chloro-3-éthoxy-4-méthylsulfonylbenzoyle)-5-cyclopropylisoxazole
4-(2-chloro-3-éthoxy-4-éthylsulfonylbenzoyle)-5-méthylisoxazole
4-(2-chloro-3-éthoxy-4-méthylsulfonylbenzoyle)-5-(1-méthyléthyl)-isoxazole
4-(2-chloro-3-éthoxy-4-éthylsulfonylbenzoyle)-5-cyclopropylisoxazole
5-(1-éthoxycarbonylcyclopropyl)-4-(2-nitro-4-trifluorométhylbenzoyl)-isoxazole
4-(3-méthoxycarbonyl-2-méthyl-4-méthylsulfonylbenzoyle)-5-méthylisoxazole
5-(2-méthylcyclopropyl)-4-(2-nitro-4-trifluorométhylbenzoyl)-isoxazole
4-[2-chloro-3-(1-méthyléthoxy)-4-méthylsulfonylbenzoyle)-5-méthylisoxazole
5-méthyl-4-[2-méthyl-3-(1-méthyléthoxycarbonyl)-4-méthylsulfonyl benzoyl]-isoxazole
5-cyclopropyl-4-[2-méthyl-3-(1-méthyléthoxycarbonyl)-4-méthylsulfonyl benzoyl]-isoxazole ou
5-cyclopropyl-4-trichlorobenzoyl]-isoxazole

10. Un procédé de préparation d'une composition herbicide comprenant la formulation d'un dérivé de l'isoxazole de formule générale III 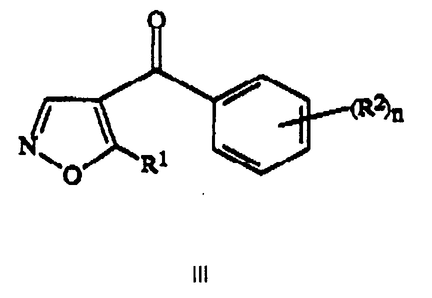 dans laquelle :
R¹ représente :
un radical alcoyle, alcényle ou alcynyle, linéaire ou ramifié, contenant de 1 à 6 atomes de carbone, éventuellement substitué par un ou plusieurs atomes d'halogènes ;
un radical cycloalkyle contenant de 3 à 6 atomes de carbone éventuellement substitué par un ou plusieurs radicaux R³ ou par un ou plusieurs atomes d'halogènes ou par un radical ester CO₂R³¹ ;
un radical cycloalkènyle contenant 5 ou 6 atomes de carbone éventuellement substitué par un ou plusieurs radicaux R³ ou par un ou plusieurs atomes d'halogènes ou par un radical ester CO₂R³¹
un radical aryle ou aralkyle ;
un radical CO₂R³ ;
un aldéhyde ou un radical acyle COR³
un radical cyano ;
un radical nitro ;
un radical amino NR³R⁴ ; ou
un atome d'halogène ;
R² représente
un radical nitro ;
un radical cyano ;
un atome d'halogène ;
un radical R⁵ ;
un radical sulfènyle, sulfinyle ou sulfonyle S(O)ₘR⁵ ;
un radical sulfamoyle SONR³R⁴ ;
un radical CO₂R³ ;
un aldéhyde ou un radical acyle COR³
un radical carbamoyle ou thiocarbamoyle CONR³R⁴ ou CSNR³R⁴ ;
un radical alcoxy OR⁵ ; ou
un radical alkyle contenant de 1 à 3 atomes de carbone substitué par un radical OR⁵ ;
R³ et R⁴ qui peuvent être identiques ou différents représentent chacun :
l'hydrogène ou un radical alkyle linéaire ou ramifié contenant de 1 à 6 atomes de carbone, qui est éventuellement substitué par un ou plusieurs atomes d'halogènes ;
R³¹ a la même définition que R³ mais ne peut pas être l'hydrogène ;
R⁵ représente :
un radical alkyle linéaire ou ramifié contenant de 1 à 6 atomes de carbone éventuellement substitué par un ou plusieurs atomes d'halogènes ;
m représente 0, 1 ou 2
n représente un nombre entier pouvant prendre toutes les valeurs de 1 à 5 ;
avec une charge solide ou liquide acceptable pour des applications agricoles ;
à condition que si R¹ représente un radical alkyle en C₁₋₄, n est un nombre entier égal à 1, 2 ou 3 et R² représente un radical choisi parmi les radicaux suivants, halogène, alkyle en C₁₋₄, alkoxy en C₁₋₄, trifluorométhyle et trichlorométhyle la composition ne soit pas préparée dans un solvant organique unique avec un composé de formule générale 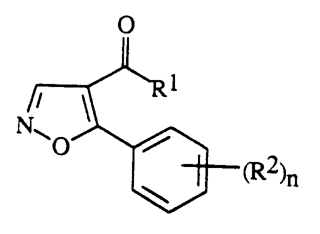 dans laquelle R¹ représente un radical alkyle en C₁₋₄, n est un nombre entier égal à 1, 2 ou 3 et R² représente un radical choisi parmi les radicaux suivants, halogène, alkyle en C₁₋₄, alkoxy en C₁₋₄, trifluorométhyle et trichlorométhyle.

11. Un procédé selon la revendication 10 pour la préparation d'une composition herbicide qui contient de 0,05 à 90 % en poids de un ou plusieurs composés de formule générale III.

12. Un procédé selon la revendication 10 ou 11 pour la préparation d'une composition herbicide qui contient jusqu'à 15% en poids d'un agent tensio-actif dans des suspensions emulsionnables concentrées liquides et jusqu'à 25% dans des concentrés liquides solubles dans l'eau.

13. Un procédé selon la revendication 10, 11 ou 12 pour la préparation d'une composition herbicide qui est sous une forme solide telle qu'une poudre mouillable ou un granulé ; ou sous forme liquide tel qu'une solution, une suspension ou une émulsion.

14. Un procédé selon la revendication 10 à 13 pour la préparation d'une composition herbicide qui contient aussi un adjuvant, un colloïde protecteur, un agent épaississant, un agent facilitant la pénétration, un stabilisant, un agent séquestrant, un agent anti-mottant, un agent colorant ou un inhibiteur de corrosion.

15. Un procédé selon la revendication 10 à 14 pour la préparation d'une composition herbicide se présentant sous la forme de :
une suspension aqueuse concentrée qui contient de 10 à 70% de un ou plusieurs composés de formule générale III, de 2 à 10% d'un agent tensio-actif, de 0,1 à 5% d'un agent épaississant et de 15 à 87,9% d'eau ;
une poudre mouillable qui contient de 10 à 90% de un ou plusieurs composés de formule générale III, de 2à 10% d'un agent tensio-actif et de 8 à 88% d'une charge solide ou liquide ;
une poudre soluble qui contient de 10 à 90% de un ou plusieurs composés de formule générale III, de 2 à 40% de carbonate de sodium et de 0 à 88% d'une charge solide ;
un concentré liquide soluble dans l'eau qui contient de 5 à50% et de préférence de 10 à 30% de un ou plusieurs composés de formule générale III, de 5 à 25% d'un agent tensio-actif et de 25 à 90% d'un solvant miscible à l'eau, ou d'un mélange de solvants miscible à l'eau et d'eau ;
une suspension émulsionnable liquide concentrée qui contient de 10 à 70% de un ou plusieurs composés de formule générale III, de 5 à15% d'un agent tensio-actif, de 0,1 à 5% d'un agent épaississant et de 10 à 84,9% de solvant organique ;
un granulé qui contient de 1 à 90% de un ou plusieurs composés de formule générale III, de 0,5 à 7%, et de préférence de 0,5 à 2% d'un agent tensio-actif et de 3 à 98,5% d'une charge granulée; ou
un concentré émulsionnable qui contient de 0,05 à 90% de un ou plusieurs composés de formule générale III, de 0,01 à 10% d'un agent tensio-actif et de 9,99 à 99 ,94% de solvant organique.

16. Un procédé de contrôle de la croissance des mauvaises herbes en un lieu qui consiste à appliquer sur le dit lieu une quantité herbicide efficace d'un dérivé de l'isoxazole de formule générale III 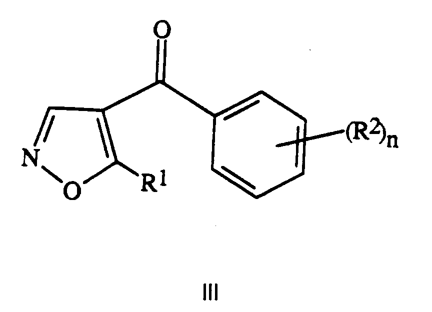 dans laquelle :
R¹ représente :
un radical alcoyle, alcényle ou alcynyle, linéaire ou ramifié, contenant de 1 à 6 atomes de carbone, éventuellement substitué par un ou plusieurs atomes d'halogènes ;
un radical cycloalkyle contenant de 3 à 6 atomes de carbone éventuellement substitué par un ou plusieurs radicaux R³ ou par un ou plusieurs atomes d'halogènes ou par un radical ester CO₂R³¹ ;
un radical cycloalkènyle contenant 5 ou 6 atomes de carbone éventuellement substitué par un ou plusieurs radicaux R³ ou par un ou plusieurs atomes d'halogènes ou par un radical ester CO₂R³¹
un radical aryle ou aralkyle ;
un radical CO₂R³ ;
un aldéhyde ou un radical acyle COR³
un radical cyano ;
un radical nitro ;
un radical amino NR³R⁴ ; ou
un atome d'halogène ;
R² représente
un radical nitro ;
un radical cyano ;
un atome d'halogène ;
un radical R⁵ ;
un radical sulfènyle, sulfinyle ou sulfonyle S(O)ₘR⁵ ;
un radical sulfamoyle SONR³R⁴ ;
un radical CO₂R³ ;
un aldéhyde ou un radical acyle COR³
un radical carbamoyle ou thiocarbamoyle CONR³R⁴ ou CSNR³R⁴ ;
un radical alcoxy OR⁵ ; ou
un radical alkyle contenant de 1 à 3 atomes de carbone substitué par un radical OR⁵ ;
R³ et R⁴ qui peuvent être identiques ou différents représentent chacun :
l'hydrogène ou un radical alkyle linéaire ou ramifié contenant de 1 à 6 atomes de carbone, qui est éventuellement substitué par un ou plusieurs atomes d'halogènes ;
R³¹ a la même définition que R³ mais ne peut pas être l'hydrogène ;
R⁵ représente :
un radical alkyle linéaire ou ramifié contenant de 1 à 6 atomes de carbone éventuellement substitué par un ou plusieurs atomes d'halogènes ;
m représente 0, 1 ou 2
n représente un nombre entier pouvant prendre toutes les valeurs de 1 à 5 ;
ou un sel de ces composés acceptable pour des usages agricoles.

17. Un procédé selon la revendication 16 dans lequel la dose d'application du dérivé de l'isoxazole de formule générale III ou de ses sels acceptables pour des usages agricoles est comprise entre 0,01 kg et 20 kg par hectare.

18. Un procédé selon la revendication 17 pour le contrôle sélectif de la croissance des mauvaises herbes dans les cultures dans lequel la dose d'application est comprise entre 0,01 kg et 8 kg par hectare.

19. Un procédé selon la revendication 18 pour le contrôle sélectif de la croissance des mauvaises herbes dans les cultures dans lequel la dose d'application est comprise entre 0,1 kg et 4 kg par hectare.

20. Un procédé selon la revendication 16 réalisé en application de préparé- ou de post-émergence dans des vergers établis ou d'autres zones ou plantations où poussent des arbres, avant ou après la plantation des arbres à une dose d'emploi comprise entre 0,25 kg et 10,0 kg par hectare.

21. Un procédé selon la revendication 20 dans lequel la dose d'emploi est comprise entre 0,5 kg et 8,0 kg par hectare.

22. Un procédé de préparation d'un intermédiaire de formule générale IV 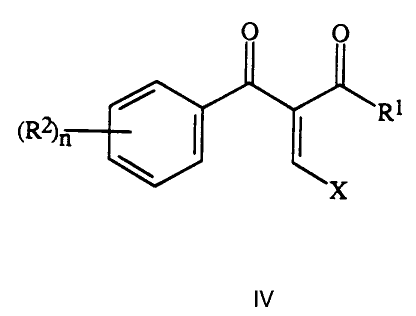 dans laquelle R¹ représente :
un radical alcoyle, alcényle ou alcynyle, linéaire ou ramifié, contenant de 1 à 6 atomes de carbone, éventuellement substitué par un ou plusieurs atomes d'halogènes ;
un radical cycloalkyle contenant de 3 à 6 atomes de carbone éventuellement substitué par un ou plusieurs radicaux R³ ou par un ou plusieurs atomes d'halogènes ou par un radical ester CO₂R³¹ ;
un radical cycloalkènyle contenant 5 ou 6 atomes de carbone éventuellement substitué par un ou plusieurs radicaux R³ ou par un ou plusieurs atomes d'halogènes ou par un radical ester CO₂R³¹
un radical aryle ou aralkyle ;
un radical CO₂R³ ;
un aldéhyde ou un radical acyle COR³
un radical cyano ;
un radical nitro ; ou
un radical amino NR³R⁴ ; ou
un atome d'halogène ;
R² représente
un radical nitro ; ou
un radical cyano ;
un atome d'halogène ;
un radical R⁵ ;
un radical sulfènyle, sulfinyle ou sulfonyle S(O)ₘR⁵ ;
un radical sulfamoyle SONR³R⁴ ;
un radical CO₂R³ ;
un aldéhyde ou un radical acyle COR³
un radical carbamoyle ou thiocarbamoyle CONR³R⁴ ou CSNR³R⁴ ;
un radical alcoxy OR⁵ ; ou
un radical alkyle contenant de 1 à 3 atomes de carbone substitué par un radical OR⁵ ;
R³ et R⁴ qui peuvent être identiques ou différents représentent chacun :
l'hydrogène ou un radical alkyle linéaire ou ramifié contenant de 1 à 6 atomes de carbone, qui est éventuellement substitué par un ou plusieurs atomes d'halogènes ;
R³¹ a la même définition que R³ mais ne peut pas être l'hydrogène ;
R⁵ représente :
un radical alkyle linéaire ou ramifié contenant de 1 à 6 atomes de carbone éventuellement substitué par un ou plusieurs atomes d'halogènes ;
m représente 0, 1 ou 2
n représente un nombre entier pouvant prendre toutes les valeurs de 1 à 5 ;
à condition que :
(a) si X représente -N(CH₃)₂, R² est choisi parmi les radicaux suivants, halogène, C₁₋₄ alkyle, C₁₋₄ alkoxy, trifluorométhyle et trichlorométhyle, et n est un un nombre entier pouvant prendre toutes les valeurs de 1 à 3 ; R¹ ne soit pas un radical alkyle en C₁₋₄ ;
(b) si X et R¹ représente -N(CH₃)₂, (R²)ₙ ne soit pas un radical 2,4-dichloro-5-fluoro ; et
(c) si X représente -N(CH₃)₂ et R¹ représente radical méthyle, (R²)ₙ ne représente pas un radical 4-chloro ou 4-méthoxy
comprenant la réaction d'une dicétone de formule générale VII 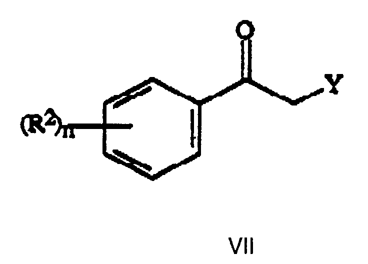 dans laquelle R² et n ont la définition donnée ci-dessus, Y est COR¹ et R¹ a la définition donnée ci-dessus, avec :
(i) un ortho ester en présence d'un catalyseur acide ; ou
(ii) un amide acétal dans un solvant inerte.
